# EUROPEAN PATENT SPECIFICATION

(11) **EP 3 807 413 B1**
(45) Date of publication and mention of the grant of the patent: **21.05.2025**
(21) Application number: 19749864.5
(22) Date of filing: 17.06.2019
(51) Int. Cl.: C12N 15/86, A61K 48/00

(54) **ADENO-ASSOCIATED VIRUS VECTOR DELIVERY OF MUSCLE SPECIFIC MICRO-DYSTROPHIN TO TREAT MUSCULAR DYSTROPHY**
ADENO-ASSOZIIERTE VIRUSVEKTORVERABREICHUNG VON MUSKELSPEZIFISCHEM MIKRODYSTROPHIN ZUR BEHANDLUNG VON MUSKELDYSTROPHIE
ADMINISTRATION PAR VECTEUR DE VIRUS ADÉNO-ASSOCIÉ DE MICRO-DYSTROPHINE SPÉCIFIQUE DES MUSCLES POUR TRAITER LA DYSTROPHIE MUSCULAIRE

(30) Priority: 18.06.2018 US 201862686668 P; 02.10.2018 US 201862740402 P; 30.10.2018 US 201862752841 P; 25.03.2019 US 201962823649 P; 11.06.2019 US 201962860220 P
(43) Date of publication of application: 21.04.2021
(73) Proprietor: Research Institute at Nationwide Children's Hospital, Columbus, Ohio 43205 (US)
(72) Inventor: RODINO-KLAPAC, Louise, Groveport, OH 43125 (US); MENDELL, Jerry, R., Columbus, OH 43235 (US)
(74) Representative: D Young & Co LLP
(86) International application number: PCT/US2019/037489
(87) International publication number: WO 2019/245973

(56) References cited:
- WO-A1-2017/181014
- WO-A2-2008/088895
- FRANZ W M ET AL: "Association of nonsense mutation of dystrophin gene with disruption of sarcoglycan complex in X-linked dilated cardiomyopathy", LANCET, ELSEVIER, AMSTERDAM, NL, vol. 355, no. 9217, 20 May 2000 (2000-05-20), pages 1781 - 1785, XP004815656, ISSN: 0140-6736, DOI: 10.1016/S0140-6736(00)02266-2

## Description

### FIELD OF INVENTION

The invention provides recombinant adeno-associated virus (AAV) vectors expressing a miniaturized human micro-dystrophin gene for use in treating muscular dystrophy in a human subject in need thereof.

### BACKGROUND

The importance of muscle mass and strength for daily activities, such as locomotion and breathing, and for whole body metabolism is unequivocal. Deficits in muscle function produce muscular dystrophies (MDs) that are characterized by muscle weakness and wasting and have serious impacts on quality of life. The most well-characterized MDs result from mutations in genes encoding members of the dystrophin-associated protein complex (DAPC). These MDs result from membrane fragility associated with the loss of sarcolemmal-cytoskeleton tethering by the DAPC. Duchenne Muscular Dystrophy (DMD) is one of the most devastating muscle disease affecting 1 in 5000 newborn males.

DMD is caused by mutations in the DMD gene leading to reductions in mRNA and the absence of dystrophin, a 427 kD sarcolemmal protein associated with the dystrophin-associated protein complex (DAPC) (Hoffman et al., Cell 51(6):919-28, 1987). The DAPC is composed of multiple proteins at the muscle sarcolemma that form a structural link between the extra-cellular matrix (ECM) and the cytoskeleton via dystrophin, an actin binding protein, and alpha-dystroglycan, a laminin-binding protein. These structural links act to stabilize the muscle cell membrane during contraction and protect against contraction-induced damage. With dystrophin loss, membrane fragility results in sarcolemmal tears and an influx of calcium, triggering calcium-activated proteases and segmental fiber necrosis (Straub et al., Curr Opin. Neurol. 10(2): 168-75, 1997). This uncontrolled cycle of muscle degeneration and regeneration ultimately exhausts the muscle stem cell population (Sacco et al., Cell, 2010. 143(7): p. 1059-71; Wallace et al., Annu Rev Physiol, 2009. 71: p. 37-57), resulting in progressive muscle weakness, endomysial inflammation, and fibrotic scarring.

Without membrane stabilization from dystrophin or a micro-dystrophin, DMD will manifest uncontrolled cycles of tissue injury and repair ultimately replace lost muscle fibers with fibrotic scar tissue through connective tissue proliferation. Fibrosis is characterized by the excessive deposits of ECM matrix proteins, including collagen and elastin. ECM proteins are primarily produced from cytokines such as TGFβ that is released by activated fibroblasts responding to stress and inflammation. Although the primary pathological feature of DMD is myofiber degeneration and necrosis, fibrosis as a pathological consequence has equal repercussions. The overproduction of fibrotic tissue restricts muscle regeneration and contributes to progressive muscle weakness in the DMD patient. In one study, the presence of fibrosis on initial DMD muscle biopsies was highly correlated with poor motor outcome at a 10-year follow-up (Desguerre et al., J Neuropathol Exp Neurol, 2009. 68(7): p. 762-7). These results point to fibrosis as a major contributor to DMD muscle dysfunction and highlight the need for early intervention prior to overt fibrosis.

There is a need for treatments that increase muscle strength and protect against muscle injury in patients suffering from DMD.

### SUMMARY OF INVENTION

The present invention is directed to a recombinant adeno-associated virus (rAAV) for use in treating muscular dystrophy in a human subject in need thereof, wherein the rAAV is of the serotype rh.74 and comprises a polynucleotide comprising the nucleotide sequence of SEQ ID NO: 9, wherein the rAAV is administered using an intravenous route of administration and at a dose of 2×10¹⁴ vg/kg.

The present invention is also directed to a recombinant adeno-associated virus (rAAV) for use in treating Duchenne muscular dystrophy in a human subject in need thereof, wherein the rAAV is of the serotype rh.74 and comprises a polynucleotide comprising the nucleotide sequence of SEQ ID NO: 9, wherein the rAAV is administered by intravenous infusion over approximately one hour at a dose of 2x 10¹⁴ vg/kg.

Gene therapy vectors, e.g. AAV, may express the micro-dystrophin gene to skeletal muscles including diaphragm and cardiac muscle to protect muscle fibers from injury, increase muscle strength and reduce and/or prevent fibrosis.

Therapies and approaches for increasing muscular force and/or increasing muscle mass using gene therapy vectors may deliver micro-dystrophin to address the gene defect observed in DMD. Example 2 describes a systemic gene delivery clinical trial for Duchenne muscular dystrophy, in this study the subjects received 2x10¹⁴ vg/kg AAVrh74.MHCK7.micro-dystrophin. The clinical study described in Example 3 provides a novel pivotal clinical protocol that includes a randomized double-blind placebo controlled design. At study initiation, subjects are randomized and either receive 2x10¹⁴ vg/kg AAVrh74.MHCK7.micro-dystrophin or lactated ringers.

Methods of treating a muscular dystrophy in a human subject in need thereof may comprise the step of administering a recombinant adeno-virus associated (rAAV) rAAV.MHCK7.microdystrophin, wherein the rAAV is administered by a systemic route of administration at a dose of about 5.0x10¹² vg/kg to about 1.0x10¹⁵vg/kg. The muscular dystrophy may be Duchenne muscular dystrophy or Becker's muscular dystrophy.

For example, the dose of rAAV administered is about 5.0x10¹² vg/kg to about 1.0x10¹⁴ vg/kg, or about 5.0x10¹² vg/kg to1.0x10¹⁴ vg/kg, or about 5.0x10¹² vg/kg to about 2.0x10¹⁴ vg/kg, or about 5.0x10¹² vg/kg to about 1.0x10¹⁴ vg/kg, or about 5.0x10¹² vg/kg to about 5.0x10¹³ vg/kg, or about 5.0x10¹² vg/kg to about 2.0x10¹³ vg/kg, or about 5.0x10¹² vg/kg to about 1.0x10¹³ vg/kg, or 1.0x10¹⁴ vg/kg to about 1.0x10¹⁵ vg/kg, or 1.0x10¹³ vg/kg to about 1.0x10¹⁴ vg/kg, or about 1.0x10¹³ vg/kg to1.0x10¹⁴ vg/kg, or about 1.0x10¹³ vg/kg to about 2.0x10¹⁴ vg/kg, or about 1.0x10¹³ vg/kg to about 1.0x10¹⁴ vg/kg, or about 1.0x10¹³ vg/kg to about 5.0x10¹³ vg/kg, or about 1.0x10¹³ vg/kg to about 3.0x10¹⁴ vg/kg, or about 1.0x10¹³ vg/kg to about 5.0x10¹⁴ vg/kg, or about 1.0x10¹³ vg/kg to about 6.0x10¹⁴vg/kg, or 1.0x10¹³ vg/kg to about 1.0x10¹⁵ vg/kg, or 5.0x10¹³ vg/kg to about 1.0x10¹⁴ vg/kg, or about 5.0x10¹³ vg/kg to1.0x10¹⁴ vg/kg, or about 5.0x10¹³ vg/kg to about 2.0x10¹⁴ vg/kg, or about 5.0x10¹³ vg/kg to about 1.0x10¹⁴ vg/kg, or about 5.0x10¹³ vg/kg to about 3.0x10¹⁴ vg/kg, or about 5.0x10¹³ vg/kg to about 5.0x10¹⁴ vg/kg, or about 5.0x10¹³ vg/kg to about 6.0x10¹⁴ vg/kg, or 5.0x10¹³ vg/kg to about 1.0x10¹⁵ vg/kg, or 1.0x10¹⁴ vg/kg to about 6.0x10¹⁴ vg/kg, or 1.0x10¹⁴ vg/kg to about 5.0x10¹⁴ vg/kg, or 1.0x10¹⁴ vg/kg to about 4.0x10¹⁴ vg/kg, or 1.0x10¹⁴ vg/kg to about 1.0x10¹⁵ vg/kg, or 1.0x10¹⁴ vg/kg to about 3.0x10¹⁴ vg/kg, or about 1.0x10¹⁴ vg/kg to about 2.5x10¹⁴ vg/kg, or 1.0x10¹⁴ vg/kg to about 2.0x10¹⁴ vg/kg, or about 1.25x10¹⁴ vg/kg to about 3.75x10¹⁴ vg/kg, or about 1.25x10¹⁴ vg/kg to 6.0x10¹⁴, or about 1.25x10¹⁴ vg/kg to 5.0x10¹⁴, or about 1.25x10¹⁴ vg/kg to 4.0x10¹⁴, or about 1.25x10¹⁴ vg/kg to 1.0x10¹⁵, or about 1.25x10¹⁴ vg/kg to about 3.5x10¹⁴ vg/kg, or about 1.25x10¹⁴ vg/kg to about 3.0x10¹⁴ vg/kg, or about 1.25x10¹⁴ vg/kg to about 2.75x10¹⁴ vg/kg, or about 1.25x10¹⁴ vg/kg to about 2.5x10¹⁴ vg/kg, or about 1.25x10¹⁴ vg/kg to about 2.0x10¹⁴ vg/kg, or 1.25x10¹⁴ vg/kg to about 3.75x10¹⁴ vg/kg, or about 1.25x10¹⁴ vg/kg to about 3.5x10¹⁴ vg/kg, or 1.5x10¹⁴ vg/kg to about 1.0x10¹⁵ vg/kg, or about 1.5x10¹⁴ vg/kg to 6.0x10¹⁴, or about 1.5x10¹⁴ vg/kg to 5.0x10¹⁴, or about 1.5x10¹⁴ vg/kg to 4.0x10¹⁴, or about 1.5x10¹⁴ vg/kg to about 3.75x10¹⁴ vg/kg, or about 1.5x10¹⁴ vg/kg to about 3.5x10¹⁴ vg/kg, or about 1.5x10¹⁴ vg/kg to about 3.25x10¹⁴ vg/kg, or about 1.5x10¹⁴ vg/kg to about 3.0x10¹⁴ vg/kg, or about 1.5x10¹⁴ vg/kg to about 2.75x10¹⁴ vg/kg, or about 1.5x10¹⁴ vg/kg to about 2.5x10¹⁴ vg/kg, or about 1.5x10¹⁴ vg/kg to about 2.0x10¹⁴ vg/kg, or 1.75x10¹⁴ vg/kg to about 1.0x10¹⁵ vg/kg, or about 1.75x10¹⁴ vg/kg to 6.0x10¹⁴, or about 1.75x10¹⁴ vg/kg to 5.0x10¹⁴, or about 1.75x10¹⁴ vg/kg to 4.0x10¹⁴, or about 1.75x10¹⁴ vg/kg to about 3.75x10¹⁴ vg/kg, or about 1.75x10¹⁴ vg/kg to about 3.5x10¹⁴ vg/kg, or about 1.75x10¹⁴ vg/kg to about 3.25x10¹⁴ vg/kg, or about 1.75x10¹⁴ vg/kg to about 3.0x10¹⁴ vg/kg, or about 1.75x10¹⁴ vg/kg to about 2.75x10¹⁴ vg/kg, or about 1.75x10¹⁴ vg/kg to about 2.5x10¹⁴ vg/kg, or about 1.75x10¹⁴ vg/kg to about 2.25x10¹⁴ vg/kg, or about 1.75x10¹⁴ vg/kg to about 2.0x10¹⁴ vg/kg, or about 2.0x10¹⁴ vg/kg to 1.0x10¹⁵, or about 2.0x10¹⁴ vg/kg to 6.0x10¹⁴, or about 2.0x10¹⁴ vg/kg to 5.0x10¹⁴, or about 2.0x10¹⁴ vg/kg to about 4.0x10¹⁴ vg/kg, or about 2.0x10¹⁴ vg/kg to about 3.75x10¹⁴ vg/kg, or about 2.0x10¹⁴ vg/kg to about 3.5x10¹⁴ vg/kg, or about 2.0x10¹⁴ vg/kg to about 3.25x10¹⁴ vg/kg.

The dose of rAAV can be administered at about 5 mL/kg to about 15 mL/kg, or about 8 mL/kg to about 12 mL/kg, or 8 mL/kg to about 10 mL/kg, or 5 mL/kg to about 10 mL/kg or about 10 mL/kg to 12 mL/k, or about 10 mL/kg to 15 mL/kg or 10 mL/kg to about 20 mL/kg. In a particular embodiment, the dose or the rAAV is administered in about 10 mL/kg.

The dose of rAAV can be administered by injection, infusion or implantation. For example, the dose of rAAV is administered by infusion over approximately one hour. In addition, the dose of rAAV is administered by an intravenous route through a peripheral limb vein, such as a peripheral arm vein or a peripheral leg vein. Alternatively, the infusion may be administered over approximately 30 minutes, or approximately 1.5 hours, or approximately 2 hours, or approximately 2.5 hours or approximately 3 hours.

The rAAV administered by any of the methods described herein can comprise the human micro-dystrophin nucleotide sequence of SEQ ID NO: 1, the MHCK7 promoter sequence of SEQ ID NO: 2 or SEQ ID NO:7. In addition, the rAAV administered by any of the methods described herein comprises the human micro-dystrophin nucleotide sequence of SEQ ID NO: 1 and the MHCK7 promoter sequence of SEQ ID NO: 2 or SEQ ID NO:7. For example, the rAAV can comprise the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6

The treatment may be of Duchenne muscular dystrophy or Becker's muscular dystrophy. An exemplary method of treating Duchenne muscular dystrophy or Becker's muscular dystrophy in a human subject in need thereof comprises the step of administering a dose recombinant adeno-virus associated (rAAV) rAAV.MHCK7.microdystrophin, wherein the route of administration is intravenous infusion and the dose of the rAAV administered is about 2x10¹⁴ vg/kg over approximately one hour, and wherein the rAAV vector comprises the AAVrh74.MHCK7.micro-dystrophin construct nucleotide sequence of SEQ ID NO: 9 or of nucleotides 55-5021 of SEQ ID NO: 3, nucleotides 1-4977 of SEQ ID NO: 8, or nucleotides 56-5022 of SEQ IDNO: 6.

The micro-dystrophin protein provides stability to the muscle membrane during muscle contraction, e.g. micro-dystrophin acts as a shock absorber during muscle contraction.

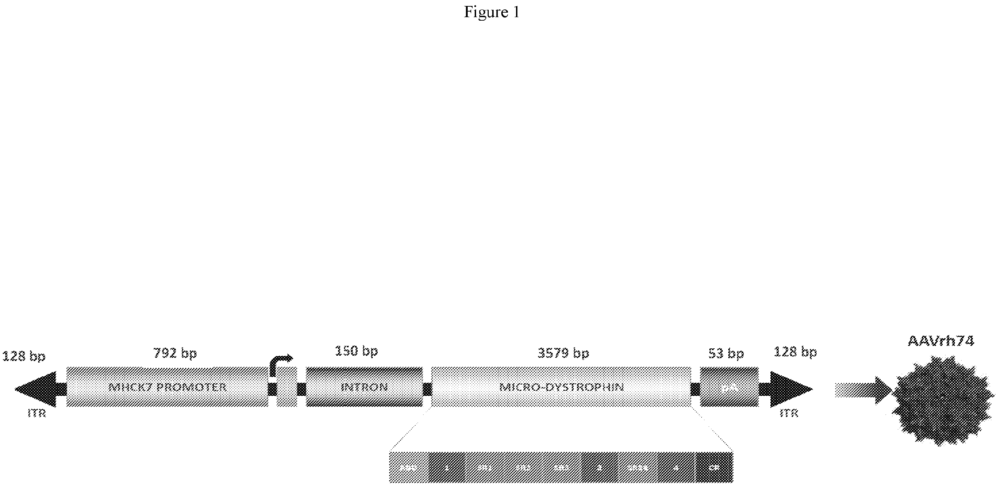

rAAV may be a non-replicating, recombinant adeno-associated virus (AAV) termed AAVrh74.MHCK7.micro-dystrophin of SEQ ID NO: 9, nucleotides 55-5021 of SEQ ID NO: 3, nucleotides 1-4977 of SEQ ID NO: 8, or nucleotides 56-5022 of SEQ IDNO: 6. This vector genome contains minimal elements required for gene expression, including AAV2 inverted terminal repeats (ITR), the micro-dystrophin, SV40 intron (SD/SA), and synthetic polyadenylation (Poly A) signal, all under the control of the MHCK7 promoter/enhancer. The schematic of the vector genome and expression cassette is shown Figure 1. The AAVrh74 serotype can be employed to achieve efficient gene transfer in skeletal and cardiac muscle following IV administration.

The term "stringent" is used to refer to conditions that are commonly understood in the art as stringent. Hybridization stringency is principally determined by temperature, ionic strength, and the concentration of denaturing agents such as formamide. Examples of stringent conditions for hybridization and washing are 0.015 M sodium chloride, 0.0015 M sodium citrate at 65-68°C or 0.015 M sodium chloride, 0.0015M sodium citrate, and 50% formamide at 42°C. See Sambrook et al., Molecular Cloning: A Laboratory Manual, 2nd Ed., Cold Spring Harbor Laboratory, (Cold Spring Harbor, N.Y. 1989). More stringent conditions (such as higher temperature, lower ionic strength, higher formamide, or other denaturing agent) may also be used, however, the rate of hybridization will be affected. In instances wherein hybridization of deoxyoligonucleotides is concerned, additional exemplary stringent hybridization conditions include washing in 6x SSC 0.05% sodium pyrophosphate at 37°C (for 14-base oligos), 48°C (for 17-base oligos), 55°C (for 20-base oligos), and 60°C (for 23-base oligos).

Other agents may be included in the hybridization and washing buffers for the purpose of reducing non-specific and/or background hybridization. Examples are 0.1% bovine serum albumin, 0.1% polyvinyl-pyrrolidone, 0.1% sodium pyrophosphate, 0.1% sodium dodecylsulfate, NaDodSO4, (SDS), ficoll, Denhardt's solution, sonicated salmon sperm DNA (or other non-complementary DNA), and dextran sulfate, although other suitable agents can also be used. The concentration and types of these additives can be changed without substantially affecting the stringency of the hybridization conditions. Hybridization experiments are usually carried out at pH 6.8-7.4, however, at typical ionic strength conditions, the rate of hybridization is nearly independent of pH. See Anderson et al., Nucleic Acid Hybridisation: A Practical Approach, Ch. 4, IRL Press Limited (Oxford, England). Hybridization conditions can be adjusted by one skilled in the art in order to accommodate these variables and allow DNAs of different sequence relatedness to form hybrids.

The term "muscle specific control element" refers to a nucleotide sequence that regulates expression of a coding sequence that is specific for expression in muscle tissue. These control elements include enhancers and promoters, such as the muscle specific control elements MCKH7 promoter, the MCK promoter and the MCK enhancer.

The term "operably linked" refers to the positioning of the regulatory element nucleotide sequence, e.g. promoter nucleotide sequence, to confer expression of said nucleotide sequence by said regulatory element.

Muscle specific control elements may be a human skeletal actin gene element, cardiac actin gene element, myocyte-specific enhancer binding factor (MEF), muscle creatine kinase (MCK), truncated MCK (tMCK), myosin heavy chain (MHC), hybrid α-myosin heavy chain enhancer-/MCK enhancer-promoter (MHCK7), C5-12, murine creatine kinase enhancer element, skeletal fast-twitch troponin c gene element, slow-twitch cardiac troponin c gene element, the slow-twitch troponin i gene element, hypoxia-inducible nuclear factors, steroid-inducible element or glucocorticoid response element (GRE).

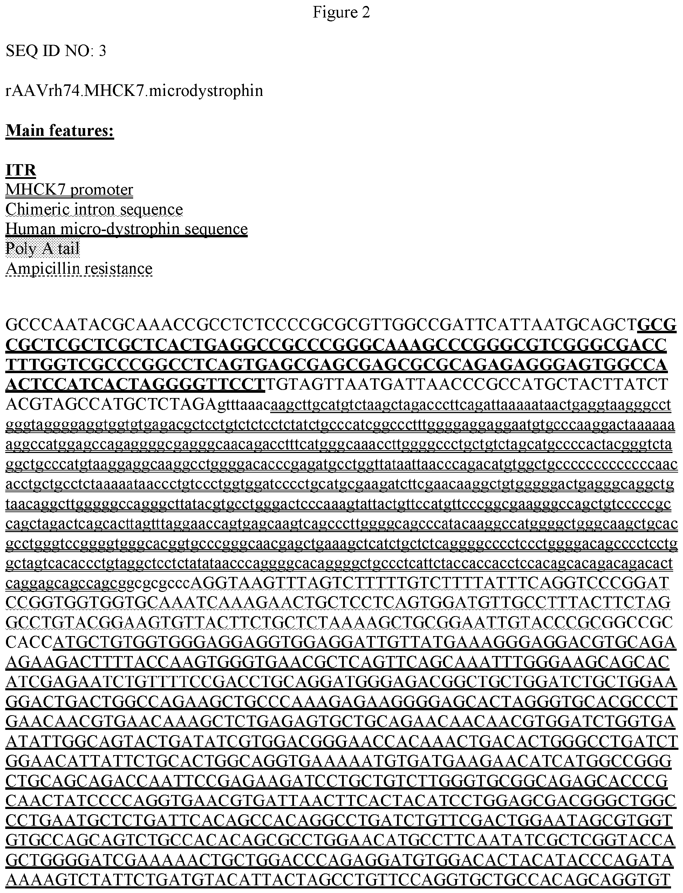

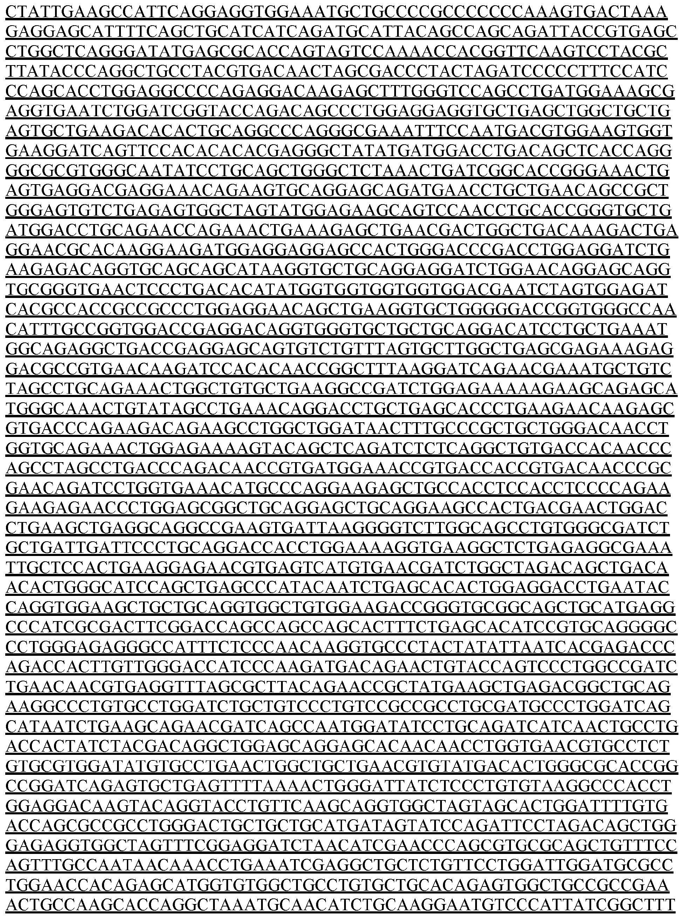

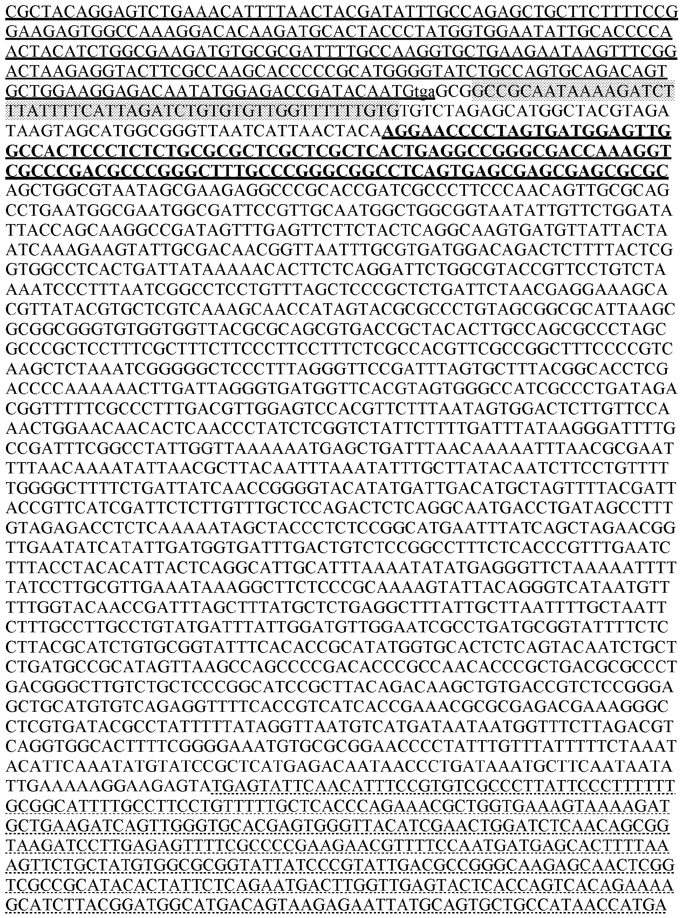

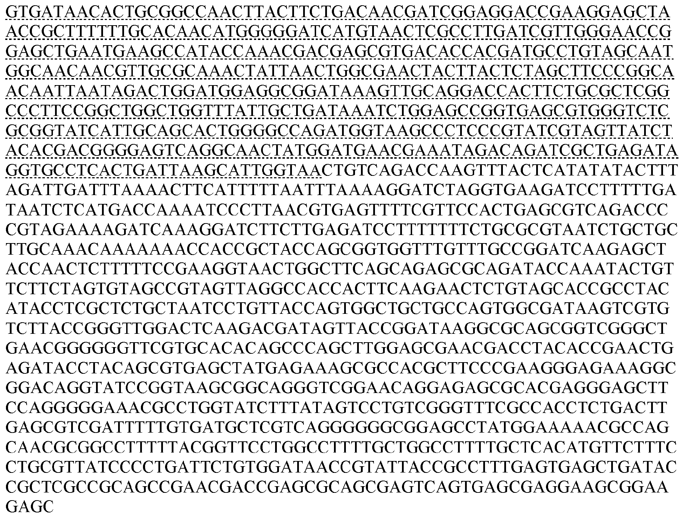

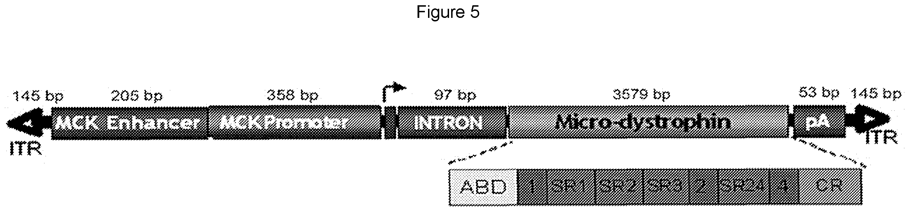

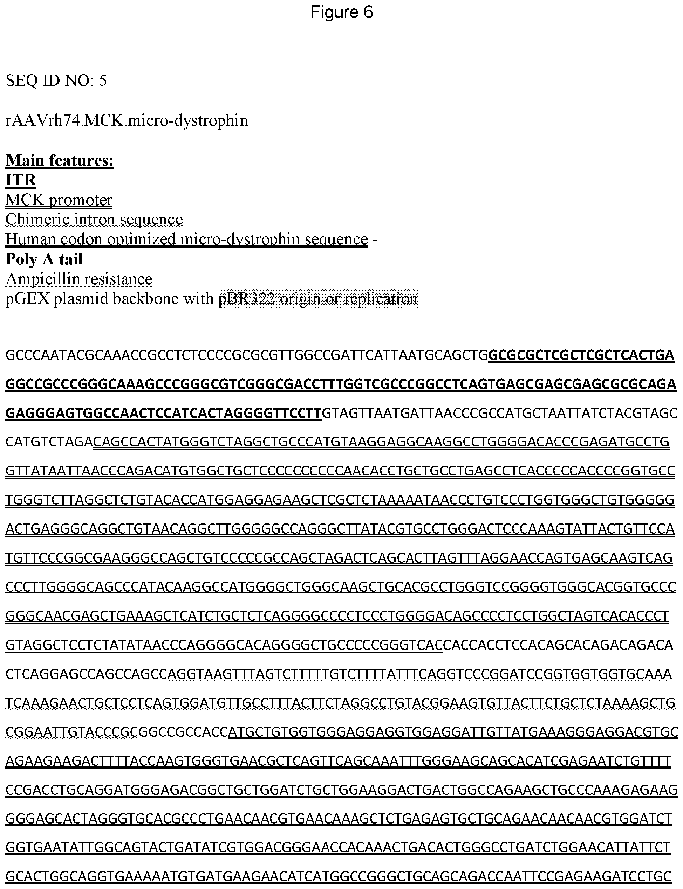

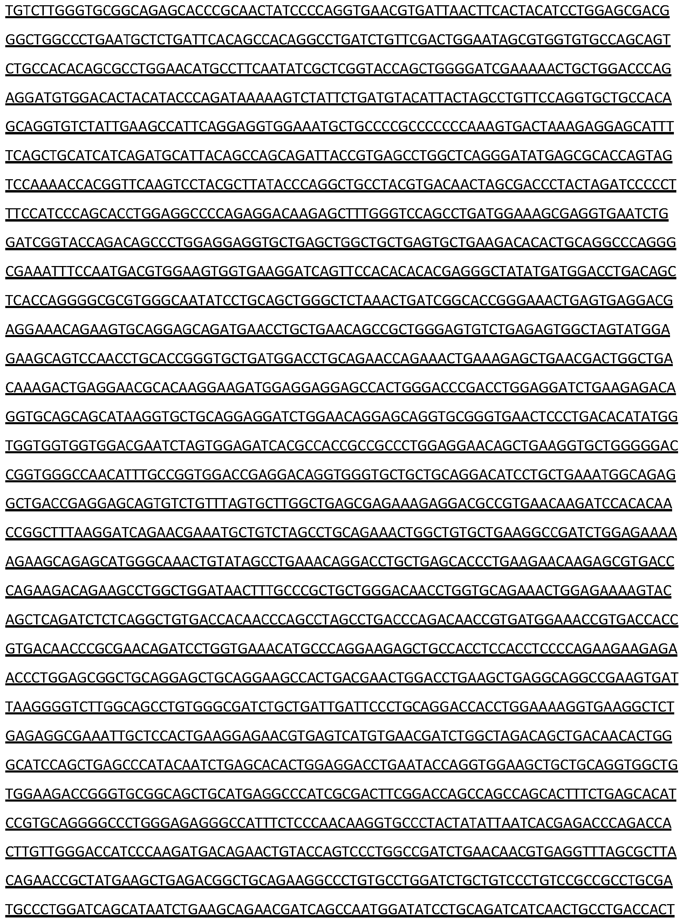

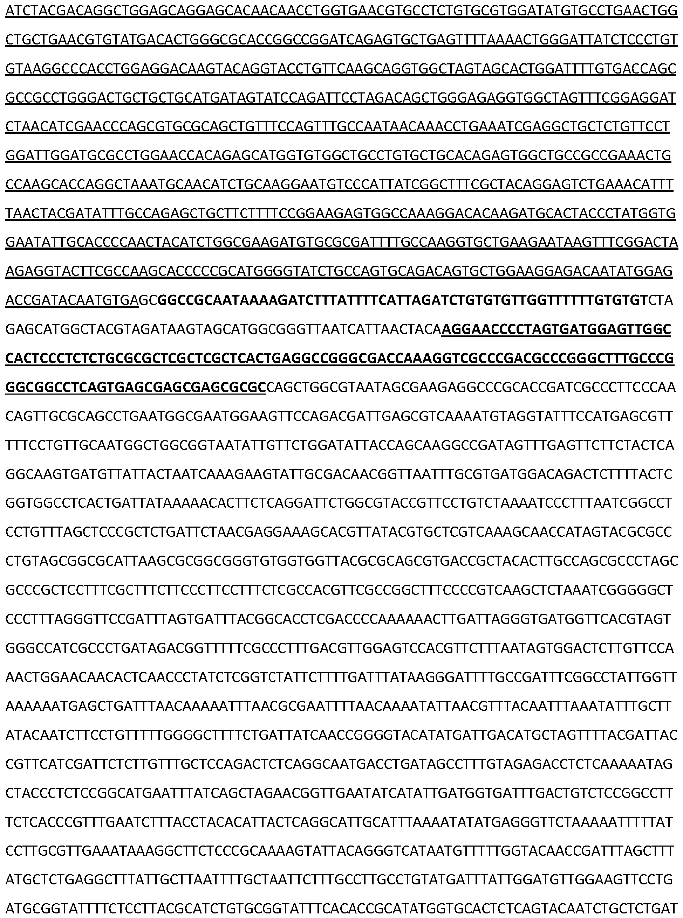

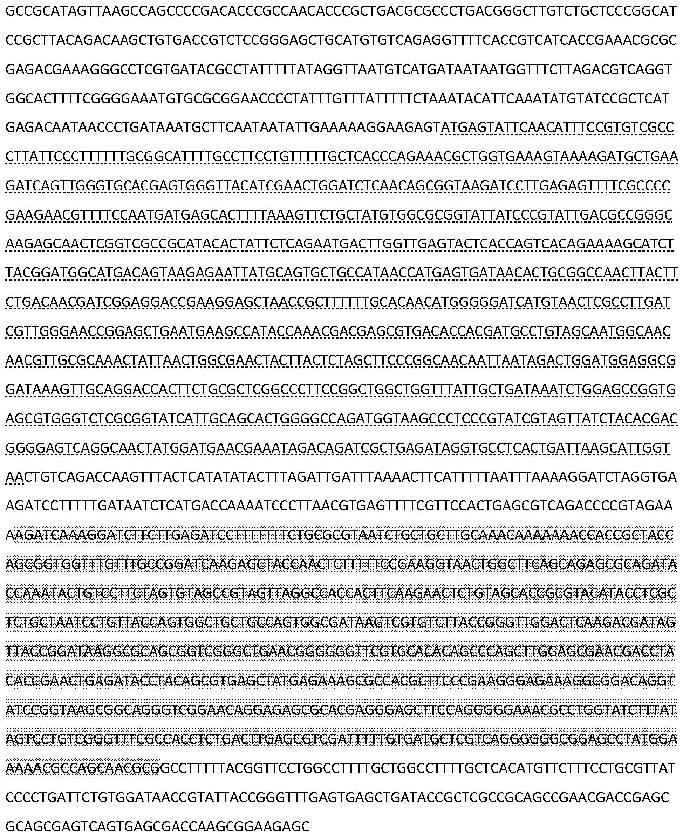

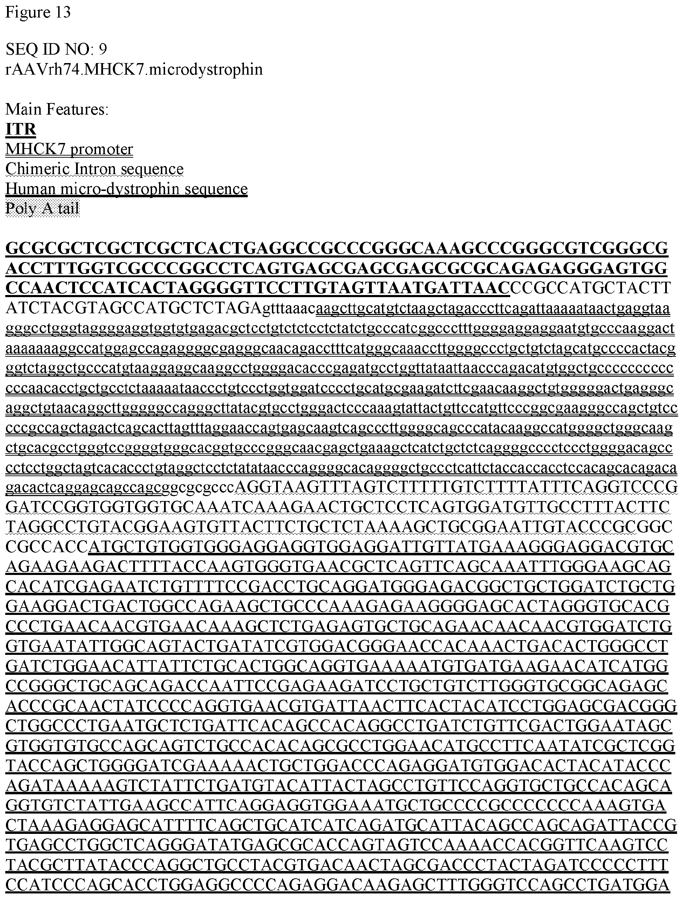

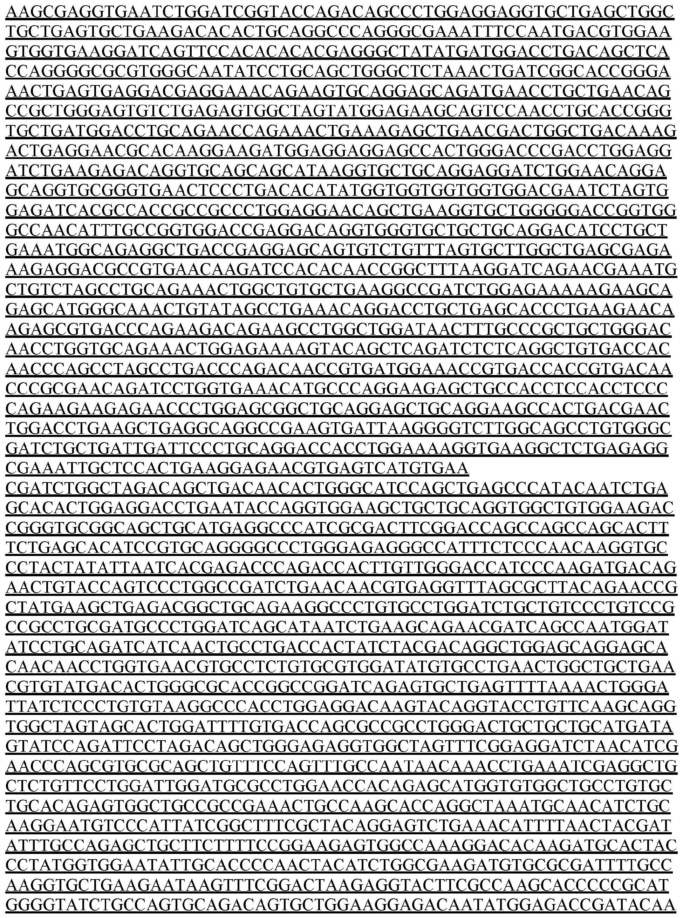

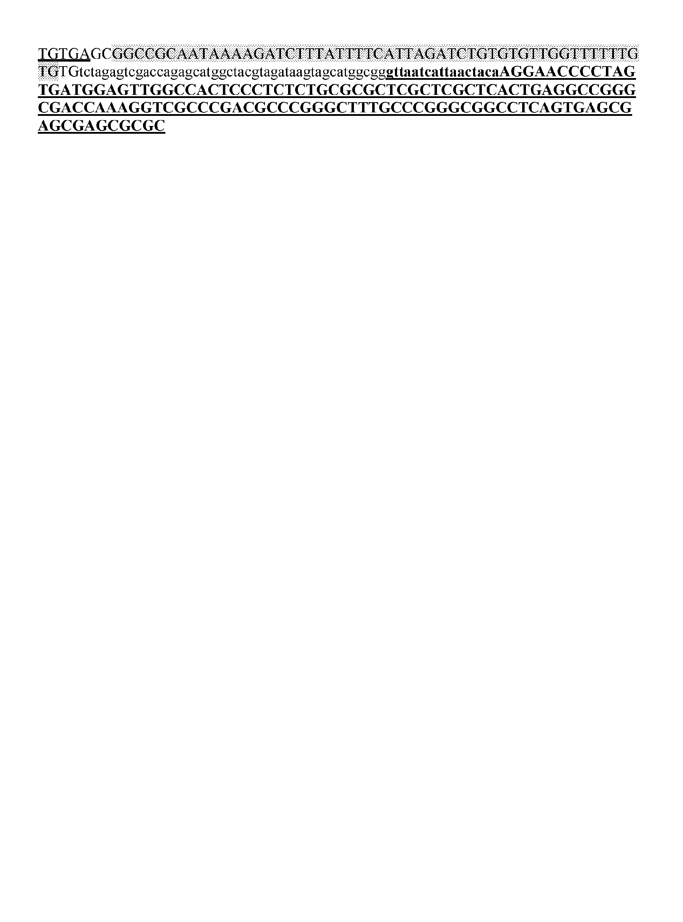

For example, the muscle specific control element may be the MHCK7 promoter nucleotide sequence SEQ ID NO: 2 or SEQ ID NO: 7, or the muscle specific control element is MCK nucleotide sequence SEQ ID NO: 4. In addition, in any of the rAAV vectors, the muscle specific control element nucleotide sequence, e.g. MHCK7 or MCK nucleotide sequence, is operably linked to the nucleotide sequence encoding the micro-dystrophin protein. For example, the MHCK7 promoter nucleotide sequence (SEQ ID NO: 2 or SEQ ID NO: 7) is operably linked to the human micro-dystrophin coding sequence (SEQ ID NO: 1) as set out in the construct provided in Figure 1 or Figure 2 (SEQ ID NO: 3) or Figure 13 (SEQ ID NO: 9). In another example, the MCK promoter (SEQ ID NO: 4) is operably linked to the human micro-dystrophin coding sequence (SEQ ID NO: 1) as set out in the construct provided in Figure 5 or Figure 6 (SEQ ID NO: 5).

In another aspect, the invention provides for a rAAV comprising the nucleotide sequence of SEQ ID NO: 9. For example, the AAVrh74.MHCK7.microdystrophin vector construct comprises the nucleotide sequence of SEQ ID NO: 9 and shown in Figure 13. This rAAV vector construct comprises the MHCK7 promoter, a chimeric intron sequence, the coding sequence for the human micro-dystrophin gene, and polyA. In one embodiment, the rAAV vector construct further comprises an ITR 5' to the promoter, and an ITR 3' to the polyA.

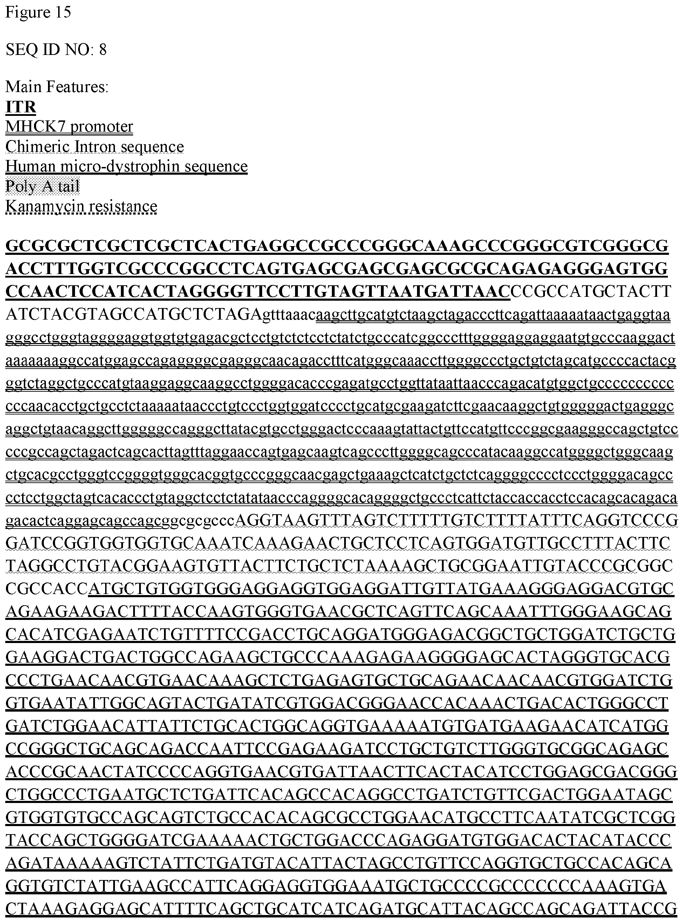

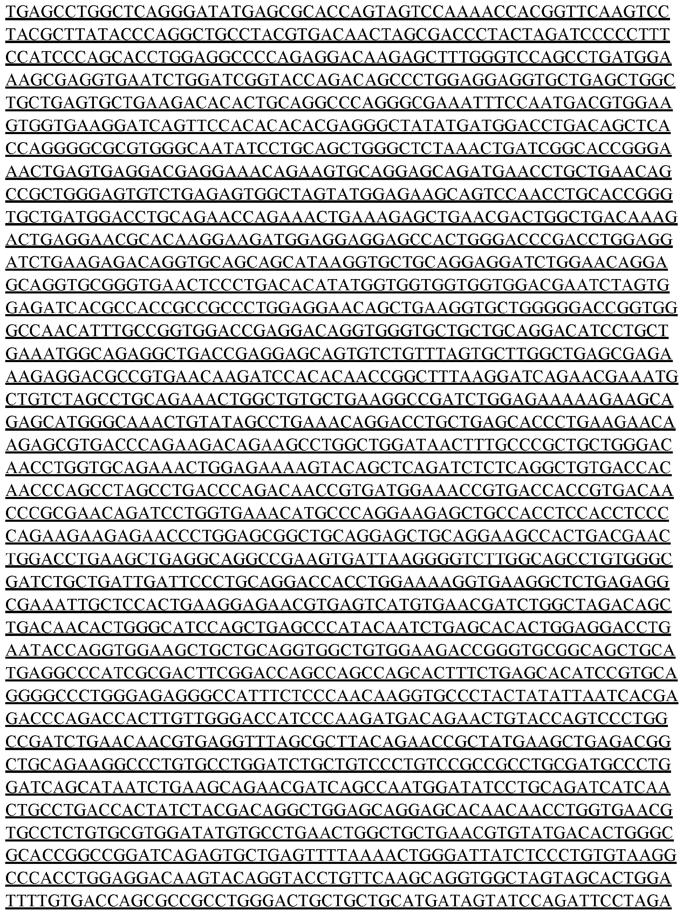

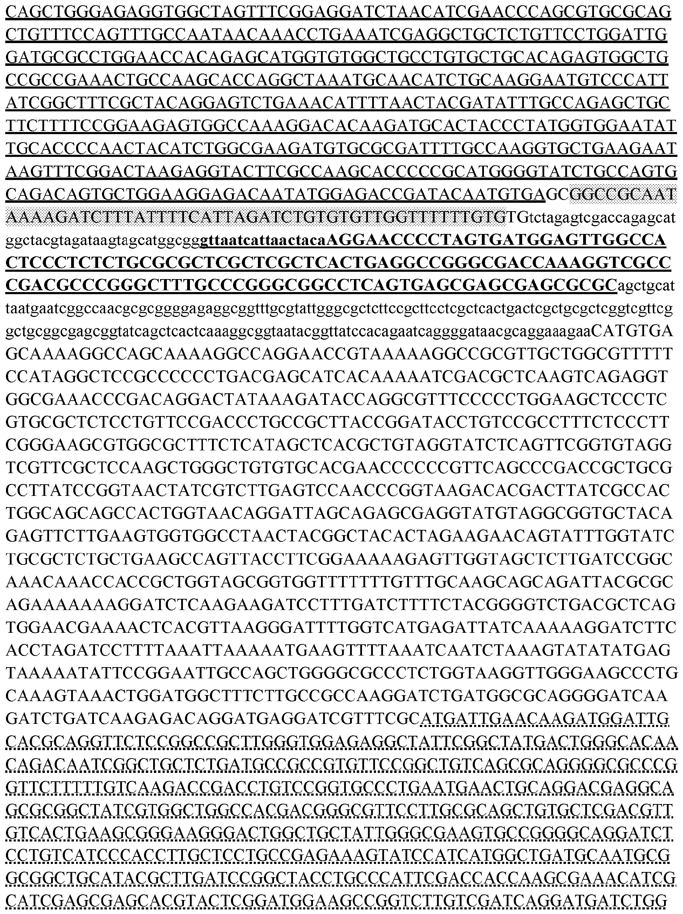

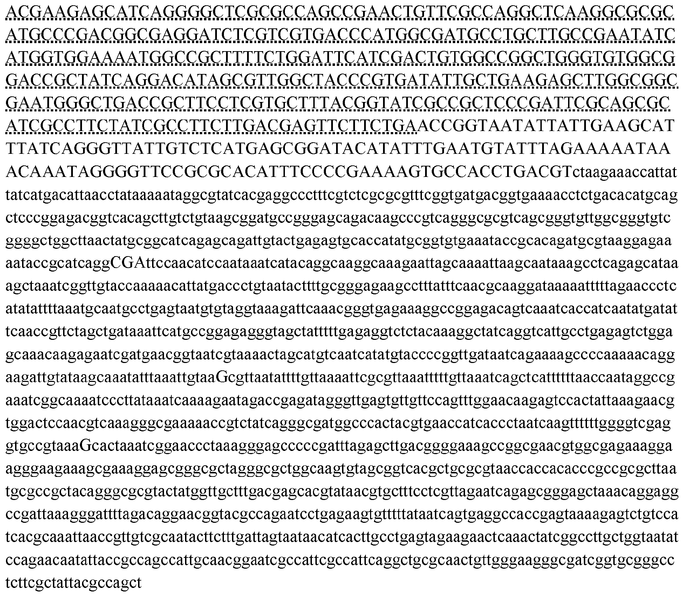

The AAVrh74.MHCK7.microdystrophin vector may comprise the nucleotide sequence within and inclusive of the ITR's of SEQ ID NO: 8 and shown in Figure 15. The rAAV vector comprises the 5' ITR, MHCK7 promoter, a chimeric intron sequence, the coding sequence for the human micro-dystrophin gene, polyA, and 3' ITR. The plasmid set forth in SEQ ID NO:3 further comprises kanamycin resistance and the pGEX plasmid backbone with pBR322 origin of replication.

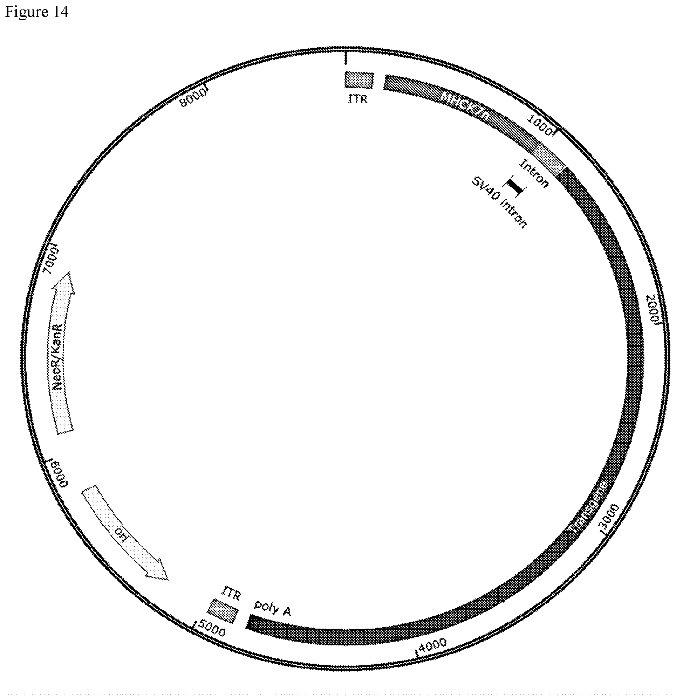

A plasmid may comprise the AAVrh74.MHCK7.microdystrophin vector construct. The plasmid may comprise the 5' ITR, MHCK7 promoter, a chimeric intron sequence, the coding sequence for the human micro-dystrophin gene, polyA, and 3' ITR. The plasmid may comprise kanamycin resistance and optionally comprise the pGEX plasmid backbone with pBR322 origin of replication. The plasmid nay be as set forth in SEQ ID NO:8, and shown in Figures 14 and 15.

A recombinant AAV vector may comprise the human micro-dystrophin nucleotide sequence of SEQ ID NO: 1 and the MHCK7 promoter nucleotide sequence of SEQ ID NO: 2 or SEQ ID NO:7. This rAAV vector is the AAV serotype AAVrh.74.

A rAAV may comprise the AAVrh74.MHCK7.micro-dystrophin construct nucleotide sequence within and inclusive of the ITR's in SEQ ID NO: 3, the nucleotide sequence within and inclusive of the ITR's in SEQ ID NO: 8 or the nucleotide sequence as set forth in SEQ ID NO: 9. This rAAV vector is the AAV serotype AAVrh.74.

rAAV vectors may be any AAV serotype, such as the serotype AAVrh.74, AAV1, AAV2, AAV4, AAV5, AAV6, AAV7, AAV8, AAV9, AAV10, AAV11, AAV12 or AAV13.

Pharmaceutical compositions (or sometimes referred to herein as simply "compositions") may comprise any of the rAAV vectors.

In any of the methods of treating a muscular dystrophy, the level of micro-dystrophin gene expression in a cell of the subject is increased after administration of the rAAV. Expression of the micro-dystrophin gene in the cell is detected by measuring the micro-dystrophin protein level by Western blot in muscle biopsied before and after administration of the rAAV. In particular, the level of micro-dystrophin protein is increased by at least about 70% to at least about 80%, or at least about 70% to at least about 90%, or at least about 80% to at least about 90% after administration of rAAV compared to the level of micro-dystrophin before administration of rAAV. For example, the level of micro-dystrophin protein is increased by at least about 70% or at least about 71% or at least about 72% or at least about 73% or at least about 74% or at least about 75% or at least about 76% or at least about 77% or at least about 78% or at least about 79% or at least about 80%, or at least about 81%, or at least about 82%, or at least about 83%, or at least about 84%, or at least about 85% after administration of rAAV compared to the level of micro-dystrophin before administration of rAAV.

In addition, expression of the micro-dystrophin gene in the cell is detected by measuring the micro-dystrophin protein level by immunohistochemistry in muscle biopsies before and after administration of the rAAV. The level of micro-dystrophin protein is increased by at least about 70% to at least about 80%, or at least about 70% to at least about 90%, or at least about 80% to at least about 90% after administration of rAAV compared to the level of micro-dystrophin before administration of rAAV. For example, the level of micro-dystrophin protein is increased by at least about 70% or at least about 71% or at least about 72% or at least about 73% or at least about 74% or at least about 75% or at least about 76% or at least about 77% or at least about 78% or at least about 79% or at least about 80%, or at least about 81%, or at least about 82%, or at least about 83%, or at least about 84%, or at least about 85% after administration of rAAV compared to the level of micro-dystrophin before administration of rAAV.

In any of the methods of treating a muscular dystrophy, the serum CK level in the subject is decreased after administration of the rAAV as compared to serum CK level before administration of the rAAV. For example, the serum CK level in the subject is decreased by about 65 % to about 90% or about 65% to about 95% or about 75% to about 90% or about 80% to about 90% or about 85% to about 95% or about 87% to about 95% or about 87% to about 90% by 60 days after administration of the rAAV as compared to the serum CK level before administration of the rAAV. In particular, in any of the methods of treating a muscular dystrophy, the serum CK level in the subject is decreased by about 87% by 60 days after administration of the rAAV as compared to the serum CK level before administration of the rAAV or in any of the methods of treating a muscular dystrophy, the serum CK level in the subject is decreased by about 72% by 60 days after administration of the rAAV as compared to the serum CK level before administration of the rAAV, or in any of the methods of treating a muscular dystrophy, the serum CK level in the subject is decreased by about 73% by 60 days after administration of the rAAV as compared to the serum CK level before administration of the rAAV, or in any of the methods of treating a muscular dystrophy, the serum CK level in the subject is decreased by about 78% by 60 days after administration of the rAAV as compared to the serum CK level before administration of the rAAV. or in any of the methods of treating a muscular dystrophy, the serum CK level in the subject is decreased by about 95% by 60 days after administration of the rAAV as compared to the serum CK level before administration of the rAAV. In any of the methods of treating a muscular dystrophy, the number of micro-dystrophin positive fibers in the muscle tissue of the subject is increased after administration of the rAAV as compared to the number of micro-dystrophin positive fibers before administration of the rAAV. For example, the number of micro-dystrophin positive fibers is detected by measuring the micro-dystrophin protein level by Western blot or immunohistochemistry on muscle biopsies before and after administration of the rAAV.

In any of the methods of treating a muscular dystrophy, administration of the rAAV upregulates expression of DAPC proteins such as alpha-sarcoglycan or beta-sarcoglycan. For example, the level of alpha-sarcoglycan in the subject is increased after administration of the rAAV as compared to the level of alpha-sarcoglycan before administration of the rAAV. In addition, the level of beta-sarcoglycan in the subject is increased after administration of the rAAV as compared to the level of the beta-sarcoglycan before administration of the rAAV. The level of alpha-sarcoglycan or beta-sarcoglycan is detected by measuring the alpha-sarcoglycan or beta-sarcoglycan protein level by Western blot or immunohistochemistry on muscle biopsies before and after administration of the rAAV.

In any of the methods of the treating a muscular dystrophy, disease progression in the subject is delayed after administration of the rAAV as measured by any of: the six minute walk test, time to rise, ascend 4 steps, ascend and descend 4 steps, North Star Ambulatory Assessment (NSAA), 10 meter timed test, 100 meter timed test, hand held dynamometry (HHD), Timed Up and Go, and/or Gross Motor Subtest Scaled (Bayley-III) score.

For example, in any of the methods, the subject has at least a 6-point improvement in NSAA score at least 270 days after administration of the rAAV as compared to NSAA score before administration of the rAAV. Further, in any of the methods, the subject has at least about 0.8 second improvement in time to rise at least 270 days after administration of the rAAV as compared to time to rise before administration of the rAAV. In addition, in any of the methods, the subject has at least about 1.2 second improvement in time to ascend 4 steps test at least 270 days after administration of the rAAV as compared to time to ascend 4 steps test before administration of the rAAV. In addition, in any of the methods, the subject has at least about 7 second improvement in the 100 m timed test at least 270 days after administration of the rAAV as compared to the 100 m timed test before administration of the rAAV.

Methods of expressing micro-dystrophin gene in a patient cell may comprise administering to the patient the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8 or nucleotides 56-5022 of SEQ ID NO: 6. For example, expression of the micro-dystrophin gene in the patient cell is detected by measuring the micro-dystrophin protein level by Western blot or immunohistochemistry in muscle biopsies before and after administration of the rAAV.MHCK7.micro-dystrophin construct. In addition, the expression of the micro-dystrophin gene is measured in the patient by detecting greater the number of vector genomes per nucleus, wherein 1vector genome per nucleus is about 50% micro-dystrophin expression and great than 1 copy per nucleus is consistent with micro-dystrophin expression level. For example, the cells have 1.2 vector copies per nucleus, or 1.3 vector copies per nucleus, or 1.4 vector copies per nucleus, or 1.5 vector copies per nucleus, or 1.6 vector copies per nucleus, or 1.7 vector copies per nucleus, or 1.8 vector copies per nucleus, or 1.9 vector copies per nucleus.

Methods of decreasing serum CK levels in a patient in need thereof may comprise administering to the patient the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. For example, the serum CK level in the patient is decreased by at least about 65% to about 90% or about 65% to about 95% or about 75% to about 90% or about 80% to about 90% or about 85% to about 95% or about 87% to about 95% or about 87% to about 90% by 60 days after administration of the rAAV as compared to the serum CK level before administration of the rAAV. In particular, the serum CK level in the subject is decreased by about 87% by 60 days after administration of the rAAV as compared to the serum CK level before administration of the rAAV or in any of the methods of treating a muscular dystrophy, the serum CK level in the subject is decreased by about 72% by 60 days after administration of the rAAV as compared to the serum CK level before administration of the rAAV, or in any of the methods of treating a muscular dystrophy, the serum CK level in the subject is decreased by about 73% by 60 days after administration of the rAAV as compared to the serum CK level before administration of the rAAV, or in any of the methods of treating a muscular dystrophy, the serum CK level in the subject is decreased by about 78% by 60 days after administration of the rAAV as compared to the serum CK level before administration of the rAAV, or in any of the methods of treating a muscular dystrophy, the serum CK level in the subject is decreased by about 95% by 60 days after administration of the rAAV as compared to the serum CK level before administration of the rAAV.

Methods of increasing micro-dystrophin positive fibers in a patient muscle tissue may comprise administering to the patient the AAVrh74.MHCK7.microdystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. For example, the number of micro-dystrophin positive fibers is detected by measuring the dystrophin protein level by Western blot or immunohistochemistry on muscle biopsies before and after administration of the rAAV. In addition, the expression of the micro-dystrophin gene is measured in the patient by detecting greater the number of vector genomes per nucleus, wherein 1 vector genome per nucleus is about 50% micro-dystrophin expression and great than 1 copy per nucleus is consistent with micro-dystrophin expression level. For example, the cells have 1.2 vector copies per nucleus, or 1.3 vector copies per nucleus, or 1.4 vector copies per nucleus, or 1.5 vector copies per nucleus, or 1.6 vector copies per nucleus, or 1.7 vector copies per nucleus, or 1.8 vector copies per nucleus, or 1.9 vector copies per nucleus.

Methods of increasing the expression of alpha-sarcoglycan in a patient in need thereof may comprise administering to the patient the AAVrh74.MHCK7.microdystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. For example, the level of alpha-sarcoglycan is detected by measuring the alpha-sarcoglycan protein level by Western blot or immunohistochemistry on muscle biopsies before and after administration of the rAAV.

Methods of increasing the expression of beta-sarcoglycan in a patient in need thereof may comprise administering to the patient the AAVrh74.MHCK7.microdystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. For example, the level of beta-sarcoglycan is detected by measuring the beta-sarcoglycan protein level by Western blot or immunohistochemistry on muscle biopsies before and after administration of the rAAV.

Methods of treating a patient with Duchenne muscular dystrophy or Becker muscular dystrophy may comprise administering to the patient the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6, such that disease progression in the patient is delayed as measured by any of: the six minute walk test, time to rise, ascend 4 steps, ascend and descend 4 steps, North Star Ambulatory Assessment (NSAA), 10 meter timed test, 100 meter timed test, hand held dynamometry (HHD), Timed Up and Go, and/or Gross Motor Subtest Scaled (Bayley-III) score.

For example, in any of the methods, the subject has at least a 6-point improvement in NSAA score at least 270 days after administration of the rAAV as compared to NSAA score before administration of the rAAV. Further, in any of the methods, the subject has at least about 0.8 second improvement in time to rise at least 270 days after administration of the rAAV as compared to time to rise before administration of the rAAV. In addition, in any of the methods, the subject has at least about 1.2 second improvement in time to ascend 4 steps test at least 270 days after administration of the rAAV as compared to time to ascend 4 steps test before administration of the rAAV. In addition, in any of the methods, the subject has at least about 7 second improvement in the 100 m timed test at least 270 days after administration of the rAAV as compared to the 100 m timed test before administration of the rAAV.

"Fibrosis" refers to the excessive or unregulated deposition of extracellular matrix (ECM) components and abnormal repair processes in tissues upon injury, including skeletal muscle, cardiac muscle, liver, lung, kidney, and pancreas. The ECM components that are deposited include fibronectin and collagen, e.g. collagen 1, collagen 2 or collagen 3.

Methods of reducing or preventing fibrosis in a subject suffering from muscular dystrophy may comprise administering a therapeutically effective amount of a rAAV comprising the human micro-dystrophin nucleotide sequence of SEQ ID NO: 1 and the MHCK7 promoter nucleotide sequence of SEQ ID NO: 2 or SEQ ID NO: 7; or a rAAV vector comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. The rAAV may be AAVrh74.MHCK7.microdystrophin. The AAVrh74.MHCK7.microdystrophin may be the AAVrh74.MHCK7.microdystrophin of nucleotides 55-5021 of SEQ ID NO: 3. The AAVrh74.MHCK7.microdystrophin may be the AAVrh74.MHCK7.microdystrophin of SEQ ID NO: 9. The AAVrh74.MHCK7.microdystrophin may be the AAVrh74.MHCK7.microdystrophin of nucleotides 1-4977 of SEQ ID NO: 8 or nucleotides 56-5066 of SEQ ID NO: 6. The rAAV may be AAVrh74.MCK.microdystrophin. The AAVrh74.MCK.microdystrophin may be the AAVrh74.MCK.microdystrophin of nucleotides 56-4820 of SEQ ID NO: 5.

Methods of preventing fibrosis in a subject in need thereof, may comprise administering a therapeutically effective amount of the human micro-dystrophin nucleotide sequence of SEQ ID NO: 1 and the MHCK7 promoter nucleotide sequence of SEQ ID NO: 2 or SEQ ID NO:7; or rAAV vector comprising the AAV74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. For example, any of the rAAV of the invention can be administered to subjects suffering from muscular dystrophy to prevent fibrosis, e.g. the rAAV of the invention expressing a human micro-dystrophin protein administered before fibrosis is observed in the subject. In addition, the rAAV of the invention expressing a human micro-dystrophin gene can be administered to a subject at risk of developing fibrosis, such as those suffering or diagnosed with muscular dystrophy, e.g. DMD. The rAAV of the invention can be administered to the subject suffering from muscular dystrophy in order to prevent new fibrosis in these subjects.

The rAAV may be administered before fibrosis is observed in the subject. In addition, the rAAV can be administered to a subject at risk of developing fibrosis, such as those suffering or diagnosed with a muscular dystrophy, e.g. DMD. The rAAV can be administered to the subject suffering from muscular dystrophy who already has developed fibrosis in order to prevent new fibrosis in these subjects.

Methods of increasing muscular force and/or muscle mass in a subject suffering from a muscular dystrophy may comprise administering a therapeutically effective amount of the human micro-dystrophin nucleotide sequence of SEQ ID NO: 1 and the MHCK7 promoter nucleotide sequence of SEQ ID NO: 2 or SEQ ID NO: 7; or a rAAV comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6.

The rAAV vectors may be administered to subjects diagnosed with DMD before fibrosis is observed in the subject or before the muscle force has been reduced or before the muscle mass has been reduced.

The human micro-dystrophin nucleotide sequence of SEQ ID NO: 1 and the MHCK7 promoter nucleotide sequence of SEQ ID NO: 2 or SEQ ID NO:7; or a rAAV comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 may be administered to a subject suffering from a muscular dystrophy who already has developed fibrosis, in order to prevent new fibrosis in these subjects or to reduce fibrosis in these subjects. The human micro-dystrophin nucleotide sequence of SEQ ID NO: 1 and the MHCK7 promoter nucleotide sequence of SEQ ID NO: 2 or SEQ ID NO:7; or a rAAV vector comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 may be administered to the subject suffering from a muscular dystrophy who already has reduced muscle force or has reduced muscle mass in order to protect the muscle from further injury.

In any of the methods, the subject may be suffering from a muscular dystrophy such as DMD or any other dystrophin-associated muscular dystrophy.

In any of the methods described herein, the serum CK level in the subject is decreased after administration of the rAAV as compared to the serum CK level before administration of the rAAV by a percentage level selected from the group consisting of:
a) at least 78% by 90, 180, or 270 days after the administration;
b) at least 46, 55, 56, 57, 58, 59, 60, 61, 62, 63, 64, 65, 66, 67, 68, 69, 70 or 85% by 270 days after the administration;
c) at least 72, 73, 74, or 95% by 180 days after the administration;
d) at least 87, 88, 93 or 95% by 90 days after the administration;
e) at least 70 % by 270 days after the administration;
f) 70 to 95% by 90, 180, or 270 days after the administration;
g) at least 56, 57, 58, 59, 60, 61, 62, 63, 64, 65, 66, 67, 68, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration; and
h) at least 56, 57, 58, 59, 60, 61, 62, 63, 64, 65, 66, 67, 68, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration.

Compositions for treating a muscular dystrophy in a human subject in need, may comprise a dose of recombinant adeno-virus associated (rAAV) rAAV.MHCK7.microdystrophin, wherein composition is formulated for a systemic route of administration and the dose of the rAAV is about 1x10¹⁴ vg/kg to about 4x 10¹⁴ vg/kg. The rAAV may be AAVrh74.MHCK7.microdystrophin. The AAVrh74.MHCK7.microdystrophin may be the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ IDNO: 6. The rAAV may be AAVrh74.MCK.microdystrophin. The AAVrh74.MCK.microdystrophin may be the AAVrh74.MCK.microdystrophin of nucleotides 56-4820 of SEQ ID NO: 5.

For example, the composition may comprise a dose of rAAV of about 5.0x10¹² vg/kg to about 1.0x10¹⁴ vg/kg, or about 5.0x10¹² vg/kg to1.0x10¹⁴ vg/kg, or about 5.0x10¹² vg/kg to about 2.0x10¹⁴ vg/kg, or about 5.0x10¹² vg/kg to about 1.0x10¹⁴ vg/kg, or about 5.0x10¹² vg/kg to about 5.0x10¹³ vg/kg, or about 5.0x10¹² vg/kg to about 2.0x10¹³ vg/kg, or about 5.0x10¹² vg/kg to about 1.0x10¹³ vg/kg, or 1.0x10¹⁴ vg/kg to about 1.0x10¹⁵ vg/kg, or 1.0x10¹³ vg/kg to about 1.0x10¹⁴ vg/kg, or about 1.0x10¹³ vg/kg to1.0x10¹⁴ vg/kg, or about 1.0x10¹³ vg/kg to about 2.0x10¹⁴ vg/kg, or about 1.0x10¹³ vg/kg to about 1.0x10¹⁴ vg/kg, or about 1.0x10¹³ vg/kg to about 5.0x10¹³ vg/kg, or about 1.0x10¹³ vg/kg to about 3.0x10¹⁴vg/kg, or about 1.0x10¹³ vg/kg to about 5.0x10¹⁴ vg/kg, or about 1.0x10¹³ vg/kg to about 6.0x10¹⁴ vg/kg, or 1.0x10¹³ vg/kg to about 1.0x10¹⁵ vg/kg, or 5.0x10¹³ vg/kg to about 1.0x10¹⁴ vg/kg, or about 5.0x10¹³ vg/kg to1.0x10¹⁴ vg/kg, or about 5.0x10¹³ vg/kg to about 2.0x10¹⁴ vg/kg, or about 5.0x10¹³ vg/kg to about 1.0x10¹⁴ vg/kg, or about 5.0x10¹³ vg/kg to about 3.0x10¹⁴ vg/kg, or about 5.0x10¹³ vg/kg to about 5.0x10¹⁴ vg/kg, or about 5.0x10¹³ vg/kg to about 6.0x10¹⁴ vg/kg, or 5.0x10¹³ vg/kg to about 1.0x10¹⁵ vg/kg, or 1.0x10¹⁴ vg/kg to about 6.0x10¹⁴ vg/kg, or 1.0x10¹⁴ vg/kg to about 5.0x10¹⁴ vg/kg, or 1.0x10¹⁴ vg/kg to about 4.0x10¹⁴ vg/kg, or 1.0x10¹⁴ vg/kg to about 1.0x10¹⁵ vg/kg, or 1.0x10¹⁴ vg/kg to about 3.0x10¹⁴ vg/kg, or about 1.0x10¹⁴ vg/kg to about 2.5x10¹⁴ vg/kg, or 1.0x10¹⁴ vg/kg to about 2.0x10¹⁴ vg/kg, or about 1.25x10¹⁴ vg/kg to about 3.75x10¹⁴ vg/kg, or about 1.25x10¹⁴ vg/kg to 6.0x10¹⁴, or about 1.25x10¹⁴ vg/kg to 5.0x10¹⁴, or about 1.25x10¹⁴ vg/kg to 4.0x10¹⁴, or about 1.25x10¹⁴ vg/kg to 1.0x10¹⁵, or about 1.25x10¹⁴ vg/kg to about 3.5x10¹⁴ vg/kg, or about 1.25x10¹⁴ vg/kg to about 3.0x10¹⁴ vg/kg, or about 1.25x10¹⁴ vg/kg to about 2.75x10¹⁴ vg/kg, or about 1.25x10¹⁴ vg/kg to about 2.5x10¹⁴ vg/kg, or about 1.25x10¹⁴ vg/kg to about 2.0x10¹⁴ vg/kg, or 1.25x10¹⁴ vg/kg to about 3.75x10¹⁴ vg/kg, or about 1.25x10¹⁴ vg/kg to about 3.5x10¹⁴ vg/kg, or 1.5x10¹⁴ vg/kg to about 1.0x10¹⁵ vg/kg, or about 1.5x10¹⁴ vg/kg to 6.0x10¹⁴, or about 1.5x10¹⁴ vg/kg to 5.0x10¹⁴, or about 1.5x10¹⁴ vg/kg to 4.0x10¹⁴, or about 1.5x10¹⁴ vg/kg to about 3.75x10¹⁴ vg/kg, or about 1.5x10¹⁴ vg/kg to about 3.5x10¹⁴ vg/kg, or about 1.5x10¹⁴ vg/kg to about 3.25x10¹⁴ vg/kg, or about 1.5x10¹⁴ vg/kg to about 3.0x10¹⁴ vg/kg, or about 1.5x10¹⁴ vg/kg to about 2.75x10¹⁴ vg/kg, or about 1.5x10¹⁴ vg/kg to about 2.5x10¹⁴ vg/kg, or about 1.5x10¹⁴ vg/kg to about 2.0x10¹⁴ vg/kg, or 1.75x10¹⁴ vg/kg to about 1.0x10¹⁵ vg/kg, or about 1.75x10¹⁴ vg/kg to 6.0x10¹⁴, or about 1.75x10¹⁴ vg/kg to 5.0x10¹⁴, or about 1.75x10¹⁴ vg/kg to 4.0x10¹⁴, or about 1.75x10¹⁴ vg/kg to about 3.75x10¹⁴ vg/kg, or about 1.75x10¹⁴ vg/kg to about 3.5x10¹⁴ vg/kg, or about 1.75x10¹⁴ vg/kg to about 3.25x10¹⁴ vg/kg, or about 1.75x10¹⁴ vg/kg to about 3.0x10¹⁴ vg/kg, or about 1.75x10¹⁴ vg/kg to about 2.75x10¹⁴ vg/kg, or about 1.75x10¹⁴ vg/kg to about 2.5x10¹⁴ vg/kg, or about 1.75x10¹⁴ vg/kg to about 2.25x10¹⁴ vg/kg, or about 1.75x10¹⁴ vg/kg to about 2.0x10¹⁴ vg/kg, or about 2.0x10¹⁴ vg/kg to 1.0x10¹⁵, or about 2.0x10¹⁴ vg/kg to 6.0x10¹⁴, or about 2.0x10¹⁴ vg/kg to 5.0x10¹⁴, or about 2.0x10¹⁴ vg/kg to about 4.0x10¹⁴ vg/kg, or about 2.0x10¹⁴ vg/kg to about 3.75x10¹⁴ vg/kg, or about 2.0x10¹⁴ vg/kg to about 3.5x10¹⁴ vg/kg, or about 2.0x10¹⁴ vg/kg to about 3.25x10¹⁴vg/kg. The rAAV may be AAVrh74.MHCK7.microdystrophin. The AAVrh74.MHCK7.microdystrophin may be the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ IDNO: 6. The rAAV may be AAVrh74.MCK.microdystrophin. The AAVrh74.MCK.microdystrophin may be the AAVrh74.MCK.microdystrophin of nucleotides 56-4820 of SEQ ID NO: 5.

The compositions may be formulated for intravenous administration and comprise a dose of rAAV that is about 2.0x10¹⁴ vg/kg. The compositions may be formulated for intravenous administration and comprise a dose of rAAV that is about 5.0x10¹² vg/kg, or about 6.0x10¹² vg/kg, or about 7.0x10¹² vg/kg, or about 8.0x10¹² vg/kg, or about 9.0x10¹² vg/kg, or about 1.0x10¹³ vg/kg, or about 1.25x10¹³ vg/kg, or about 1.5x10¹³ vg/kg, or about 1.75x10¹³ vg/kg, or about 2.25x10¹³ vg/kg, or about 2.5x10¹³ vg/kg, or about 2.75x10¹³ vg/kg, or about 3.0x10¹³ vg/kg, or about 3.25x10¹³ vg/kg, or about 3.5x10¹³ vg/kg, or about 3.75x10¹³ vg/kg, or about 4.0x10¹³ vg/kg, or about 5.0x10¹³ vg/kg, or about 6.0x10¹³ vg/kg, or about 7.0x10¹³ vg/kg, or about 8.0x10¹³ vg/kg, or about 9.0x10¹³ vg/kg, or about 1.0x10¹⁴ vg/kg, or about 1.25x10¹⁴ vg/kg, or about 1.5x10¹⁴ vg/kg, or about 1.75x10¹⁴ vg/kg, or about 2.25x10¹⁴ vg/kg, or about 2.5x10¹⁴ vg/kg, or about 2.75x10¹⁴ vg/kg, or about 3.0x10¹⁴ vg/kg, or about 3.25x10¹⁴ vg/kg, or about 3.5x10¹⁴ vg/kg, or about 3.75x10¹⁴ vg/kg, or about 4.0x10¹⁴ vg/kg, or about 5.0x10¹⁴ vg/kg, or about 6.0x10¹⁴ vg/kg, or about 1x10¹⁵. The rAAV may be AAVrh74.MHCK7.microdystrophin. The AAVrh74.MHCK7.microdystrophin may be the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ IDNO: 6. The rAAV may be AAVrh74.MCK.microdystrophin. The AAVrh74.MCK.microdystrophin may be the AAVrh74.MCK.microdystrophin of nucleotides 56-4820 of SEQ ID NO: 5.

In any of the compositions, the dose of rAAV may be delivered in about 5mL/kg to about 15 mL/kg, or about 8 mL/kg to about 12 mL/kg, or 8 mL/kg to about 10 mL/kg, or 5 mL/kg to about 10 mL/kg or about 10 mL/kg to 12 mL/kg, or about 10 mL/kg to 15 mL/kg or 10 mL/kg to about 20 mL/kg. In a particular example, the composition comprises a dose of the rAAV delivered in about 10 mL/kg. The rAAV may be AAVrh74.MHCK7.microdystrophin. The AAVrh74.MHCK7.microdystrophin may be the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ IDNO: 6. The rAAV may be AAVrh74.MCK.microdystrophin. The AAVrh74.MCK.microdystrophin may be the AAVrh74.MCK.microdystrophin of nucleotides 56-4820 of SEQ ID NO: 5.

The compositions may be formulated for administration by injection, infusion or implantation. For example, the compositions are formulated for administration by infusion over approximately one hour. In addition, the compositions may be formulated for intravenous administration through a peripheral limb vein such as a peripheral arm vein or a peripheral leg vein. Alternatively, the infusion may be administered over approximately 30 minutes, or approximately 1.5 hours, or approximately 2 hours, or approximately 2.5 hours or approximately 3 hours.

Any of the compositions may comprise a rAAV comprising the human micro-dystrophin nucleotide sequence of SEQ ID NO: 1 and the MHCK7 promoter sequence of SEQ ID NO: 2 or SEQ ID NO:7 or a rAAV vector comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6.

In particular, the compositions may be for treating Duchenne muscular dystrophy or Becker's muscular dystrophy. For example, compositions for treating Duchenne muscular dystrophy or Becker's muscular dystrophy in a human subject in need thereof may comprise a dose of recombinant adeno-virus associated (rAAV) rAAV.MHCK7.microdystrophin, wherein the composition is formulated for administration by intravenous infusion over approximately one hour and the dose of the rAAV administered is about 2x10¹⁴ vg/kg, and wherein the rAAV comprises the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6.

A composition may comprise rAAYV for reducing fibrosis in a subject in need thereof. In addition, a composition may comprise a rAAV vectors for preventing fibrosis in a subject suffering from a muscular dystrophy.

Compositions may comprise rAAYV for increasing muscular force and/or muscle mass in a subject suffering from a muscular dystrophy. Compositions may comprise any of the rAAV for treatment of muscular dystrophy.

After administration of said composition to a human subject in need of treatment for muscular dystrophy, the serum CK level in the subject may be decreased as compared to the serum CK level before administration of the composition by a percentage level selected from the group consisting of:
a) at least 78% by 90, 180, or 270 days after the administration;
b) at least 46, 55, 70, or 85 % by 270 days after the administration;
c) at least 72, 73, 74, or 95 % by 180 days after the administration;
d) at least 87, 99, 93 or 95% by 90 days after the administration;
e) at least 70 % by 270 days after the administration;
f) 70 to 95% by 90, 180, or 270 days after the administration;
g) at least 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration; and
h) 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration.

A use of a dose of recombinant adeno-virus associated (rAAV) rAAV.MHCK7.microdystrophin may be for the preparation of a medicament for the treatment of muscular dystrophy in a human subject in need thereof, wherein the medicament is formulated for a systemic route of administration and the dose of the rAAV is about 1x10¹⁴ vg/kg to about 4x 101⁴ vg/kg. The rAAV may be AAVrh74.MHCK7.microdystrophin. The AAVrh74.MHCK7.microdystrophin may be the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ ID NO: 6. The rAAV may be AAVrh74.MCK.microdystrophin. The AAVrh74.MCK.microdystrophin may be the AAVrh74.MCK.microdystrophin of nucleotides 56-4820 of SEQ ID NO: 5.

For example, the medicament comprises a dose of rAAV of about 5.0x10¹² vg/kg to about 1.0x10¹⁴ vg/kg, or about 5.0x10¹² vg/kg to1.0x10¹⁴ vg/kg, or about 5.0x10¹² vg/kg to about 2.0x10¹⁴ vg/kg, or about 5.0x10¹² vg/kg to about 1.0x10¹⁴ vg/kg, or about 5.0x10¹² vg/kg to about 5.0x10¹³ vg/kg, or about 5.0x10¹² vg/kg to about 2.0x10¹³ vg/kg, or about 5.0x10¹² vg/kg to about 1.0x10¹³ vg/kg, or 1.0x10¹⁴ vg/kg to about 1.0x10¹⁵ vg/kg, or 1.0x10¹³ vg/kg to about 1.0x10¹⁴ vg/kg, or about 1.0x10¹³ vg/kg to1.0x10¹⁴ vg/kg, or about 1.0x10¹³ vg/kg to about 2.0x10¹⁴ vg/kg, or about 1.0x10¹³ vg/kg to about 1.0x10¹⁴ vg/kg, or about 1.0x10¹³ vg/kg to about 5.0x10¹³ vg/kg, or about 1.0x10¹³ vg/kg to about 3.0x10¹⁴ vg/kg, or about 1.0x10¹³ vg/kg to about 5.0x10¹⁴ vg/kg, or about 1.0x10¹³ vg/kg to about 6.0x10¹⁴ vg/kg, or 1.0x10¹³ vg/kg to about 1.0x10¹⁵ vg/kg, or 5.0x10¹³ vg/kg to about 1.0x10¹⁴ vg/kg, or about 5.0x10¹³ vg/kg to1.0x10¹⁴ vg/kg, or about 5.0x10¹³ vg/kg to about 2.0x10¹⁴ vg/kg, or about 5.0x10¹³ vg/kg to about 1.0x10¹⁴ vg/kg, or about 5.0x10¹³ vg/kg to about 3.0x10¹⁴ vg/kg, or about 5.0x10¹³ vg/kg to about 5.0x10¹⁴ vg/kg, or about 5.0x10¹³ vg/kg to about 6.0x10¹⁴ vg/kg, or 5.0x10¹³ vg/kg to about 1.0x10¹⁵ vg/kg, or 1.0x10¹⁴ vg/kg to about 6.0x10¹⁴ vg/kg, or 1.0x10¹⁴ vg/kg to about 5.0x10¹⁴ vg/kg, or 1.0x10¹⁴ vg/kg to about 4.0x10¹⁴ vg/kg, or 1.0x10¹⁴ vg/kg to about 1.0x10¹⁵ vg/kg, or 1.0x10¹⁴ vg/kg to about 3.0x10¹⁴ vg/kg, or about 1.0x10¹⁴ vg/kg to about 2.5x10¹⁴ vg/kg, or 1.0x10¹⁴ vg/kg to about 2.0x10¹⁴ vg/kg, or about 1.25x10¹⁴ vg/kg to about 3.75x10¹⁴ vg/kg, or about 1.25x10¹⁴ vg/kg to 6.0x10¹⁴, or about 1.25x10¹⁴ vg/kg to 5.0x10¹⁴, or about 1.25x10¹⁴ vg/kg to 4.0x10¹⁴, or about 1.25x10¹⁴ vg/kg to 1.0x10¹⁵, or about 1.25x10¹⁴ vg/kg to about 3.5x10¹⁴ vg/kg, or about 1.25x10¹⁴ vg/kg to about 3.0x10¹⁴ vg/kg, or about 1.25x10¹⁴ vg/kg to about 2.75x10¹⁴ vg/kg, or about 1.25x10¹⁴ vg/kg to about 2.5x10¹⁴ vg/kg, or about 1.25x10¹⁴ vg/kg to about 2.0x10¹⁴ vg/kg, or 1.25x10¹⁴ vg/kg to about 3.75x10¹⁴ vg/kg, or about 1.25x10¹⁴ vg/kg to about 3.5x10¹⁴ vg/kg, or 1.5x10¹⁴ vg/kg to about 1.0x10¹⁵ vg/kg, or about 1.5x10¹⁴ vg/kg to 6.0x10¹⁴, or about 1.5x10¹⁴ vg/kg to 5.0x10¹⁴, or about 1.5x10¹⁴ vg/kg to 4.0x10¹⁴, or about 1.5x10¹⁴ vg/kg to about 3.75x10¹⁴ vg/kg, or about 1.5x10¹⁴ vg/kg to about 3.5x10¹⁴ vg/kg, or about 1.5x10¹⁴ vg/kg to about 3.25x10¹⁴ vg/kg, or about 1.5x10¹⁴ vg/kg to about 3.0x10¹⁴ vg/kg, or about 1.5x10¹⁴ vg/kg to about 2.75x10¹⁴ vg/kg, or about 1.5x10¹⁴ vg/kg to about 2.5x10¹⁴ vg/kg, or about 1.5x10¹⁴ vg/kg to about 2.0x10¹⁴ vg/kg, or 1.75x10¹⁴ vg/kg to about 1.0x10¹⁵ vg/kg, or about 1.75x10¹⁴ vg/kg to 6.0x10¹⁴, or about 1.75x10¹⁴ vg/kg to 5.0x10¹⁴, or about 1.75x10¹⁴ vg/kg to 4.0x10¹⁴, or about 1.75x10¹⁴ vg/kg to about 3.75x10¹⁴ vg/kg, or about 1.75x10¹⁴ vg/kg to about 3.5x10¹⁴ vg/kg, or about 1.75x10¹⁴ vg/kg to about 3.25x10¹⁴ vg/kg, or about 1.75x10¹⁴ vg/kg to about 3.0x10¹⁴ vg/kg, or about 1.75x10¹⁴ vg/kg to about 2.75x10¹⁴ vg/kg, or about 1.75x10¹⁴ vg/kg to about 2.5x10¹⁴ vg/kg, or about 1.75x10¹⁴ vg/kg to about 2.25x10¹⁴ vg/kg, or about 1.75x10¹⁴ vg/kg to about 2.0x10¹⁴ vg/kg, or about 2.0x10¹⁴ vg/kg to 1.0x10¹⁵, or about 2.0x10¹⁴ vg/kg to 6.0x10¹⁴, or about 2.0x10¹⁴ vg/kg to 5.0x10¹⁴, or about 2.0x10¹⁴ vg/kg to about 4.0x10¹⁴ vg/kg, or about 2.0x10¹⁴ vg/kg to about 3.75x10¹⁴ vg/kg, or about 2.0x10¹⁴ vg/kg to about 3.5x10¹⁴ vg/kg, or about 2.0x10¹⁴ vg/kg to about 3.25x10¹⁴vg/kg. The rAAV may be AAVrh74.MHCK7.microdystrophin. The AAVrh74.MHCK7.microdystrophin may be the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ ID NO: 6. The rAAV may be AAVrh74.MCK.microdystrophin. The AAVrh74.MCK.microdystrophin may be the AAVrh74.MCK.microdystrophin of nucleotides 56-4820 of SEQ ID NO: 5.

The medicaments may be formulated for systemic administration of a dose of rAAV wherein the systemic route of administration is an intravenous route and the dose of the rAAV administered is about 2.0x10¹⁴ vg/kg. The medicament may be formulated for systemic administration of a dose of rAAV wherein the systemic route of administration is an intravenous route and the dose of the rAAV is about 5.0x10¹² vg/kg, or about 6.0x10¹² vg/kg, or about 7.0x10¹² vg/kg, or about 8.0x10¹² vg/kg, or about 9.0x10¹² vg/kg, or about 1.0x10¹³ vg/kg, or about 1.25x10¹³ vg/kg, or about 1.5x10¹³ vg/kg, or about 1.75x10¹³ vg/kg, or about 2.25x10¹³ vg/kg, or about 2.5x10¹³ vg/kg, or about 2.75x10¹³ vg/kg, or about 3.0x10¹³ vg/kg, or about 3.25x10¹³ vg/kg, or about 3.5x10¹³ vg/kg, or about 3.75x10¹³ vg/kg, or about 4.0x10¹³ vg/kg, or about 5.0x10¹³ vg/kg, or about 6.0x10¹³ vg/kg, or about 7.0x10¹³ vg/kg, or about 8.0x10¹³ vg/kg, or about 9.0x10¹³ vg/kg, or about 1.0x10¹⁴ vg/kg, or about 1.25x10¹⁴ vg/kg, or about 1.5x10¹⁴ vg/kg, or about 1.75x10¹⁴ vg/kg, or about 2.25x10¹⁴ vg/kg, or about 2.5x10¹⁴ vg/kg, or about 2.75x10¹⁴ vg/kg, or about 3.0x10¹⁴ vg/kg, or about 3.25x10¹⁴ vg/kg, or about 3.5x10¹⁴ vg/kg, or about 3.75x10¹⁴ vg/kg, or about 4.0x10¹⁴ vg/kg, or about 5.0x10¹⁴ vg/kg, or about 6.0x10¹⁴ vg/kg, or about 1x10¹⁵ vg/kg. The rAAV may be AAVrh74.MHCK7.microdystrophin. The AAVrh74.MHCK7.microdystrophin may be the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ ID NO: 6. The rAAV may be AAVrh74.MCK.microdystrophin. The AAVrh74.MCK.microdystrophin may be the AAVrh74.MCK.microdystrophin of nucleotides 56-4820 of SEQ ID NO: 5.

In any of the uses , the medicament may comprise a dose of rAAV in about 5mL/kg to about 15 mL/kg, or about 8 mL/kg to about 12 mL/kg, or 8 mL/kg to about 10 mL/kg, or 5 mL/kg to about 10 mL/kg or about 10 mL/kg to 12 mL/k, or about 10 mL/kg to 15 mL/kg or 10 mL/kg to about 20 mL/kg. The dose or the rAAV may be in about 10 mL/kg. The rAAV may be AAVrh74.MHCK7.microdystrophin. The AAVrh74.MHCK7.microdystrophin may be the AAVrh74.MHCK7.microdystrophin of SEQ ID NO: 9, nucleotides 55-5021 of SEQ ID NO: 3, nucleotides 1-4977 of SEQ ID NO: 8, or nucleotides 56-5022 of SEQ ID NO: 6. The rAAV may be AAVrh74.MCK.microdystrophin. The AAVrh74.MCK.microdystrophin may be the AAVrh74.MCK.microdystrophin of nucleotides 56-4820 of SEQ ID NO: 5.

In any of the uses , the medicament may be formulated for administration by injection, infusion or implantation. For example, the medicament is formulated for administration by infusion over approximately one hour. In addition, the medicament is formulated for intravenous administration through a peripheral limb vein, such as a peripheral arm vein or a peripheral leg vein. Alternatively, the infusion may be administered over approximately 30 minutes, or approximately 1.5 hours, or approximately 2 hours, or approximately 2.5 hours or approximately 3 hours.

In any of the uses , the medicament may comprise an rAAV comprising the human micro-dystrophin nucleotide sequence of SEQ ID NO: 1 and the MHCK7 promoter sequence of SEQ ID NO: 2 or SEQ ID NO:7 or the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ ID NO: 6.

A particular use is for preparation of a medicament for the treatment of Duchenne muscular dystrophy or Becker's muscular dystrophy. For example, use of a dose of recombinant adeno-virus associated (rAAV) rAAV.MHCK7.microdystrophin for the preparation of a medicament for treating Duchenne muscular dystrophy in a or Becker's muscular dystrophy human subject in need thereof, wherein the medicament is formulated for administration by intravenous infusion over approximately one hour and the dose of the rAAV administered is about 2x 10¹⁴ vg/kg, and wherein the rAAV comprises the AAVrh74.MHCK7.microdystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ ID NO: 6.

Use of a rAAV may be for preparation of a medicament for reducing fibrosis in a subject in need thereof. For example, the subject in need can be suffering from a muscular dystrophy, such as DMD or any other dystrophin associated muscular dystrophy.

Use of a rAAV may be for the preparation of a medicament to prevent fibrosis in a subject suffering from a muscular dystrophy.

Use of a rAAV may be for the preparation of a medicament to increase muscular strength and/or muscle mass in a subject suffering from muscular dystrophy.

Use of the rAAV may be for the preparation of a medicament for treatment of muscular dystrophy.

Use of a rAAV vector comprising the human micro-dystrophin nucleotide sequence of SEQ ID NO: 1 and the MHCK7 promoter nucleotide sequence of SEQ ID NO: 2 or SEQ ID NO:7 may be for preparation of a medicament for the treatment of a muscular dystrophy or a rAAV vector comprising the AAVrf74.MHCK7.microdystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ ID NO: 6 for treatment of muscular dystrophy.

In any of the uses , the serum CK level in the subject may be decreased after administration of the rAAV to the subject as compared to the serum CK level before administration of the rAAV by a percentage level selected from the group consisting of:
a) at least 78% by 90, 180, or 270 days after the administration;
b) at least 46, 55, 70, or 95 % by 270 days after the administration;
c) at least 72, 73, 74, or 95 % by 180 days after the administration;
d) at least 87, 88, 93 or 95% by 90 days after the administration;
e) at least 70 % by 270 days after the administration;
f) 70 to 95% by 90, 180, or 270 days after the administration;
g) at least 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration; and
h) 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration.

In any of the compositions for treating a muscular dystrophy or the uses of a medicament for treating a muscular dystrophy, the level of micro-dystrophin gene expression in a cell of the subject is increased after administration of the composition or medicament. Expression of the micro-dystrophin gene in the cell is detected by measuring the micro-dystrophin protein level by Western blot in muscle biopsied before and after administration of the composition or medicament. In particular, the level of micro-dystrophin protein is increased by at least about 70% to at least about 80%, or at least about 70% to at least about 90%, or at least about 80% to at least about 90% after administration of the composition or medicament compared to the level of micro-dystrophin before administration of the composition or medicament. For example, the level of micro-dystrophin protein is increased by at least about 70% or at least about 71% or at least about 72% or at least about 73% or at least about 74% or at least about 75% or at least about 76% or at least about 77% or at least about 78% or at least about 79% or at least about 80%, or at least about 81%, or at least about 82%, or at least about 83%, or at least about 84%, or at least about 85% after administration of the composition compared to the level of micro-dystrophin before administration of the composition or medicament.

In addition, expression of the micro-dystrophin gene in the cell is detected by measuring the micro-dystrophin protein level by immunohistochemistry in muscle biopsies before and after administration of the composition or medicament. The level of micro-dystrophin protein is increased by at least about 70% to at least about 80%, or at least about 70% to at least about 90%, or at least about 80% to at least about 90% after administration of rAAV compared to the level of micro-dystrophin before administration of the composition or medicament. For example, the level of microdystrophin protein is increased by at least about 70% or at least about 71% or at least about 72% or at least about 73% or at least about 74% or at least about 75% or at least about 76% or at least about 77% or at least about 78% or at least about 79% or at least about 80%, or at least about 81%, or at least about 82%, or at least about 83%, or at least about 84%, or at least about 85% after administration of the composition or medicament compared to the level of micro-dystrophin before administration of the composition or medicament.

In any of the compositions for treating a muscular dystrophy, the serum CK level in the subject is decreased after administration of the rAAV as compared to serum CK level before administration of the composition or medicament. For example, the serum CK level in the subject is decreased by about 65 % to about 90% or about 65% to about 95% or about 75% to about 90% or about 80% to about 90% or about 85% to about 95% or about 87% to about 95% or about 87% to about 90% by 60 days after administration of the composition or medicament as compared to the serum CK level before administration of the composition or medicament. In particular, in any of the compositions for treating a muscular dystrophy, the serum CK level in the subject is decreased by about 87% by 60 days after administration of the composition or medicament as compared to the serum CK level before administration of the composition or medicament or in any of the compositions for treating a muscular dystrophy or the uses of a medicament for treating a muscular dystrophy, the serum CK level in the subject is decreased by about 72% by 60 days after administration of the composition or medicament as compared to the serum CK level before administration of the composition or medicament, or in any of the compositions for treating a muscular dystrophy, the serum CK level in the subject is decreased by about 73% by 60 days after administration of the composition or medicament as compared to the serum CK level before administration of the composition or medicament, or in any of the compositions for treating a muscular dystrophy or the uses of a medicament for treating a muscular dystrophy, the serum CK level in the subject is decreased by about 78% by 60 days after administration of the composition or medicament as compared to the serum CK level before administration of the composition or in any of the compositions for treating a muscular dystrophy or the uses of a medicament for treating a muscular dystrophy, the serum CK level in the subject is decreased by about 95% by 60 days after administration of the composition or medicament as compared to the serum CK level before administration of the composition or medicament. In any of the composition s for treating a muscular dystrophy or the uses of a medicament for treating a muscular dystrophy, the number of micro-dystrophin positive fibers in the muscle tissue of the subject is increased after administration of the composition or medicament as compared to the number of micro-dystrophin positive fibers before administration of the composition or medicament. For example, the number of micro-dystrophin positive fibers is detected by measuring the micro-dystrophin protein level by Western blot or immunohistochemistry on muscle biopsies before and after administration of the composition or medicament.

In any of the compositions for treating a muscular dystrophy or the uses of a medicament for treating a muscular dystrophy, administration of the composition or medicament upregulates expression of DAPC proteins such as alpha-sarcoglycan or beta-sarcoglycan. For example, the level of alpha-sarcoglycan in the subject is increased after administration of the composition or medicament as compared to the level of alpha-sarcoglycan before administration of the composition or medicament. In addition, the level of beta-sarcoglycan in the subject is increased after administration of the composition or medicament as compared to the level of the beta-sarcoglycan before administration of the composition or medicament. The level of alpha-sarcoglycan or beta-sarcoglycan is detected by measuring the alpha-sarcoglycan or beta-sarcoglycan protein level by Western blot or immunohistochemistry on muscle biopsies before and after administration of the composition or medicament.

In any of the compositions for treating a muscular dystrophy or the uses of a medicament for treating a muscular dystrophy, disease progression in the subject is delayed after administration of the composition or medicament as measured by any of: the six minute walk test, time to rise, ascend 4 steps, ascend and descend 4 steps, North Star Ambulatory Assessment (NSAA), 10 meter timed test, 100 meter timed test, hand held dynamometry (HHD), Timed Up and Go, and/or Gross Motor Subtest Scaled (Bayley-III) score.

For example, after administration of any of the compositions for treating a muscular dystrophy or the uses of a medicament for treating a muscular dystrophy, the subject has at least a 6-point improvement in NSAA score at least 270 days after administration of the composition or medicament as compared to NSAA score before administration of the rAAV. Further, in any of the methods, the subject has at least about 0.8 second improvement in time to rise at least 270 days after administration of the composition or medicament as compared to time to rise before administration of the composition or medicament. In addition, in any of the methods or uses, the subject has at least about 1.2 second improvement in time to ascend 4 steps test at least 270 days after administration of the composition or medicament as compared to time to ascend 4 steps test before administration of the composition or medicament. In addition, in any of the methods or uses, the subject has at least about 7 second improvement in the 100 m timed test at least 270 days after administration of the composition or medicament as compared to the 100 m timed test before administration of the composition or medicament.

Compositions may be for expressing micro-dystrophin gene in a patient cell comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8 or nucleotides 56-5022 of SEQ ID NO: 6. Use of a dose of a AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8 or nucleotides 56-5022 of SEQ ID NO: 6 may be for the preparation of a medicament for expressing micro-dystrophin gene in a patient cell. For example, expression of the micro-dystrophin gene in the patient cell is detected by measuring the microdystrophin protein level by Western blot or immunohistochemistry in muscle biopsies before and after administration of the rAAV.MHCK7.micro-dystrophin construct. In addition, the expression of the micro-dystrophin gene is measured in the patient by detecting greater the number of vector genomes per nucleus, wherein 1 vector genome per nucleus is about 50% micro-dystrophin expression and great than 1 copy per nucleus is consistent with micro-dystrophin expression level. For example, the cells have 1.2 vector copies per nucleus, or 1.3 vector copies per nucleus, or 1.4 vector copies per nucleus, or 1.5 vector copies per nucleus, or 1.6 vector copies per nucleus, or 1.7 vector copies per nucleus, or 1.8 vector copies per nucleus, or 1.9 vector copies per nucleus.

Compositions may be for decreasing serum CK levels in a patient in need thereof, the composition comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. In addition, use of a dose of AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 may be for the preparation of a medicament for decreasing serum CK levels in a patient in need thereof. For example, the serum CK level in the patient is decreased by at least about 65% to about 90% or about 65% to about 95% or about 75% to about 90% or about 80% to about 90% or about 85% to about 95% or about 87% to about 95% or about 87% to about 90% by 60 days after administration of the composition or medicament as compared to the serum CK level before administration of the composition or medicament. In particular, the serum CK level in the subject is decreased by about 87% by 60 days after administration of the composition or medicament as compared to the serum CK level before administration of the composition or medicament, or decreased by about 72% by 60 days after administration of the composition or medicament as compared to the serum CK level before administration of the composition or medicament, or decreased by about 73% by 60 days after administration of the composition or medicament as compared to the serum CK level before administration of the composition or medicament, or decreased by about 78% by 60 days after administration of the composition or medicament as compared to the serum CK level before administration of the composition or medicament, or decreased by about 95% by 60 days after administration of the composition or medicament as compared to the serum CK level before administration of the composition or medicament.

Compositions may be for increasing micro-dystrophin positive fibers in a patient muscle tissue comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. In addition, use of a dose of AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 may be for the preparation of a medicament for increasing micro-dystrophin positive fibers in a patient muscle tissue. For example, the number of micro-dystrophin positive fibers is detected by measuring the dystrophin protein level by Western blot or immunohistochemistry on muscle biopsies before and after administration of the composition or medicament. In addition, the expression of the micro-dystrophin gene is measured in the patient by detecting greater the number of vector genomes per nucleus, wherein 1 vector genome per nucleus is about 50% micro-dystrophin expression and great than 1 copy per nucleus is consistent with micro-dystrophin expression level. For example, the cells have 1.2 vector copies per nucleus, or 1.3 vector copies per nucleus, or 1.4 vector copies per nucleus, or 1.5 vector copies per nucleus, or 1.6 vector copies per nucleus, or 1.7 vector copies per nucleus, or 1.8 vector copies per nucleus, or 1.9 vector copies per nucleus.

Compositions may be for increasing the expression of alpha-sarcoglycan in a patient in need thereof comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. Use of a dose of AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 may be for the preparation of a medicament for increasing the expression of alpha-sarcoglycan in a patient in need thereof. For example, the level of alpha-sarcoglycan is detected by measuring the alpha-sarcoglycan protein level by Western blot or immunohistochemistry on muscle biopsies before and after administration of the composition or medicament.

Compositions may be for increasing the expression of beta-sarcoglycan in a patient in need thereof comprising the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6. Use of the AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 may be for the preparation of a medicament for increasing the expression of beta-sarcoglycan in a patient in need thereof. For example, the level of beta-sarcoglycan is detected by measuring the beta-sarcoglycan protein level by Western blot or immunohistochemistry on muscle biopsies before and after administration of the composition or medicament.

Use of a dose of AAVrh74.MHCK7.micro-dystrophin construct nucleotide sequence of SEQ ID NO: 9, nucleotides 55-5021 of SEQ ID NO: 3, nucleotides 1-4977 of SEQ ID NO: 8, or nucleotides 56-5022 of SEQ IDNO: 6 may be for the preparation of a medicament for treating a patient with Duchenne muscular dystrophy or Becker muscular dystrophy, such that administration of the medicament results in disease progression in the patient is delayed as measured by any of: the six minute walk test, time to rise, ascend 4 steps, ascend and descend 4 steps, North Star Ambulatory Assessment (NSAA), 10 meter timed test, 100 meter timed test, hand held dynamometry (HHD), Timed Up and Go, and/or Gross Motor Subtest Scaled (Bayley-III) score.

For example, the subject has at least a 6-point improvement in NSAA score at least 270 days after administration of the composition or medicament as compared to NSAA score before administration of the composition or medicament. Further, the subject has at least about 0.8 second improvement in time to rise at least 270 days after administration of the composition or medicament as compared to time to rise before administration of the composition or medicament. In addition, the subject has at least about 1.2 second improvement in time to ascend 4 steps test at least 270 days after administration of the composition or medicament as compared to time to ascend 4 steps test before administration of the composition or medicament. In addition, the subject has at least about 7 second improvement in the 100 m timed test at least 270 days after administration of the composition or medicament as compared to the 100 m timed test before administration of the composition or medicament.

### BRIEF DESCRIPTION OF THE DRAWINGS

Figure 1 illustrates the rAAV.MHCK7.micro-dystrophin construct. In this construct, the cDNA expression cassette is flanked by AAV2 inverted terminal repeat sequences (ITR). The construct is characterized by an in-frame rod deletion (R4-R23), while hinges 1, 2 and 4 (H₁, H₂ and H₄) and the cysteine rich domain remain producing a 138 kDa protein. The expression of the micro-dystrophin protein (3579 bp) is guided by a MHCK7 promoter (795 bp). The intron and 5' UTR are derived from plasmid pCMVβ (Clontech). The micro-dystrophin cassette had a consensus Kozak immediately in front of the ATG start and a small 53 bp synthetic polyA signal for mRNA termination. The human micro-dystrophin cassette contained the (R4-R23/Δ71-78) domains as previously described by Harper et al. (Nature Medicine 8, 253-261 (2002)).
**Figure 2** provides the nucleic acid sequence (SEQ ID NO: 3) AAVrh74.MHCK7.micro-dystrophin.
**Figure 3** provides the pNLREP2-Caprh74 AAV helper plasmid map.
**Figure 4** provides the Ad Helper plasmid pHELP.
**Figure 5** illustrates the rAAV.MCK.micro-dystrophin plasmid construct.
**Figure 6** provides the nucleic acid sequence (SEQ ID NO: 5) rAAVrh74.MCK.micro-dystrophin.
**Figure 7** demonstrates micro-dystrophin gene expression in muscle fibers of gastrocnemius muscle biopsy as measured by immunocytochemistry.
**Figures 8A- 8C** provide Western blots demonstrating micro-dystrophin protein expression at the correct molecular weight. In Fig. 8C, Subject 4 samples (*) were diluted 1:4 (to linear range) as ULDQ (>80%) exceeded in initial analysis, and mean values were multiplied by the dilution correction factor for final value in comparison to normal. Mean Micro-dystrophin Expression Vs. Normal was 182.7% in Method 1 and 222.0% in Method 2.
**Figures 9A-9C** demonstrates administration of rAAVrh74.MHCK7. microdystrophin upregulates expression of the DAPC proteins, alpha-sarcoglycan and beta-sarcoglycan.
**Figure 10** demonstrates sustained dramatic reduction in Creatine Kinase (CK) with administration of rAAVrh74.MHCK7. micro-dystrophin.
**Figure 11** provides the mean CK change from baseline to day 270. This data demonstrated that CK significantly decreases over time after administration of rAAVrh74.MHCK7. micro-dystrophin.
**Figure 12** provides the mean NSAA change and the mean CK change from baseline to day 270. This data demonstrated that NSAA significantly increased over time after administration of rAAVrh74.MHCK7.micro-dystrophin.
**F** provides the nucleic acid sequence (SEQ ID NO: 9) AAVrh74.MHCK7.micro-dystrophin.
**Figure 14** illustrates the AAVrh74.MHCK7.micro-dystrophin plasmid construct.

AAVrh74.MHCK7.micro-dystrophin plasmid construct, which comprises kanamycin resistance gene.

### DETAILED DESCRIPTION

Muscle biopsies taken at the earliest age of diagnosis of DMD reveal prominent connective tissue proliferation. Muscle fibrosis is deleterious in multiple ways. It reduces normal transit of endomysial nutrients through connective tissue barriers, reduces the blood flow and deprives muscle of vascular-derived nutritional constituents, and functionally contributes to early loss of ambulation through limb contractures. Over time, treatment challenges multiply as a result of marked fibrosis in muscle. This can be observed in muscle biopsies comparing connective tissue proliferation at successive time points. The process continues to exacerbate leading to loss of ambulation and accelerating out of control, especially in wheelchair-dependent patients.

Without early treatment including a parallel approach to reduce fibrosis it is unlikely that the benefits of exon skipping, stop-codon read-through, or gene replacement therapies can ever be fully achieved. Even small molecules or protein replacement strategies are likely to fail without an approach to reduce muscle fibrosis. Previous work in aged *mdx* mice with existing fibrosis treated with AAV.microdystrophin demonstrated that we could not achieve full functional restoration (Liu, M., et al., Mol Ther 11, 245-256 (2005)). It is also known that progression of DMD cardiomyopathy is accompanied by scarring and fibrosis in the ventricular wall.

As used herein, the term "AAV" is a standard abbreviation for adeno-associated virus. Adeno-associated virus is a single-stranded DNA parvovirus that grows only in cells in which certain functions are provided by a co-infecting helper virus. There are currently thirteen serotypes of AAV that have been characterized. General information and reviews of AAV can be found in, for example, Carter, 1989, Handbook of Parvoviruses, Vol. 1, pp. 169-228, and Berns, 1990, Virology, pp. 1743-1764, Raven Press, (New York). However, it is fully expected that these same principles will be applicable to additional AAV serotypes since it is well known that the various serotypes are quite closely related, both structurally and functionally, even at the genetic level. (See, for example, Blacklowe, 1988, pp. 165-174 of Parvoviruses and Human Disease, J. R. Pattison, ed.; and Rose, Comprehensive Virology 3:1-61 (1974)). For example, all AAV serotypes apparently exhibit very similar replication properties mediated by homologous rep genes; and all bear three related capsid proteins such as those expressed in AAV2. The degree of relatedness is further suggested by heteroduplex analysis which reveals extensive cross-hybridization between serotypes along the length of the genome; and the presence of analogous self-annealing segments at the termini that correspond to "inverted terminal repeat sequences" (ITRs). The similar infectivity patterns also suggest that the replication functions in each serotype are under similar regulatory control.

An "AAV vector" as used herein refers to a vector comprising one or more polynucleotides of interest (or transgenes) that are flanked by AAV terminal repeat sequences (ITRs). Such AAV vectors can be replicated and packaged into infectious viral particles when present in a host cell that has been transfected with a vector encoding and expressing rep and cap gene products.

An "AAV virion" or "AAV viral particle" or "AAV vector particle" refers to a viral particle composed of at least one AAV capsid protein and an encapsidated polynucleotide AAV vector. If the particle comprises a heterologous polynucleotide (i.e. a polynucleotide other than a wild-type AAV genome such as a transgene to be delivered to a mammalian cell), it is typically referred to as an "AAV vector particle" or simply an "AAV vector". Thus, production of AAV vector particle necessarily includes production of AAV vector, as such a vector is contained within an AAV vector particle.

### AAV

Adeno-associated virus (AAV) is a replication-deficient parvovirus, the single-stranded DNA genome of which is about 4.7 kb in length including 145 nucleotide inverted terminal repeat (ITRs). There are multiple serotypes of AAV. The nucleotide sequences of the genomes of the AAV serotypes are known. For example, the nucleotide sequence of the AAV serotype 2 (AAV2) genome is presented in Srivastava et al., J Virol, 45: 555-564 (1983) as corrected by Ruffing et al., J Gen Virol, 75: 3385-3392 (1994). As other examples, the complete genome of AAV-1 is provided in GenBank Accession No. NC_002077; the complete genome of AAV-3 is provided in GenBank Accession No. NC_1829; the complete genome of AAV-4 is provided in GenBank Accession No. NC_001829; the AAV-5 genome is provided in GenBank Accession No. AF085716; the complete genome of AAV-6 is provided in GenBank Accession No. NC_00 1862; at least portions of AAV-7 and AAV-8 genomes are provided in GenBank Accession Nos. AX753246 and AX753249, respectively (see also U.S. Patent Nos. 7,282,199 and 7,790,449 relating to AAV-8); the AAV-9 genome is provided in Gao et al., J. Virol., 78: 6381-6388 (2004); the AAV-10 genome is provided in Mol. Ther., 13(1): 67-76 (2006); and the AAV-11 genome is provided in Virology, 330(2): 375-383 (2004). Cloning of the AAVrh.74 serotype is described in Rodino-Klapac., et al. Journal of translational medicine 5, 45 (2007). Cis-acting sequences directing viral DNA replication (rep), encapsidation/packaging and host cell chromosome integration are contained within the ITRs. Three AAV promoters (named p5, p19, and p40 for their relative map locations) drive the expression of the two AAV internal open reading frames encoding rep and cap genes. The two rep promoters (p5 and p19), coupled with the differential splicing of the single AAV intron (*e.g.,* at AAV2 nucleotides 2107 and 2227), result in the production of four rep proteins (rep 78, rep 68, rep 52, and rep 40) from the rep gene. Rep proteins possess multiple enzymatic properties that are ultimately responsible for replicating the viral genome. The cap gene is expressed from the p40 promoter and it encodes the three capsid proteins VP1, VP2, and VP3. Alternative splicing and non-consensus translational start sites are responsible for the production of the three related capsid proteins. A single consensus polyadenylation site is located at map position 95 of the AAV genome. The life cycle and genetics of AAV are reviewed in Muzyczka, Current Topics in Microbiology and Immunology, 158: 97-129 (1992).

AAV possesses unique features that make it attractive as a vector for delivering foreign DNA to cells, for example, in gene therapy. AAV infection of cells in culture is noncytopathic, and natural infection of humans and other animals is silent and asymptomatic. Moreover, AAV infects many mammalian cells allowing the possibility of targeting many different tissues *in vivo.* Moreover, AAV transduces slowly dividing and non-dividing cells, and can persist essentially for the lifetime of those cells as a transcriptionally active nuclear episome (extrachromosomal element). The AAV proviral genome is infectious as cloned DNA in plasmids which makes construction of recombinant genomes feasible. Furthermore, because the signals directing AAV replication, genome encapsidation and integration are contained within the ITRs of the AAV genome, some or all of the internal approximately 4.3 kb of the genome (encoding replication and structural capsid proteins, rep-cap) may be replaced with foreign DNA such as a gene cassette containing a promoter, a DNA of interest and a polyadenylation signal. The rep and cap proteins may be provided *in trans.* Another significant feature of AAV is that it is an extremely stable and hearty virus. It easily withstands the conditions used to inactivate adenovirus (56°C to 65°C for several hours), making cold preservation of AAV less critical. AAV may even be lyophilized. Finally, AAV-infected cells are not resistant to superinfection.

Multiple studies have demonstrated long-term (> 1.5 years) recombinant AAV-mediated protein expression in muscle. See, Clark et al., Hum Gene Ther, 8: 659-669 (1997); Kessler et al., Proc Nat. Acad Sc. USA, 93: 14082-14087 (1996); and Xiao et al., J Virol, 70: 8098-8108 (1996). See also, Chao et al., Mol Ther, 2:619-623 (2000) and Chao et al., Mol Ther, 4:217-222 (2001). Moreover, because muscle is highly vascularized, recombinant AAV transduction has resulted in the appearance of transgene products in the systemic circulation following intramuscular injection as described in Herzog et al., Proc Natl Acad Sci USA, 94: 5804-5809 (1997) and Murphy et al., Proc Natl Acad Sci USA, 94: 13921-13926 (1997). Moreover, Lewis et al., J Virol, 76: 8769-8775 (2002) demonstrated that skeletal myofibers possess the necessary cellular factors for correct antibody glycosylation, folding, and secretion, indicating that muscle is capable of stable expression of secreted protein therapeutics.

Recombinant AAV genomes may comprise nucleic acid molecule described herein and one or more AAV ITRs flanking a nucleic acid molecule. AAV DNA in the rAAV genomes may be from any AAV serotype for which a recombinant virus can be derived including, but not limited to, AAV serotypes AAVrh.74, AAV-1, AAV-2, AAV-3, AAV-4, AAV-5, AAV-6, AAV-7, AAV-8, AAV-9, AAV-10, AAV-11, AAV-12 and AAV-13. Production of pseudotyped rAAV is disclosed in, for example, WO 01/83692. Other types of rAAV variants, for example rAAV with capsid mutations, are also contemplated. See, for example, Marsic et al., Molecular Therapy, 22(11): 1900-1909 (2014). As noted in the Background section above, the nucleotide sequences of the genomes of various AAV serotypes are known in the art. To promote skeletal muscle specific expression, AAV1, AAV6, AAV8 or AAVrh.74 can be used.

DNA plasmids may comprise rAAV genomes . The DNA plasmids are transferred to cells permissible for infection with a helper virus of AAV (e.g., adenovirus, E1-deleted adenovirus or herpesvirus) for assembly of the rAAV genome into infectious viral particles. Techniques to produce rAAV particles, in which an AAV genome to be packaged, rep and cap genes, and helper virus functions are provided to a cell are standard in the art. Production of rAAV requires that the following components are present within a single cell (denoted herein as a packaging cell): a rAAV genome, AAV rep and cap genes separate from (*i.e.,* not in) the rAAV genome, and helper virus functions. The AAV rep and cap genes may be from any AAV serotype for which recombinant virus can be derived and may be from a different AAV serotype than the rAAV genome ITRs, including, but not limited to, AAV serotypes AAV-1, AAV-2, AAV-3, AAV-4, AAV-5, AAV-6, AAV-7, AAVrh.74, AAV-8, AAV-9, AAV-10, AAV-11, AAV-12 and AAV-13. Production of pseudotyped rAAV is disclosed in, for example, WO 01/83692.

A method of generating a packaging cell is to create a cell line that stably expresses all the necessary components for AAV particle production. For example, a plasmid (or multiple plasmids) comprising a rAAV genome lacking AAV rep and cap genes, AAV rep and cap genes separate from the rAAV genome, and a selectable marker, such as a neomycin resistance gene, are integrated into the genome of a cell. AAV genomes have been introduced into bacterial plasmids by procedures such as GC tailing (Samulski et al., 1982, Proc. Natl. Acad. S6. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Biol. Chem., 259:4661-4666). The packaging cell line is then infected with a helper virus such as adenovirus. The advantages of this method are that the cells are selectable and are suitable for large-scale production of rAAV. Other examples of suitable methods employ adenovirus or baculovirus rather than plasmids to introduce rAAV genomes and/or rep and cap genes into packaging cells.

General principles of rAAV production are reviewed in, for example, Carter, 1992, Current Opinions in Biotechnology, 1533-539; and Muzyczka, 1992, Curr. Topics in Microbial. and Immunol., 158:97-129). Various approaches are described in Ratschin et al., Mol. Cell. Biol. 4:2072 (1984); Hermonat et al., Proc. Natl. Acad. Sci. USA, 81:6466 (1984); Tratschin et al., Mol. Cell. Biol. 5:3251 (1985); McLaughlin et al., J. Virol., 62:1963 (1988); and Lebkowski et al., Mol. Cell. Biol., 7:349 (1988). Samulski et al., J. Virol., 63:3822-3828 (1989); U.S. Patent No. 5,173,414; WO 95/13365 and corresponding U.S. Patent No. 5,658.776 ; WO 95/13392; WO 96/17947; PCT/US98/18600; WO 97/09441 (PCT/US96/14423); WO 97/08298 (PCT/US96/13872); WO 97/21825 (PCT/US96/20777); WO 97/06243 (PCT/FR96/01064); WO 99/11764; Perrin et al. Vaccine 13:1244-1250 (1995); Paul et al. Human Gene Therapy 4:609-615 (1993); Clark et al. Gene Therapy 3:1124-1132 (1996); U.S. Patent. No. 5,786,211; U.S. Patent No. 5,871,982; and U.S. Patent. No. 6,258,595.

Packaging cells may be used to produce infectious rAAV. In one embodiment packaging cells may be stably transformed cancer cells such as HeLa cells, 293 cells and PerC.6 cells (a cognate 293 line). In another embodiment, packaging cells are cells that are not transformed cancer cells, such as low passage 293 cells (human fetal kidney cells transformed with E1 of adenovirus), MRC-5 cells (human fetal fibroblasts), WI-38 cells (human fetal fibroblasts), Vero cells (monkey kidney cells) and FRhL-2 cells (rhesus fetal lung cells).

Recombinant AAV (*i.e.,* infectious encapsidated rAAV particles) may comprise a rAAV genome. For example, the genomes of both rAAV lack AAV rep and cap DNA, that is, there is no AAV rep or cap DNA between the ITRs of the genomes. Examples of rAAV that may be constructed to comprise the nucleic acid molecules are set out in International Patent Application No. PCT/US2012/047999 (WO 2013/016352).

In an example, the recombinant AAV vector is produced by the triple transfection method (Xiao et al. , J Virol 72, 2224-2232 (1998) using the AAV vector plasmids rAAV.MHCK7.micro-dystrophin, pNLRep2-Caprh74 and pHelp, rAAV contains the micro-dystrophin gene expression cassette flanked by AAV2 inverted terminal repeat sequences (ITR). It is this sequence that is encapsidated into AAVrh74 virions. The plasmid contains the micro-dystrophin sequence and the MHCK7 enhancer and core promoter elements of the muscle specific promoter to drive gene expression. The expression cassette also contains an SV40 intron (SD/SA) to promote high-level gene expression and the bovine growth hormone polyadenylation signal is used for efficient transcription termination.

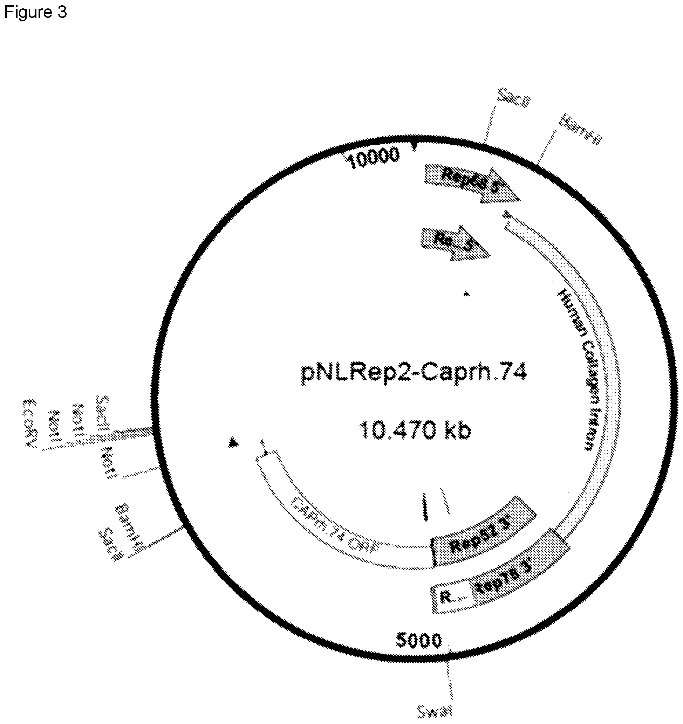

The pNLREP2-Caprh74 is an AAV helper plasmid that encodes the 4 wild-type AAV2 rep proteins and the 3 wild-type AAV VP capsid proteins from serotype rh74. A schematic map of the pNLREP2-Caprh74 plasmid is shown in Figure 3.

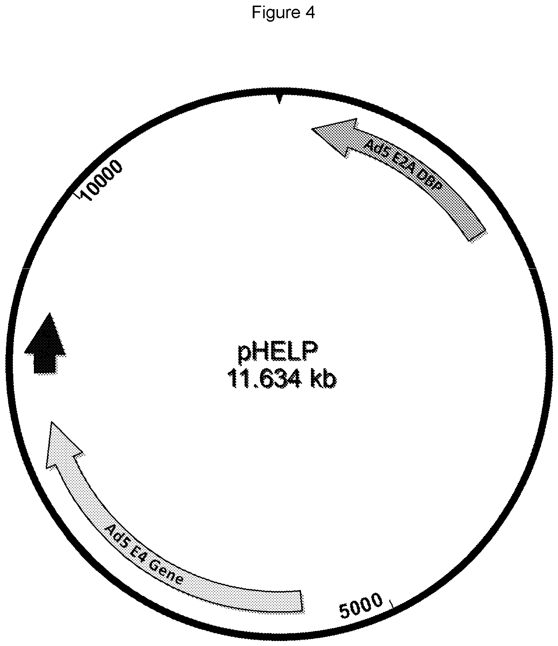

The pHELP adenovirus helper plasmid is 11,635 bp and was obtained from Applied Viromics. The plasmid contains the regions of adenovirus genome that are important for AAV replication, namely E2A, E4ORF6, and VA RNA (the adenovirus E1 functions are provided by the 293 cells). The adenovirus sequences present in this plasmid only represents ~40% of the adenovirus genome, and does not contain the *cis* elements critical for replication such as the adenovirus terminal repeats. Therefore, no infectious adenovirus is expected to be generated from such a production system. A schematic map of the pHELP plasmid is shown in Figure 4.

The rAAV may be purified by methods standard in the art such as by column chromatography or cesium chloride gradients. Methods for purifying rAAV vectors from helper virus are known in the art and include methods disclosed in, for example, Clark et al., Hum. Gene Ther., 10(6): 1031-1039 (1999); Schenpp and Clark, Methods Mol. Med., 69 427-443 (2002); U.S. Patent No. 6,566,118 and WO 98/09657.

Compositions may comprise rAAV. Compositions may comprise rAAV and a pharmaceutically acceptable carrier. The compositions may also comprise other ingredients such as diluents and adjuvants. Acceptable carriers, diluents and adjuvants are nontoxic to recipients and are preferably inert at the dosages and concentrations employed and include buffers and surfactants such as pluronics.

Titers of rAAV to be administered in methods will vary depending, for example, on the particular rAAV, the mode of administration, the treatment goal, the individual, and the cell type(s) being targeted, and may be determined by methods standard in the art. Titers of rAAV may range from about 1x10⁶, about 1x10⁷, about 1x10⁸, about 1x10⁹, about 1x10¹⁰, about 1x10¹¹, about 1x10¹², about 1x10¹³ to about 1x10¹⁴ or more DNase resistant particles (DRP) per ml. Dosages may also be expressed in units of viral genomes (vg). One exemplary method of determining encapsilated vector genome titer uses quantitative PCR such as the methods described in (Pozsgai et al., Mol. Ther. 25(4): 855-869, 2017).

Methods of transducing a target cell with rAAV, *in vivo* or *in vitro,* are contemplated. The *in vivo* methods comprise the step of administering an effective dose, or effective multiple doses, of a composition comprising a rAAV to an animal (including a human being) in need thereof. If the dose is administered prior to development of a disorder/disease, the administration is prophylactic. If the dose is administered after the development of a disorder/disease, the administration is therapeutic. An effective dose may be a dose that alleviates (eliminates or reduces) at least one symptom associated with the disorder/disease state being treated, that slows or prevents progression to a disorder/disease state, that slows or prevents progression of a disorder/disease state, that diminishes the extent of disease, that results in remission (partial or total) of disease, and/or that prolongs survival. An example of a disease contemplated for prevention or treatment is DMD.

Combination therapies are also contemplated. Combination as used herein includes both simultaneous treatment and sequential treatments. Combinations of methods with standard medical treatments (*e.g.,* corticosteroids) are specifically contemplated, as are combinations with novel therapies.

Administration of an effective dose of the compositions may be by routes standard in the art including, but not limited to, intramuscular, parenteral, intravenous, oral, buccal, nasal, pulmonary, intracranial, intraosseous, intraocular, rectal, or vaginal. Route(s) of administration and serotype(s) of AAV components of the rAAV (in particular, the AAV ITRs and capsid protein) may be chosen and/or matched by those skilled in the art taking into account the infection and/or disease state being treated and the target cells/tissue(s) that are to express the micro-dystrophin protein.

Local administration and systemic administration may be of an effective dose of rAAV and compositions. For example, systemic administration is administration into the circulatory system so that the entire body is affected. Systemic administration includes enteral administration such as absorption through the gastrointestinal tract and parenteral administration through injection, infusion or implantation.

In particular, actual administration of rAAV may be accomplished by using any physical method that will transport the rAAV recombinant vector into the target tissue of an animal. Administration according to the invention includes, but is not limited to, injection into muscle and injection into the bloodstream. Simply resuspending a rAAV in phosphate buffered saline has been demonstrated to be sufficient to provide a vehicle useful for muscle tissue expression, and there are no known restrictions on the carriers or other components that can be co-administered with the rAAV (although compositions that degrade DNA should be avoided in the normal manner with rAAV). Capsid proteins of a rAAV may be modified so that the rAAV is targeted to a particular target tissue of interest such as muscle. See, for example, WO 02/053703. Pharmaceutical compositions can be prepared as injectable formulations or as topical formulations to be delivered to the muscles by transdermal transport. Numerous formulations for both intramuscular injection and transdermal transport have been previously developed and can be used in the practice of the invention. The rAAV can be used with any pharmaceutically acceptable carrier for ease of administration and handling.

In one embodiment of the invention, the AAVrh74.MHCK7.microdystrophin described herein is formulated in a buffer containing 20 mM Tris (pH 8.0), 1mM magnesium chloride (MgCl₂), 200 mM sodium chloride (NaCl), and 0.001% poloxamer 188.

The dose of rAAV to be administered in methods disclosed herein will vary depending, for example, on the particular rAAV, the mode of administration, the treatment goal, the individual, and the cell type(s) being targeted, and may be determined by methods standard in the art. Titers of each rAAV administered may range from about 1x10⁶, about 1x10⁷, about 1x10⁸, about 1x10⁹, about 1x10¹⁰, about 1x10¹¹, about 1x10¹², about 1x10¹³, about 1x10¹⁴, about 2x10¹⁴, or to about 1x10¹⁵ or more DNase resistant particles (DRP) per ml. Dosages may also be expressed in units of viral genomes (vg) (*i.e.,* 1x10⁷ vg, 1x10⁸ vg, 1x10⁹ vg, 1x10¹⁰ vg, 1x10¹¹ vg, 1x10¹² vg, 1x10¹³ vg, 1x10¹⁴ vg, 2x10¹⁴vg, 1x10¹⁵vg respectively). Dosages may also be expressed in units of viral genomes (vg) per kilogram (kg) of bodyweight (*i.e.,* 1x10¹⁰ vg/kg, 1x10¹¹ vg/kg, 1x10¹² vg/kg, 1x10¹³ vg/kg, 1x10¹⁴ vg/kg, 1.25x10¹⁴ vg/kg, 1.5x10¹⁴ vg/kg, 1.75x10¹⁴ vg/kg, 2.0x10¹⁴ vg/kg, 2.25x10¹⁴ vg/kg, 2.5x10¹⁴ vg/kg, 2.75x10¹⁴ vg/kg, 3.0x10¹⁴ vg/kg, 3.25x10¹⁴ vg/kg, 3.5x10¹⁴ vg/kg, 3.75x10¹⁴ vg/kg, 4.0x10¹⁴ vg/kg, 1x10¹⁵ vg/kg respectively). Methods for titering AAV are described in Clark et al., Hum. Gene Ther., 10: 1031-1039 (1999).

In particular, actual administration of rAAV may be accomplished by using any physical method that will transport the rAAV recombinant vector into the target tissue of an animal. Administration according to the invention includes, but is not limited to, injection into muscle and injected into the bloodstream. Simply resuspending a rAAV in phosphate buffered saline has been demonstrated to be sufficient to provide a vehicle useful for muscle tissue expression, and there are no known restrictions on the carriers or other components that can be co-administered with the rAAV (although compositions that degrade DNA should be avoided in the normal manner with rAAV). Capsid proteins of a rAAV may be modified so that the rAAV is targeted to a particular target tissue of interest such as muscle. See, for example, WO 02/053703. Pharmaceutical compositions can be prepared as injectable formulations or as topical formulations to be delivered to the muscles by transdermal transport. Numerous formulations for both intramuscular injection and transdermal transport have been previously developed and can be used in the practice of the invention. The rAAV can be used with any pharmaceutically acceptable carrier for ease of administration and handling.

For purposes of intramuscular injection, solutions in an adjuvant such as sesame or peanut oil or in aqueous propylene glycol can be employed, as well as sterile aqueous solutions. Such aqueous solutions can be buffered, if desired, and the liquid diluent first rendered isotonic with saline or glucose. Solutions of rAAV as a free acid (DNA contains acidic phosphate groups) or a pharmacologically acceptable salt can be prepared in water suitably mixed with a surfactant such as hydroxpropylcellulose. A dispersion of rAAV can also be prepared in glycerol, liquid polyethylene glycols and mixtures thereof and in oils. Under ordinary conditions of storage and use, these preparations contain a preservative to prevent the growth of microorganisms. In this connection, the sterile aqueous media employed are all readily obtainable by standard techniques well-known to those skilled in the art.

The pharmaceutical carriers, diluents or excipients suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating actions of microorganisms such as bacteria and fungi. The carrier can be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating such as lecithin, by the maintenance of the required particle size in the case of a dispersion and by the use of surfactants. The prevention of the action of microorganisms can be brought about by various antibacterial and antifungal agents, for example, parabens, chlorobutanol, phenol, sorbic acid, thimerosal and the like. In many cases it will be preferable to include isotonic agents, for example, sugars or sodium chloride. Prolonged absorption of the injectable compositions can be brought about by use of agents delaying absorption, for example, aluminum monostearate and gelatin.

Sterile injectable solutions are prepared by incorporating rAAV in the required amount in the appropriate solvent with various other ingredients enumerated above, as required, followed by filter sterilization. Generally, dispersions are prepared by incorporating the sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze drying technique that yield a powder of the active ingredient plus any additional desired ingredient from the previously sterile-filtered solution thereof.

Transduction with rAAV may also be carried out *in vitro.* In one embodiment, desired target muscle cells are removed from the subject, transduced with rAAV and reintroduced into the subject. Alternatively, syngeneic or xenogeneic muscle cells can be used where those cells will not generate an inappropriate immune response in the subject.

Suitable methods for the transduction and reintroduction of transduced cells into a subject are known in the art. In one embodiment, cells can be transduced *in vitro* by combining rAAV with muscle cells, *e.g.,* in appropriate media, and screening for those cells harboring the DNA of interest using conventional techniques such as Southern blots and/or PCR, or by using selectable markers. Transduced cells can then be formulated into pharmaceutical compositions, and the composition introduced into the subject by various techniques, such as by intramuscular, intravenous, subcutaneous and intraperitoneal injection, or by injection into smooth and cardiac muscle, using *e.g.,* a catheter.

Transduction of cells with rAAV may result in sustained expression of the micro-dystrophin protein. Described herein are methods of administering/delivering rAAV which express micro-dystrophin protein to an animal, preferably a human being. These methods include transducing tissues (including, but not limited to, tissues such as muscle, organs such as liver and brain, and glands such as salivary glands) with one or more rAAV. Transduction may be carried out with gene cassettes comprising tissue specific control elements. For example, described are methods of transducing muscle cells and muscle tissues directed by muscle specific control elements, including, but not limited to, those derived from the actin and myosin gene families, such as from the myoD gene family (See Weintraub et al., Science, 251: 761-766 (1991)), the myocyte-specific enhancer binding factor MEF-2 (Cserjesi and Olson, Mol Cell Biol 11: 4854-4862 (1991)), control elements derived from the human skeletal actin gene (Muscat et al., Mol Cell Biol, 7: 4089-4099 (1987)), the cardiac actin gene, muscle creatine kinase sequence elements (See Johnson et al., Mol Cell Biol, 9:3393-3399 (1989)) and the murine creatine kinase enhancer (mCK) element, control elements derived from the skeletal fast-twitch troponin C gene, the slow-twitch cardiac troponin C gene and the slow-twitch troponin I gene: hypoxia-inducible nuclear factors (Semenza et al., Proc Natl Acad Sci USA, 88: 5680-5684 (1991)), steroid-inducible elements and promoters including the glucocorticoid response element (GRE) (See Mader and White, Proc. Natl. Acad. Sci. USA 90: 5603-5607 (1993)), and other control elements.

Muscle tissue is an attractive target for *in vivo* DNA delivery, because it is not a vital organ and is easy to access. The invention contemplates sustained expression of microdystrophin from transduced myofibers.

By "muscle cell" or "muscle tissue" is meant a cell or group of cells derived from muscle of any kind (for example, skeletal muscle and smooth muscle, *e.g.* from the digestive tract, urinary bladder, blood vessels or cardiac tissue). Such muscle cells may be differentiated or undifferentiated, such as myoblasts, myocytes, myotubes, cardiomyocytes and cardiomyoblasts.

The term "transduction" is used to refer to the administration/delivery of the coding region of the micro-dystrophin to a recipient cell either *in vivo* or *in vitro,* via a replication-deficient rAAV resulting in expression of micro-dystrophin by the recipient cell.

Thus, described herein are methods of administering an effective dose (or doses, administered essentially simultaneously or doses given at intervals) of rAAV that encode micro-dystrophin to a subject in need thereof.

The following EXAMPLES are provided by way of illustration and not limitation. Described numerical ranges are inclusive of each integer value within each range and inclusive of the lowest and highest stated integer.

### EXAMPLES

### Example 1

### A) Generation of the AAVrh74.MHCK7.micro-dystrophin construct

The AAVrh74.MHCK7.micro-dystrophin plasmid contains a human micro-dystrophin cDNA expression cassette flanked by AAV2 inverted terminal repeat sequences (ITR) (see Fig. 1). The micro-dystrophin construct was characterized by an in-frame rod deletion (R4-R23), while hinges 1, 2 and 4 and cysteine rich domain remain producing a 138 kDa protein. The expression of the micro-dystrophin protein (3579 bp) was guided by a MHCK7 promoter (792 bp). The plasmid was constructed from the rAAV.MCK.micro-dystrophin plasmid by removing the MCK promoter and inserting the MHCK7 promoter. After the core promoter, the 53 bp endogenous mouse MCK Exon1 (untranslated) is present for efficient transcription initiation, followed by the SV40 late 16S/19S splice signals (150 bp) and a small 5'UTR (61 bp). The intron and 5' UTR are derived from plasmid pCMVβ (Clontech). The micro-dystrophin cassette had a consensus Kozak immediately in front of the ATG start and a small 53 bp synthetic polyA signal for mRNA termination. The human micro-dystrophin cassette contained the (R4-R23/Δ71-78) domains as previously described by Harper et al. (Nature Medicine 8, 253-261 (2002)). The complementary DNA was codon optimized for human usage and synthesized by GenScript (Piscataway, NJ) (Mol Ther 18, 109-117 (2010)). The only viral sequences included in this vector were the inverted terminal repeats of AAV2, which are required for both viral DNA replication and packaging. The microdystrophin cassette has a small 53 bp synthetic polyA signal for mRNA termination.

Previous studies have validated cardiac expression using MHCK7 promoter (Salva et al. Mol Ther 15, 320-329 (2007) and AAVrh74 achieving skeletal, diaphragm, and cardiac muscle expression (Sondergaard et al. Annals of clinical and Transl Neurology 2, 256-270 (2015)), The sequence of construct of Fig. 1 was encapsidated into AAVrh.74 virions. The molecular clone of the AAVrh.74 serotype was cloned from a rhesus macaque lymph node and is discussed in in Rodino-Klapac et al. Journal of Translational medicine 5, 45 (2007).

**Table 1 shows the molecular features of the plasmid AAVrh74.MHCK7.micro-dystrophin (SEQ ID NO: 3)**

| TYPE | START | END | NAME | DESCRIPTION |
|---|---|---|---|---|
| REGION | 55 | 182 | 5' ITR | Wild-type AAV2 inverted terminal repeat |
| REGION | 244 | 1035 | MHCK7 | Mouse myosin heavy chain complex - E box muscle creatine kinase fusion enhancer/promoter |
| REGION | 1045 | 1194 | Chimeric intron | 5' donor site from human β-globin gene and the branchpoint and 3' splice acceptor site from IgG heavy chain variable region |
| GENE | 1205 | 4783 | huDys cDNA | Human micro-dystrophin cDNA |
| REGION | 4786 | 4838 | PolyA | Synthetic PolyA |
| REGION | 4894 | 5021 | 3' ITR | Wild-type AAV2 inverted terminal repeat |
| GENE | 6760 | 7619 | AmpR | β-lactamase gene |
| REGION | 7823 | 8442 | Ori | Plasmid origin of replication |

### B) Generation of the AAVrh74.MHCK7.micro-dystrophin construct from and plasmid encoding Kanamycin (Kan) resistance

Cloning of MHCK7.µDys.KAN was achieved by isolating the MHCK7.µDys fragment from an MHCK7.µDys.AMP plasmid and the Kanamycin Backbone, and annealing them using the NEBuilder cloning workflow. The MHCK7.µDys fragment was isolated via restriction enzyme digestion with SnaBI. The digestion was performed in a 50µL total reaction in 1x CutSmart Buffer (NEB) and 1µL SnaBI, at 37°C for 1 hour. The resulting fragment was isolated via electrophoresis using a 1% Agarose gel, running at 105 volts for 1.5 hours. The band corresponding to the MHCK7.µDys insert was cut out and purified using a gel purification kit (Macherey-Nagel). The resulting fragment had a DNA concentration of 10ng/µL. The Kan backbone fragment was isolated via XbaI restriction enzyme digestion in a 50µL reaction with 1x CutSmart Buffer (NEB) and 1µL XbaI, at 37°C for 1 hour. The resulting fragment was isolated via electrophoresis using a 1% Agarose gel, running at 105 volts for 1.5 hours. The band corresponding to the Kan Backbone was cut out and purified via gel purification kit (Macherey-Nagel). The resulting fragment had a DNA concentration of 8.1ng/µL. The two fragments were annealed using the NEB Builder cloning workflow, which has the ability to join two fragments with overlapping sequences. The NEBuilder cloning reaction was performed per manufacturer protocol at 50°C for 15 minutes, using a 1:1 ratio of MHCK7. µDys to Kanamycin backbone in 1x NEBuilder HiFi DNA Assembly Master Mix for a total reaction volume of 20µL. The resulting clone was transformed into NEB^{®} Stable Competent E. coli (C3040) by adding 2.5µL cloning product to the cells followed by 30 minutes on ice, then 30 seconds at 42°C and an additional 5 minutes on ice. After transformation, 950µL of outgrowth media was added to the cells and allowed to grow at 30°C for 1.5 hours, shaking at 225rpm. Following outgrowth, 450µL of these cells was plated on a 50µg/mL kanamycin LB agar plate and incubated overnight at 30°C in a dry incubator. A colony was picked from this plate and grown up overnight in LB containing 50µg/mL kanamycin. DNA was isolated from 3mL of this culture using QIAprep^{®} Spin Miniprep Kit (Qiagen). This DNA was used to confirm the cloning product. The cloning product was confirmed via restriction enzyme digestion with PmeI, MscI, and SmaI followed by gel electrophoresis. The cloning product was additionally confirmed via sequencing. The resultant plasmid is set forth in SEQ ID NO:8, and shown in Figures 14 and 15. The sequence of construct of Fig. 13 which corresponds to that of SEQ ID NO:9, and nucleotides 1-4977 of SEQ ID NO: 8, was encapsidated into AAVrh.74 virions as described above.

### Example 2

### Systemic Gene Delivery Clinical Trial for Duchenne Muscular Dystrophy

This is a single-dose controlled trial using the rAAVrh74.MHCK7.micro-dystrophin of SEQ ID NO: 3, nucleotides 55-5021, for DMD subjects. Cohort A will include six subjects of ages 3 months to 3 years, and Cohort B will include six subjects of ages 4 years to 7 years old. All subjects will receive intravenous micro-dystrophin vector (2×10¹⁴ vg/kg in 10mL/kg). The rAAVrh74.MHCK7.micro-dystrophin is formulated in a buffer containing 20 mM Tris (pH 8.0), 1mM magnesium chloride (MgCl₂), 200 mM sodium chloride (NaCl), and 0.001% poloxamer 188.

In the study, the rAAVrh74.MHCK7.micro-dystrophin was infused via peripheral arm vein so that it can reach all the muscles in the body. Six DMD subjects ages 3 months to 3 years in Cohort A, and six DMD subjects ages 4 years to age 7 years in Cohort B, were enrolled. All subjects received intravenous micro-dystrophin vector (2×10¹⁴ vg/kg in 10mL/kg). The encapsilated vector genome titer for the administered dose was determined using quantitative PCR using a Prism 7500 Taqman detector system (PE Applied Biosystems) with primers directed against the MHCK7 promoter compared to a supercoiled DNA plasmid standard (Pozsgai et al. Mol. Ther. 25(4): 855-869, 2017).

Subjects received infusions over 1 hour in the Pediatric Intensive Care Unit (PICU) at Nationwide Children's Hospital. Before the gene therapy, a muscle biopsy was performed at the screening visit. Subjects will have a second muscle biopsy to determine if the gene allowed for replacement of the missing dystrophin protein at 90 days post-delivery. After the gene transfer, patients were carefully monitored for any side effects of the treatment. This monitoring included blood and urine tests, as well as physical examination during the screening visits and on days 0, 1, 7, 14, 30, 60, 90, and 180, and at months 9, 12, 18, 24, 30 and 36 to make sure that there are no side effects from the gene injection.

The subjects of Cohort A (n=6) were between 3 months to 3 years of age, and received intravenous rAAVrh74.MHCK7.micro-dystrophin vector (2×10¹⁴ vg/kg in 10 mL/kg). One-day prior to gene transfer subjects in Cohort A were started on prednisone or deflazacort 1 mg/kg and maintained for 30 days while monitoring immune response. If negative at day 30, steroids were weaned over 1 week. If T cell response to AAV or micro-dys was >125 SFC/106 PBMCs, steroids were maintained until levels drop below this threshold.

The subjects of Cohort B (n=6) were between 4 years to 7 years of age, and received intravenous rAAVrh74.MHCK7.micro-dystrophin vector (2×10¹⁴ vg/kg in 10 mL/kg). These subjects were maintained on stable dose of corticosteroids throughout trial but may have been increased for short time if T cell response to AAV or micro-dystrophin was >125 SFC/106 PBMCs.

### Eligibility Criteria

Inclusion criteria for the study were as follows:
- Age of enrollment: Cohort A: 3 months to 7 years of age and Cohort B: between 4-7 years of age, inclusive.
- Molecular characterization of the DMD gene with frameshift (deletion or duplication), or premature stop codon mutation between exons 18 to 58.
- CK elevation >1000 U/L
- Cohort A subjects: below average on the Bayley-III motor assessment for gross motor defined as a scaled score of ≤9
- Cohort B: Below average on 100 meter timed test defined as < 80% predicted
- Males of any ethnic group.
- Ability to cooperate with motor assessment testing.
- Cohort A subjects: No previous treatment with corticosteroids.
- Cohort B subjects: Stable dose equivalent of oral corticosteroids for at least 12 weeks prior to screening and the dose is expected to remain constant (except for modifications to accommodate changes in weight) throughout the stud

Exclusion criteria for the study were as follows:
- Active viral infection based on clinical observations.
- Signs of cardiomyopathy, including echocardiogram with ejection fraction below 40%.
- Serological evidence of HIV infection, or Hepatitis B or C infection.
- Diagnosis of (or ongoing treatment for) an autoimmune disease.
- Abnormal laboratory values considered clinically significant
- Concomitant illness or requirement for chronic drug treatment that in the opinion of the PI creates unnecessary risks for gene transfer.
- Subjects with AAVrh74 or AAV8 antibody titers > 1:400 as determined by ELISA immunoassay.
- Medical condition or extenuating circumstance that, in the opinion of the investigator, might compromise the subject's ability to comply with the protocol required testing or procedures or compromise the subject's wellbeing, safety, or clinical interpretability.
- Severe infection (e.g., pneumonia, pyelonephritis, or meningitis) within 4 weeks before gene transfer visit (enrollment may be postponed).
- Received any investigational medication (other than corticosteroids) or exon skipping medications (including ExonDys 51^{®}), experimental or otherwise, in the last 6 months prior to screening for this study.
- Has had any type of gene therapy, cell based therapy (e.g. stem cell transplantation), or CRISPR/Cas9 therapy.
- Family does not want to disclose patient's study participation with primary care physician and other medical providers.

### Outcome Measures

The primary outcome measure was safety based on number of participants with adverse events (time frame: 3 years). Adverse effects were monitored and scored for severity and relatedness to the study article.

The secondary outcome measures were as follows:
Gross Motor Subtest Scaled (Bayley-III) score (time frame: screening, Day 30-3 Years): The Gross Motor Scaled Score measured motor development. The Bayley-III Gross Motor Subtest was scored for Cohort A on every follow up visit starting at Day 30 through 3 years. Any subject that was 43-47 months of age, inclusive, at time of screening had the scaled score calculated compared to normative data for 42 month old children. The Bayley-III provided normative data for children 1-42 months of age.

Physical Therapy Assessments The 100 Meter Timed Test (100m) (time frame: screening, Day 30-3 Years): The 100m was the primary motor outcome for Cohort B. The 100 Meter Timed Test was an exploratory outcome initiated for Cohort A as soon as the child was 3 years of age.

Physical Therapy Assessments North Star Ambulatory Assessment (NSAA) (time frame: screening, Day 30-3 Years): The North Star Ambulatory Assessment (NSAA) was an exploratory outcome initiated for Cohort A as soon as the child was four years of age and for cohort B. The NSAA measures the quality of ambulation in young boys with Duchenne Muscular Dystrophy.

Physical Therapy Assessments Timed Up and Go modified for children (TUG) (time frame: screening, Day 30-3 Years): Exploratory outcomes for Cohort B included the Timed Up and Go modified for children (TUG).

Physical Therapy Assessments Ascend and Descend of 4 steps (time frame: screening, Day 30-3 Years): Exploratory outcomes for Cohort B will include ascend and descend of 4 steps.

Physical Therapy Assessments Hand Held Dynamometry (HHD) (time frame: screening, Day 30-3 Years): Exploratory outcomes for Cohort B included hand held dynamometry (HHD) for knee extensors and knee flexors, and elbow flexors and elbow extensors.

Micro-dystrophin gene expression quantification by immunofluorescence (time frame: screening, Day 90): Micro-dystrophin gene expression levels were quantified by immunofluorescence and compared in pre and post muscle biopsies.

Micro-dystrophin gene expression quantification by western blot (time frame: screening, Day 90): Micro-dystrophin gene expression levels were quantified by western blot analysis and compared in pre and post muscle biopsies.

A decrease in CK following gene therapy (time frame: 3 years): Decrease in CK levels in circulating blood.

Cardiac magnetic resonance imaging (at 1 year).

### Micro-dystrophin Gene Expression

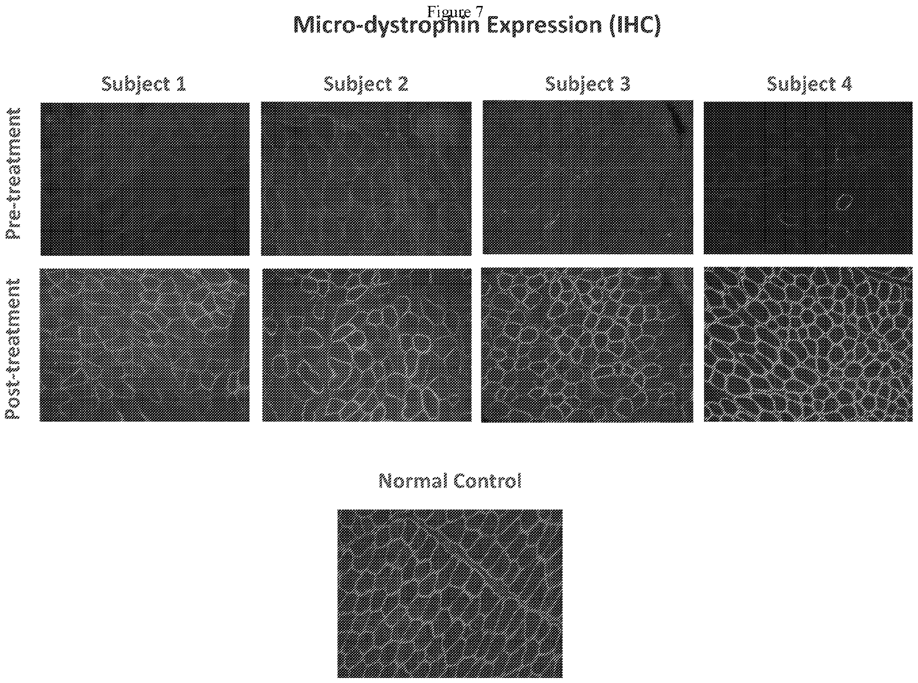

Change from Baseline in micro-dystrophin expression via immunofluorescence (IF) fiber intensity was analyzed and quantitated. As shown in Figure 7, Subject 1 (age 5) demonstrated 78% expression, of micro-dystrophin protein in the muscle fibers of the gastrocnemius muscle biopsy after administration of the rAAVrh74.MHCK7.micro-dystrophin, Subject 2 (age 4) demonstrated 73.5% expression of micro-dystrophin protein in the muscle fibers of the gastrocnemius muscle biopsy after administration of the rAAVrh74.MHCK7.micro-dystrophin, and Subject 3 (age 6) demonstrated 77.0% expression of micro-dystrophin protein in the muscle fibers of the gastrocnemius muscle biopsy after administration of the rAAVrh74.MHCK7.micro-dystrophin. Subject 4 (age 4) demonstrated 96.2% expression of micro-dystrophin in the muscle fibers of the gastrocnemius muscle biopsy after administration of the rAAVrh74.MHCK7.micro-dystrophin. All patients showed robust expression of transduced micro-dystrophin, which is properly localized to the muscle sarcolemma, as measured by immunohistochemistry. Mean gene expression, as measured by percentage of micro-dystrophin positive fibers was 76.2% and the mean intensity of the fibers was 74.5% compared to normal control.

| Subject | Mean Intensity | Percentage of Dystrophin-Positive Fibers |
|---|---|---|
| 1 | 82.0% | 78.0% |
| 2 | 59.0% | 73.5% |
| 3 | 83.0% | 77.0% |
| 4 | 160.0% | 96.2% |
| Mean | 96.0% | 81.2% |

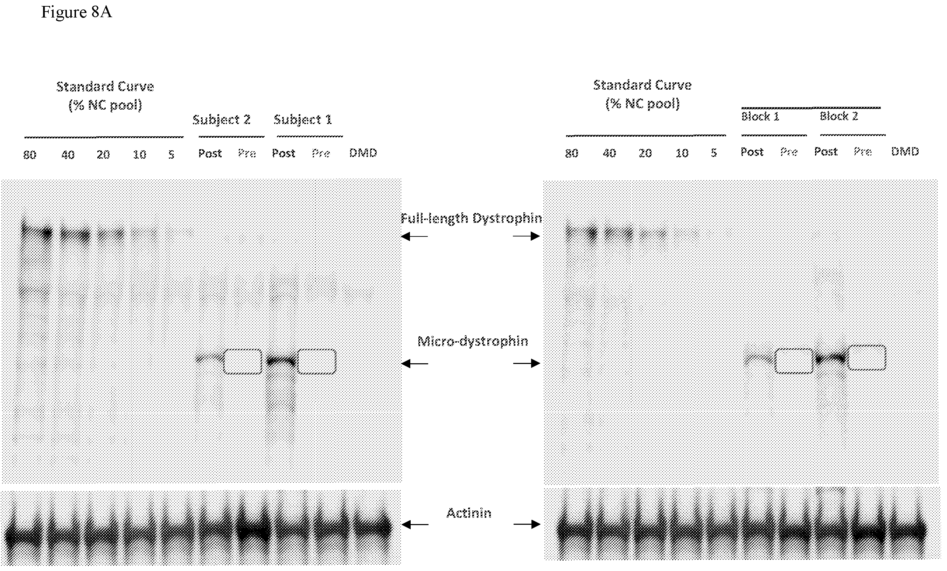

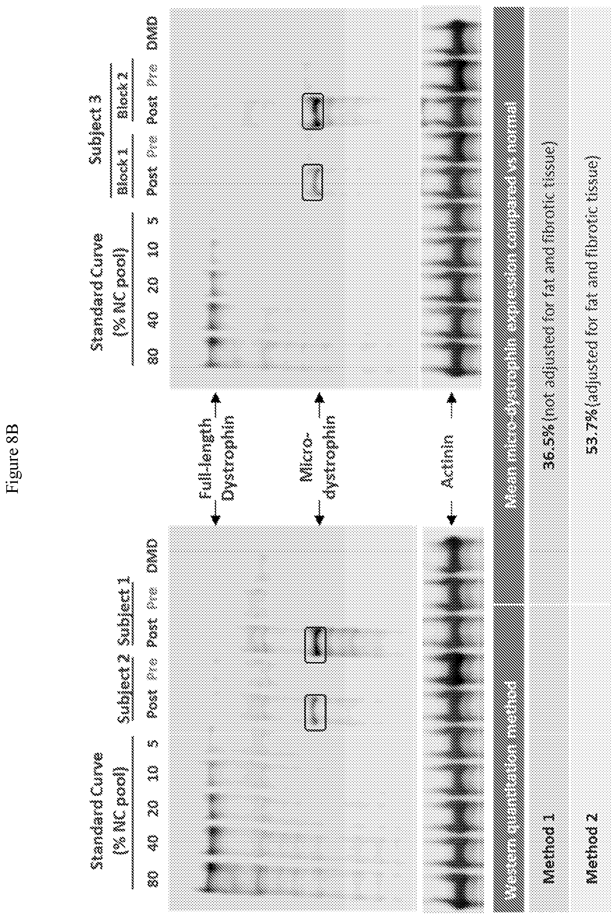

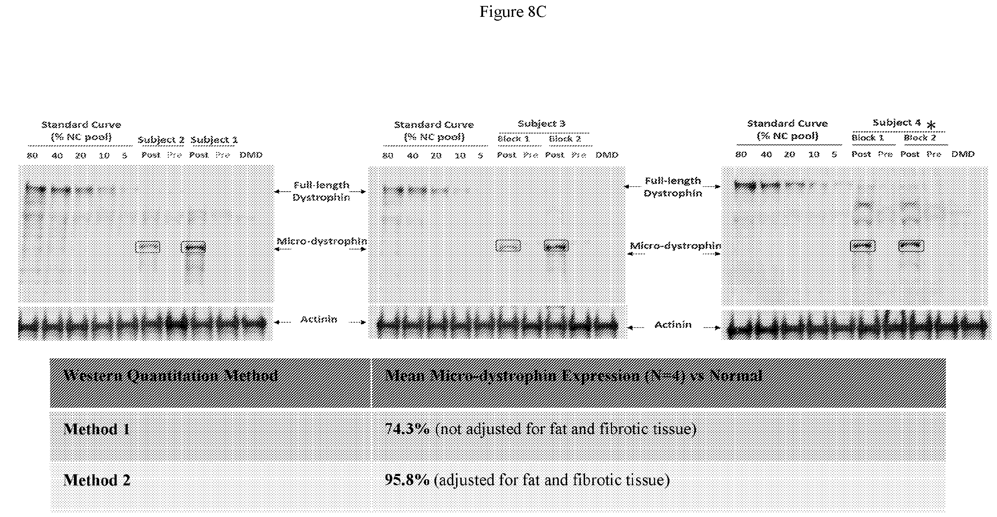

The change in micro-dystrophin gene expression from Baseline to Day 60 was also assessed by quantitating micro-dystrophin protein expression as measured by Western blot of biopsied muscle tissue. As shown in Figures 8A and 8B, Western blot analysis detected micro-dystrophin protein expression in Subject 1 (age 5), Subject 2 (age 4) and Subject 3 (age 6). Figure 8C provides the Western Blot analysis detecting micro-dystrophin protein expression in Subject 4 (age 4). All post-treatment biopsies showed robust levels of micro-dystrophin as measured by Western blot, with a mean for Subjects 1-4 of 74.3 compared to normal utilizing method 1, and 95.8% compared to normal pursuant to method 2 that adjusts for fat and fibrotic tissue.

For each subject, the vector genome copy per nucleus of the muscle fibers were measured. As shown in Table 2, the vector genome copy per nuclease was greater than 1 for each of the subjects after administration of rAAVrh74.MHCK7.micro-dystrophin. One copy of the vector indicates approximately 50% expression of the micro-dystrophin gene. A mean of 1.6 vector copies per cell nucleus was measured in Subjects 1-3, consistent with the high microdystrophin expression levels observed. When the values for Subject 4 were included, the mean vectors copies/µg DNA is >10⁵ with a mean of 3.3 vector copies per cell nucleus.

**Table 2**

| Subject | Vector copies/µg DNA | Copies per Nuclei |
|---|---|---|
| 1 | >10⁵ | 1.7 |
| 2 | >10⁵ | 1.3 |
| 3 | >10⁵ | 1.9 |

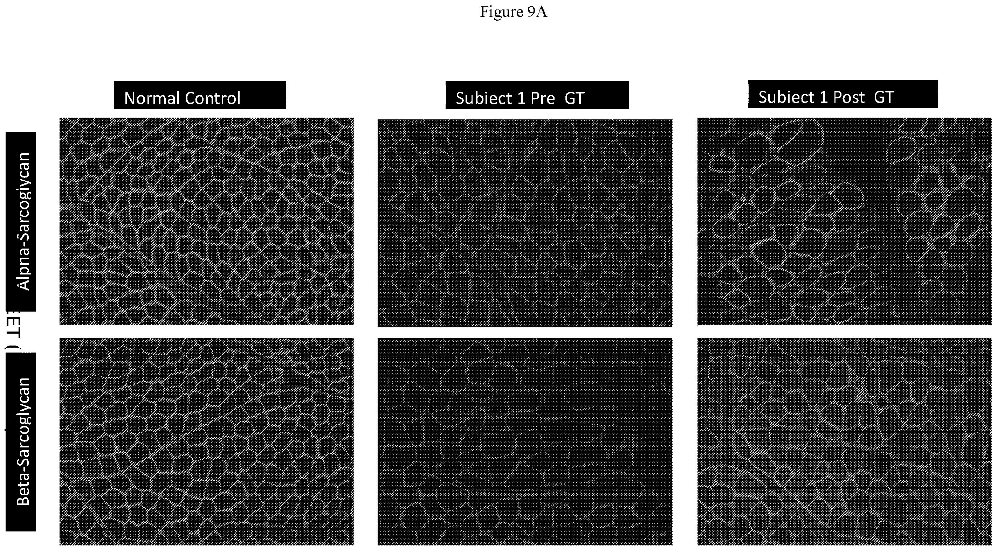

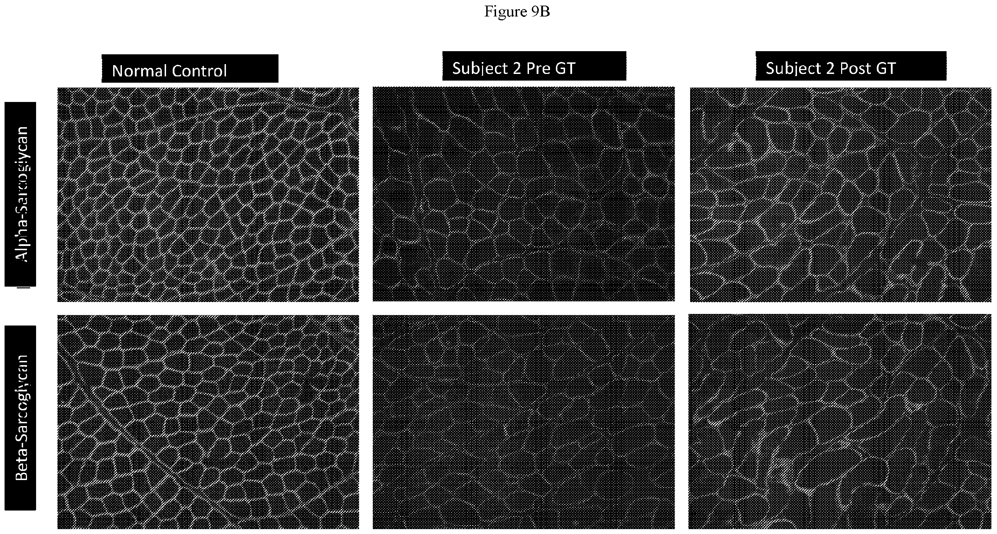

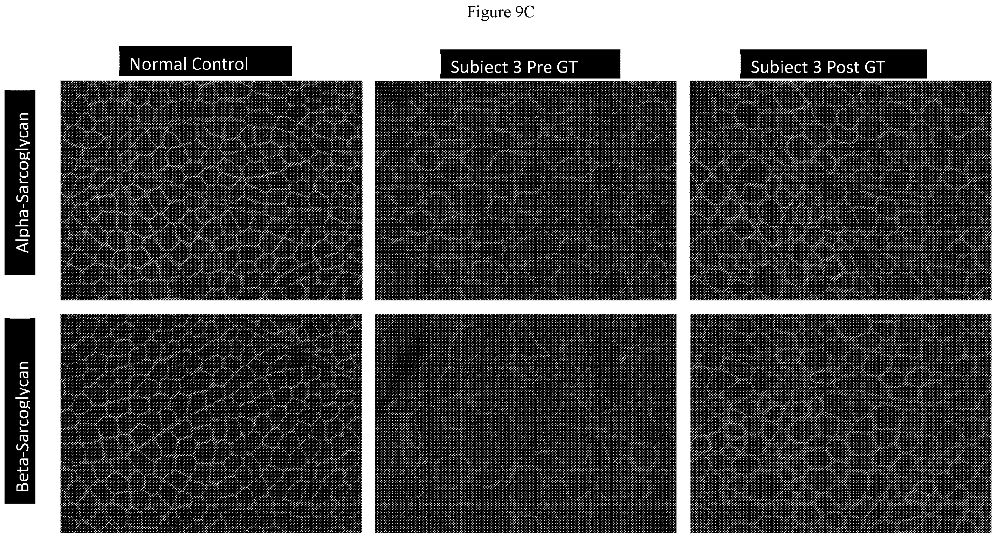

The protein levels of alpha-sarcoglycan and beta-sarcoglycan in muscle biopsy tissue were measured by immunohistochemistry before and after administration of rAAVrh74.MHCK7.micro-dystrophin. Administration of rAAVrh74.MHCK7 also resulted in upregulation of the DAPC proteins in the subjects. As shown in Figure 9, expression of alpha-sarcoglycan and beta-sarcoglycan in muscle biopsy tissue was increased compared to the level so these proteins in muscle biopsies before administration of rAAVrh74.MHCK7 in Subjects 1 (Fig. 9A), Subject 2 (Fig. 9B) and Subject 3 (Fig. 9C).

### Circulating Serum CK Levels

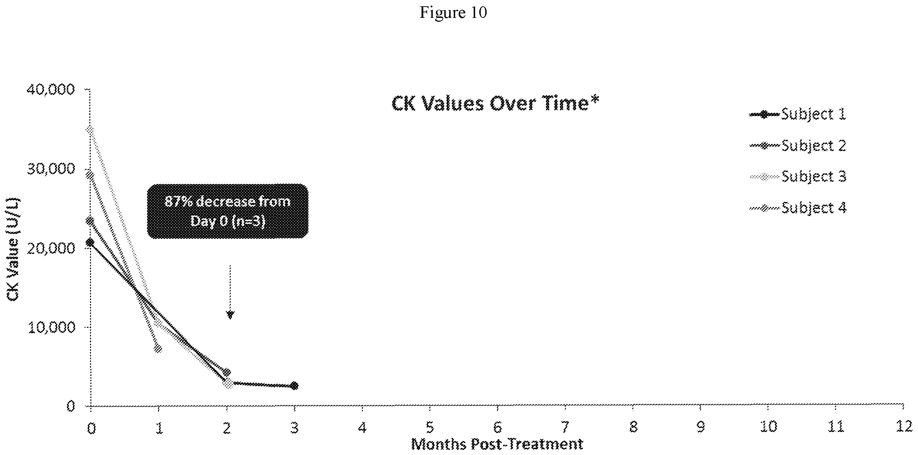

Blood samples were obtained every 30 days after administration intravenous infusion of rAAVrh74.MHCK7.micro-dystrophin vector (2x10¹⁴ vg/kg in 10 mL/kg). CK levels were measured at each visit and compared to the baseline level obtained before administration of the rAAVrh74.MHCK7.micro-dystrophin (Visit Day 0). Baseline serum CK levels (Units/Liter) are provided in Table 3 below. As shown in Figure 10, the level of circulating serum CK decreased about 87%, 2 months after administration of rAAVrh74.MHCK7.micro-dystrophin. All subjects showed significant decreases of serum creatine kinase (CK) levels, with a mean reduction of CK of over 87% at 2 months' post-treatment (n=3). CK is an enzyme associated with muscle damage and patients with DMD uniformly exhibit high levels of CK. Indeed, significantly elevated CK is often used as a preliminary diagnosis tool for DMD, which is then followed by confirmatory genetic testing.

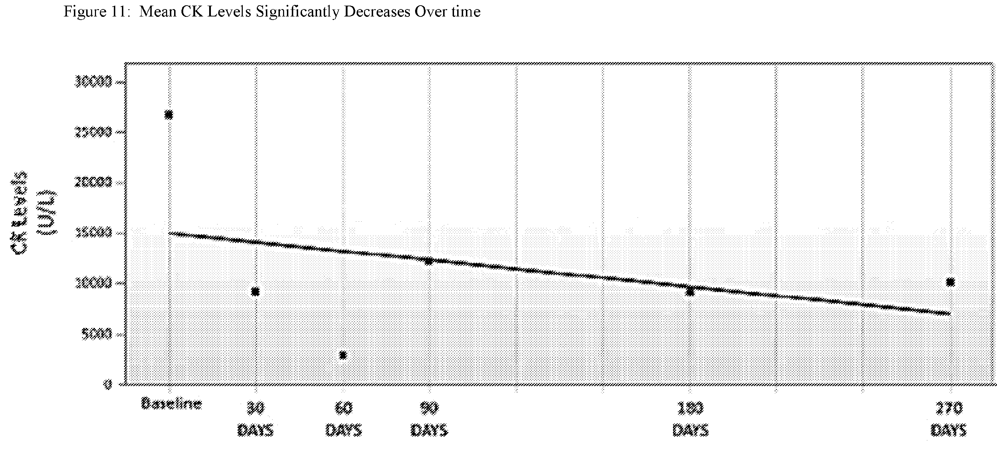

Table 4 and Figure 10 provide the CK levels for each subject. Figure 11 provides the mean CK levels over time, and demonstrates that the mean CK levels significantly decrease over time after administration of rAAVrh74.MHCK7.micro-dystrophin. The mean baseline CK level of 27,064 U/L (Mean for Table 3) is decreased by about 63% to a mean of 9,982 U/L (Mean, Day 270, Table 4).

**Table 3**

| Subject | Age (years) | CK Levels at Baseline Units/Liter (U/L) |
|---|---|---|
| 1 | 5 | 20691 |
| 2 | 4 | 23414 |
| 3 | 6 | 34942 |
| 4 | 4 | 29210 |

**Table 4: Change in CK levels from Baseline to Day 270**

| **Subject** | **Baseline** | **Day 30** | **Day 60** | **Day 90** | **Day 180** | **Day 270** | **Day 360** |
|---|---|---|---|---|---|---|---|
| 1 | | - | 2984 | 2444 | 18476 | 6317 | - |
| 2 | 23414 | 10427 | 4283 | 41920 | 6209 | 10494 | - |
| 3 | 34942 | 10430 | 2966 | 2546 | 9650 | 18855 | 6410 |
| 4 | 29210 | 7215 | 908 | 1382 | 2580 | 4262 | - |

### Efficacy Assessment

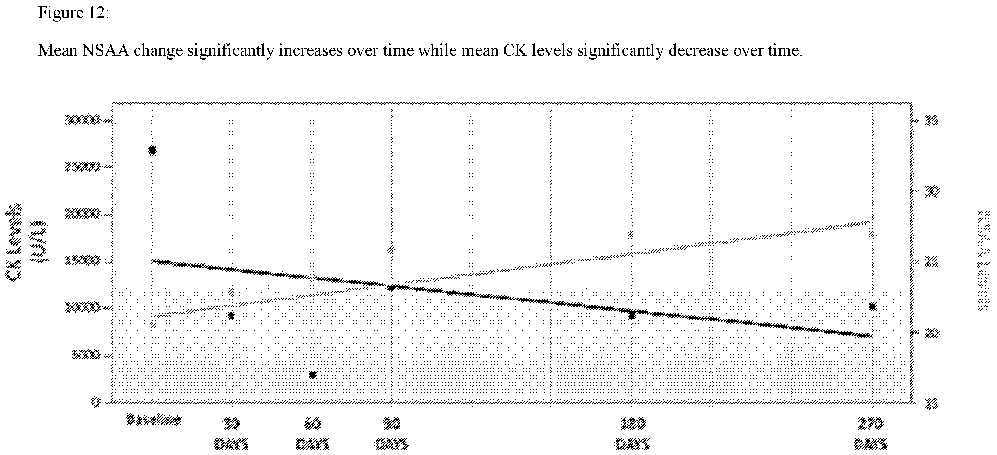

In addition to micro-dystrophin and CK levels, efficacy was measured by the following functional tests: Time to Rise from the floor, Ascend 4 steps, North Star Ambulatory Assessment (NSAA), Time to Rise Test, 4 Stairs Up Test, 10 Meter Timed Test (10m), and 100 Meter Timed Test (100m). The data is provided in Tables 5 and 6 below, and this data demonstrates consistent durable improvement at 9 months after administration of rAAVrh74.MHCK7.micro-dystrophin. The NSAA improvement over time is also provided in Figure 12.

**Table 5: NSAA Change from Baseline to Day 270**

| **Subject** | **Baseline** | **Day 30** | **Day 60** | **Day 90** | **Day 180** | **Day 270** | **Change from Baseline** |
|---|---|---|---|---|---|---|---|
| 1 | 18 | 22 | 24 | 23 | 25 | 26 | 8 |
| 2 | 19 | 21 | 23 | 25 | 27 | 27 | 8 |
| 3 | 26 | 28 | 28 | 30 | 30 | 28 | 2 |
| 4 | 19 | 20 | 20 | 25 | 25 | 27 | 8 |
| Mean Improvement | 20.5 | 22.75 | 23.75 | 25.75 | 26.75 | 27 | 6.5 |

**Table 6: Change from Baseline to Day 270**

| Subject | Assessment | NSAA | Time to Rise | 4 Stairs Up | 100m (sec) | 10 m |
|---|---|---|---|---|---|---|
| 1 | Baseline | 18 | 3.7 | 3.4 | 49.3 | 5.1 |
| | Day 270 | 26 | 3.0 | 2.3 | 43.2 | 4.3 |
| 2 | Baseline | 19 | 3.0 | 3.8 | 49.9 | 4.3 |
| | Day 180 | 27 | 3.7 | 2.6 | 48.6 | 3.9 |
| | Day 270 | 27 | 3.3 | 2.7 | 50.3 | |
| 3 | Baseline | 26 | 3.9 | 1.9 | 59.3 | 4.7 |
| | Day 180 | 30 | 3.4 | 1.8 | 48.4 | 4.1 |
| | Day 270 | 28 | 2.8 | 1.9 | 50.7 | |
| 4 | Baseline | 19 | 4.1 | 4.8 | 67.2 | 5.4 |
| | Day 90 | 25 | 2.3 | 2.2 | 50.7 | 4.4 |
| | Day 270 | 27 | 2.4 | 2.2 | 49.7 | |
| Average | Change from Baseline | 6.5 point improvement | .8 sec. improvement | 1.2 sec. improvement | 7.95 sec. improvement | 14% improvement |

### Safety Assessment

No serious adverse events (SAEs) were observed in the study. Three subjects had elevated gamma-glutamyl transferase (GGT) that resolved with increased steroids within a week and returned to baseline levels. There were no other clinically significant laboratory findings. Patients had transient nausea generally during the first week of therapy coincident with increased steroid dosing. This did not correlate with liver enzyme elevations or any other abnormality.

### Example 3

### Randomized Double-Blind Placebo Controlled Systemic gene delivery Phase I/IIa clinical trial

This is a randomized double-blind single-dose trial using rAAVrh74.MHCK7.micro-dystrophin for DMD subjects. The study includes twentyfour subjects ages 4 to 7 years old. Subjects are randomized to treatment or placebo at the time of enrollment. Twelve subjects receive intravenous rAAVrh74.MHCK7.micro-dystrophin vector (2x10¹⁴ vg/kg in approximately 10mL/kg) and twelve subjects will receive 10 mL/kg placebo (lactated ringers). Placebo subjects will roll over to treatment which will be given in the same manner as the 12 previously treated subjects one year after the last treated subject is dosed. Subjects receive infusions of rAAV carrying micro-dystrophin or lactated ringers over approximately 1 hour. Pre and post-treatment (90 Day) needle muscle biopsies are done on gastrocnemius muscles.

The primary objective of this study is the assessment of the safety of intravenous administration of rAAVrh74.MHCK7.micro-dystrophin for DMD subjects via peripheral limb vein. Safety endpoints are assessed by changes in hematology, serum chemistry, urinalysis, immunologic response to rAAVrh74 and micro-dystrophin, and reported history and observations of symptoms. Dystrophin gene expression serves as a primary outcome measure along with safety. Quantification is carried out using validated immunofluorescence and immunoblot assays. A decrease in CK following gene therapy serves as a secondary outcome. Efficacy is measured by the following functional tests: Time to Rise, Ascend 4 steps, North Star Ambulatory Assessment (NSAA), 10 Meter Timed Test (10m), 100 Meter Timed Test (100m). Exploratory measures include hand-held dynamometry (HHD) for knee extensors and knee flexors, and elbow flexors and elbow extensors.

Inclusion criteria for the study are as follows:
- Age of enrollment: between 4-7 years of age, inclusive.
- Molecular characterization of the DMD gene with frameshift (deletion or duplication), or premature stop codon mutation between exons 18 to 58.
- Indication of symptomatic muscular dystrophy: CK elevation >1000 U/L and Below mean percent predicted time on 100 meter walk test
- Males of any ethnic group will be eligible.
- Ability to cooperate with motor assessment testing.
- Stable dose equivalent of oral corticosteroids for at least 12 weeks prior to screening and the dose is expected to remain constant (except for potential modifications to accommodate changes in weight) throughout the study.

Exclusion criteria for the study are as follows:
- Active viral infection based on clinical observations.
- Signs of cardiomyopathy, including echocardiogram with ejection fraction below 40%.
- Serological evidence of HIV infection, or Hepatitis B or C infection.
- Diagnosis of (or ongoing treatment for) an autoimmune disease.
- Abnormal laboratory values considered clinically significant (GGT > 3XULN, bilirubin ≥ 3.0 mg/dL, creatinine ≥ 1.8 mg/dL, Hgb < 8 or > 18 g/Dl; WBC > 18,500 per cmm), platelets ≤ 50,000.
- Concomitant illness or requirement for chronic drug treatment that in the opinion of the PI creates unnecessary risks for gene transfer.
- Subjects with AAVrh74 or AAV8 antibody titers > 1:400 as determined by ELISA immunoassay. If endpoint titer is positive at screening, testing may be repeated prior to exclusion.
- Has a medical condition or extenuating circumstance that, in the opinion of the investigator, might compromise the subject's ability to comply with the protocol required testing or procedures or compromise the subject's wellbeing, safety, or clinical interpretability.
- Severe infection (e.g., pneumonia, pyelonephritis, or meningitis) within 4 weeks before gene transfer visit (enrollment may be postponed).
- Has received any investigational medication (other than corticosteroids) or exon skipping medications (including ExonDys 51^{®}), experimental or otherwise, in the last 6 months prior to screening for this study.
- Has had any type of gene therapy, cell based therapy (e.g. stem cell transplantation), or CRISPR/Cas9 therapy.
- Family does not want to disclose patient's study participation with primary care physician and other medical providers.

### Efficacy Assessments

Dystrophin gene expression serves as a primary outcome measure along with safety. Quantification is carried out using validated immunofluorescence and immunoblot assays. A decrease in CK following gene therapy serves as a secondary outcome. In addition, efficacy is measured by the following functional tests: Time to Rise from the floor, Ascend 4 steps, North Star Ambulatory Assessment (NSAA), 10 Meter Timed Test (10m), 100 Meter Timed Test (100m)]. Exploratory measures include hand-held dynamometry (HHD) for knee extensors and knee flexors, and elbow flexors and elbow extensors.

Muscle biopsies with ultrasound guidance are used to quantify transgene expression comparing baseline to Day 90. The biopsies are carried out on the same muscle as the original biopsies but on the opposite leg. One year after all subjects have been dosed, placebo crossover subjects will restart the study timeline at visit 1. Placebo subjects will not have the following performed at the second baseline screening: Cardiac MRI and Muscle Biopsy. Placebo subjects undergo a muscle biopsy at Day 90 (total of 3 muscle biopsies). Frozen sections are stained for dystrophin using indirect immunofluorescence (IF). Full slide scanning is performed and micro-dystrophin intensity and percent positive fibers is quantified using validated image scanning and MuscleMapTM analysis algorithm. Muscle morphometrics are performed blinded including fiber size histograms. Blinded frozen muscle biopsy shavings are used to perform quantitative protein analysis for microdystrophin using a validated western blot method.

Muscle needle biopsies of the gastrocnemius muscle (unless deemed contraindicated in a specific subject by the PI, in which case the PI will select an alternative muscle to biopsy) are used to quantify micro-dystrophin expression.

### Efficacy Analyses

The primary efficacy endpoint is the change from Baseline to Day 90 in the quantity of micro-dystrophin protein expression as measured by Western blot of biopsied muscle tissue. Treatment group differences for the primary efficacy endpoint are assessed with an analysis of covariance (ANCOVA) model with treatment as the fixed factor and baseline value as the covariate. The Wilcoxon rank-sum test is performed as a supportive analysis. Change from Baseline in micro-dystrophin expression via immunofluorescence (IF) fiber intensity is analyzed similarly.

The supportive efficacy endpoints include change from Baseline to each scheduled assessment of Time to Rise from the floor, Ascend 4 steps, NSAA, 10 Meter Timed Test (10m), 100 Meter Timed Test (100m), and change in CK. Exploratory measures include HHD for knee extensors and knee flexors, and elbow flexors and elbow extensors. Treatment group differences are assessed with ANCOVA model with treatment as the fixed factor and baseline value as the covariate. The Wilcoxon rank-sum test is performed as a supportive analysis.

### Example 4

The trials and studies described in Examples 2 and 3 above are alternatively carried out utilizing the rAAVrh74.MHCK7.micro-dystrophin construct set forth in SEQ ID NO: 9; as set forth in SEQ ID NO: 8, nucleotides 1-4977; or as set forth in SEQ ID NO: 6; nucleotides 56-5022.

### Example 5

### Generation of the pAAV.MCK.micro-dystrophin construct

The pAAV.MCK.micro-dystrophin plasmid was constructed by inserting the MCK expression cassette driving a codon optimized human micro-dystrophin cDNA sequence into the AAV cloning vector psub201 (Samulski et al., J. Virol. 61(10):3096-3101). A muscle-specific regulatory element was included in the construct to drive muscle-specific gene expression. This regulatory element comprised the mouse MCK core enhancer (206 bp) fused to the 351 bp MCK core promoter (proximal). After the core promoter, the construct comprises the 53 bp endogenous mouse MCK Exon1 (untranslated) for efficient transcription initiation, followed by the SV40 late 16S/19S splice signals (97 bp) and a small 5'UTR (61 bp). The intron and 5' UTR was derived from plasmid pCMVβ (Clontech). The micro-dystrophin cassette has a consensus Kozak immediately in front of the ATG start and a small 53 bp synthetic polyA signal for mRNA termination. The human micro-dystrophin cassette contains the (R4-R23/Δ71-78) domains as previously described by Harper et al. Nat. Med. 8(3):253-61, 2002

The pAAV.MCK.micro-dystrophin plasmid contained the human microdystrophin cDNA expression cassette flanked by AAV2 inverted terminal repeat sequences (ITR) (see Fig. 5). This sequence was encapsidated into AAVrh.74 virions. The molecular clone of the AAVrh.74 serotype was cloned from a rhesus macaque lymph node and is described in Rodino-Klapac et al. Journal of Tran. Med. 45 (2007).

### REFERENCES

1. Hoffman, E.P., Brown, R.H., Jr. & Kunkel, L.M. Dystrophin: the protein product of the Duchenne muscular dystrophy locus. Cell 51, 919-928 (1987).
2. Straub, V. & Campbell, K.P. Muscular dystrophies and the dystrophinglycoprotein complex. Curr Opin Neurol 10, 168-175 (1997).
3. Sacco, A., et al. Short telomeres and stem cell exhaustion model Duchenne muscular dystrophy in mdx/mTR mice. Cell 143, 1059-1071 (2010).
4. Wallace, G.Q. & McNally, E.M. Mechanisms of muscle degeneration, regeneration, and repair in the muscular dystrophies. Annu Rev Physiol 71, 37-57 (2009).
5. Zhou, L. & Lu, H. Targeting fibrosis in Duchenne muscular dystrophy. J Neuropathol Exp Neurol 69, 771-776 (2010).
6. Desguerre, I., et al. Endomysial fibrosis in Duchenne muscular dystrophy: a marker of poor outcome associated with macrophage alternative activation. J Neuropathol Exp Neurol 68, 762-773 (2009).
7. DiPrimio, N., McPhee, S.W. & Samulski, R.J. Adeno-associated virus for the treatment of muscle diseases: toward clinical trials. Curr Opin Mol Ther 12, 553-560 (2010).
8. Mendell, J.R., et al. Sustained alpha-sarcoglycan gene expression after gene transfer in limb-girdle muscular dystrophy, type 2D. Ann Neurol 68, 629-638 (2010).
9. Mendell, J.R., et al. Limb-girdle muscular dystrophy type 2D gene therapy restores alpha-sarcoglycan and associated proteins. Ann Neurol 66, 290-297 (2009).
10. Mendell, J.R., et al. A phase 1/2a follistatin gene therapy trial for becker muscular dystrophy. Molecular therapy : the journal of the American Society of Gene Therapy 23, 192-201 (2015).
11. Carnwath, J.W. & Shotton, D.M. Muscular dystrophy in the mdx mouse: histopathology of the soleus and extensor digitorum longus muscles. J Neurol Sci 80, 39-54 (1987).
12. Coulton, G.R., Morgan, J.E., Partridge, T.A. & Sloper, J.C. The mdx mouse skeletal muscle myopathy: I. A histological, morphometric and biochemical investigation. Neuropathol Appl Neurobiol 14, 53-70 (1988).
13. Cullen, M.J. & Jaros, E. Ultrastructure of the skeletal muscle in the X chromosome-linked dystrophic (mdx) mouse. Comparison with Duchenne muscular dystrophy. Acta Neuropathol 77, 69-81 (1988).
14. Dupont-Versteegden, E.E. & McCarter, R.J. Differential expression of muscular dystrophy in diaphragm versus hindlimb muscles of mdx mice. Muscle Nerve 15, 1105-1110 (1992).
15. Stedman, H.H., et al. The mdx mouse diaphragm reproduces the degenerative changes of Duchenne muscular dystrophy. Nature 352, 536-539 (1991).
16. Deconinck, A.E., et al. Utrophin-dystrophin-deficient mice as a model for Duchenne muscular dystrophy. Cell 90, 717-727 (1997).
17. Grady, R.M., et al. Skeletal and cardiac myopathies in mice lacking utrophin and dystrophin: a model for Duchenne muscular dystrophy. Cell 90, 729-738 (1997).
18. Love, D.R., et al. An autosomal transcript in skeletal muscle with homology to dystrophin. Nature 339, 55-58 (1989).
19. Tinsley, J.M., et al. Primary structure of dystrophin-related protein. Nature 360, 591-593 (1992).
20. Tinsley, J., et al. Expression of full-length utrophin prevents muscular dystrophy in mdx mice. Nat Med 4, 1441-1444 (1998).
21. Squire, S., et al. Prevention of pathology in mdx mice by expression of utrophin: analysis using an inducible transgenic expression system. Hum Mol Genet 11, 3333-3344 (2002).
22. Rafael, J.A., Tinsley, J.M., Potter, A.C., Deconinck, A.E. & Davies, K.E. Skeletal muscle-specific expression of a utrophin transgene rescues utrophin-dystrophin deficient mice. Nat Genet 19, 79-82 (1998).
23. Zhou, L., et al. Haploinsufficiency of utrophin gene worsens skeletal muscle inflammation and fibrosis in mdx mice. J Neurol Sci 264, 106-111 (2008).
24. Gutpell, K.M., Hrinivich, W.T. & Hoffman, L.M. Skeletal Muscle Fibrosis in the mdx/utrn+/- Mouse Validates Its Suitability as a Murine Model of Duchenne Muscular Dystrophy. PloS one 10, e0117306 (2015).
25. Rodino-Klapac, L.R., et al. Micro-dystrophin and follistatin codelivery restores muscle function in aged DMD model. Human molecular genetics 22, 4929-4937 (2013).
26. Nevo, Y., et al. The Ras antagonist, farnesylthiosalicylic acid (FTS), decreases fibrosis and improves muscle strength in dy/dy mouse model of muscular dystrophy. PloS one 6, e18049 (2011).
27. Rodino-Klapac, L.R., et al. A translational approach for limb vascular delivery of the micro-dystrophin gene without high volume or high pressure for treatment of Duchenne muscular dystrophy. J Transl Med 5, 45 (2007).
28. Mulieri, L.A., Hasenfuss, G., Ittleman, F., Blanchard, E.M. & Alpert, N.R. Protection of human left ventricular myocardium from cutting injury with 2,3-butanedione monoxime. Circ Res 65, 1441-1449 (1989).
29. Rodino-Klapac, L.R., et al. Persistent expression of FLAG-tagged micro dystrophin in nonhuman primates following intramuscular and vascular delivery. Molecular therapy : the journal of the American Society of Gene Therapy 18, 109-117 (2010).
30. Grose, W.E., et al. Homologous recombination mediates functional recovery of dysferlin deficiency following AAV5 gene transfer. PloS one 7, e39233 (2012).
31. Liu, M., et al. Adeno-associated virus-mediated microdystrophin expression protects young mdx muscle from contraction-induced injury. Mol Ther 11, 245-256 (2005).
32. Harper, S.Q., et al. Modular flexibility of dystrophin: implications for gene therapy of Duchenne muscular dystrophy. Nature medicine 8, 253-261 (2002).
33. Rodino-Klapac, L.R., et al. Persistent expression of FLAG-tagged micro dystrophin in nonhuman primates following intramuscular and vascular delivery. Mol Ther 18, 109-117 (2010).
34. Salva, M.Z., et al. Design of tissue-specific regulatory cassettes for high-level rAAV-mediated expression in skeletal and cardiac muscle. Mol Ther 15, 320-329 (2007).
35. Sondergaard, P.C., et al. AAV.Dysferlin Overlap Vectors Restore Function in Dysferlinopathy Animal Models. Annals of clinical and translational neurology 2, 256-270 (2015).
36. De, B.P., et al. High levels of persistent expression of alpha1-antitrypsin mediated by the nonhuman primate serotype rh.10 adeno-associated virus despite preexisting immunity to common human adeno-associated viruses. Mol Ther 13, 67-76 (2006).
37. Rodino-Klapac, L.R., et al. A translational approach for limb vascular delivery of the micro-dystrophin gene without high volume or high pressure for treatment of Duchenne muscular dystrophy. Journal of translational medicine 5, 45 (2007).
38. Bulfield et al., X chromosome-linked muscular dystrophy (mdx) in the mouse. Proc Natl Acad Sci U S A. 1984; 81(4): 1189-1192.
39. Sicinski et al., The molecular basis of muscular dystrophy in the mdx mouse: a point mutation. Science. 1989 30;244(4912):1578-80

## Claims

1. A recombinant adeno-associated virus (rAAV) for use in treating muscular dystrophy in a human subject in need thereof, wherein the rAAV is of the serotype rh.74 and comprises a polynucleotide comprising the nucleotide sequence of SEQ ID NO: 9, wherein the rAAV is administered using an intravenous route of administration and at a dose of 2x10¹⁴ vg/kg.

2. The rAAV for use according to claim 1, wherein the dose of the rAAV is determined by utilizing a super-coiled DNA plasmid standard.

3. The rAAV for use according to claim 1 or 2, wherein the composition is administered by infusion over approximately one hour.

4. The rAAV for use according to any preceding claim, wherein the muscular dystrophy is Duchenne muscular dystrophy or Becker's muscular dystrophy.

5. A recombinant adeno-associated virus (rAAV) for use in treating Duchenne muscular dystrophy in a human subject in need thereof, wherein the rAAV is of the serotype rh.74 and comprises a polynucleotide comprising the nucleotide sequence of SEQ ID NO: 9, wherein the rAAV is administered by intravenous infusion over approximately one hour at a dose of 2x 10¹⁴ vg/kg.

6. The rAAV for use according to claim 5, wherein the dose of the rAAV is determined by utilizing a super-coiled DNA Plasmid standard.

7. The rAAV for use according to any preceding claim, wherein the level of micro-dystrophin gene expression in a muscle cell of the subject is increased after administration of the rAAV as compared to the level of micro-dystrophin gene expression before administration of the rAAV.

8. The rAAV for use according to any preceding claim, wherein the level of micro-dystrophin protein is increased by at least 72% after administration of rAAV compared to level of micro-dystrophin before administration of rAAV.

9. The rAAV for use according to any preceding claim, wherein the serum CK level in the subject is decreased after administration of the rAAV as compared to serum CK level before administration of the rAAV.

10. The rAAV for use according to any preceding claim, wherein the number of micro-dystrophin positive fibers in the muscle tissue of the subject is increased after administration of the rAAV as compared to the number of micro-dystrophin positive fibers before administration of the rAAV.

11. The rAAV for use according to any preceding claim, wherein the level of alpha-sarcoglycan or beta-sarcoglycan in the subject is increased after administration of the rAAV as compared to the level of alpha-sarcoglycan or beta-sarcoglycan before administration of the rAAV.

12. The rAAV for use according to any preceding claim, wherein the serum CK level in the subject is decreased after administration of the rAAV as compared to the serum CK level before administration of the rAAV by a percentage level selected from the group consisting of:
a) at least 78% by 90, 180, or 270 days after the administration;
b) at least 46, 55, 56, 57, 58, 59, 60, 61, 62, 63, 64, 65, 66, 67, 68, 69, 70, or 85% by 270 days after the administration;
c) at least 72, 73, 74, or 95% by 180 days after the administration;
d) at least 87, 88, 93 or 95% by 90 days after the administration;
e) at least 70 % by 270 days after the administration;
f) 70 to 95% by 90, 180, or 270 days after the administration;
g) at least 55, 56, 57, 58, 59, 60, 61, 62, 63, 64, 65, 66, 67, 68, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration; and
h) at least 55, 56, 57, 58, 59, 60, 61, 62, 63, 64, 65, 66, 67, 68, 69, 70, 71, 72, 73, 74, 75,76, 77, 78, 79, 80, 81, 82, 83, 84, 85, 86, 87, 88, 89, 90, 91, 92, 93, 94, or 95 % by 90, 180, or 270 days after the administration.

13. The rAAV for use according to any preceding claim, wherein the rAAV is comprised in a composition.

## Patentansprüche

1. Rekombinantes Adeno-assoziiertes Virus (rAAV) zur Verwendung beim Behandeln von Muskeldystrophie in einem menschlichen Individuum, das diesbezüglich Bedarf aufweist, wobei das rAAV von dem Serotyp rh.74 stammt und ein Polynukleotid umfasst, das die Nukleotidsequenz unter SEQ ID NO: 9 umfasst, wobei das rAAV unter Verwendung eines intravenösen Verabreichungswegs und in einer Dosis von 2x10¹⁴ vg/kg verabreicht wird.

2. rAAV zur Verwendung nach Anspruch 1, wobei die rAAV-Dosis durch Nutzen eines Supercoiled-DNA-Plasmid-Standards bestimmt wird.

3. rAAV zur Verwendung nach Anspruch 1 oder 2, wobei die Zusammensetzung mittels Infusion etwa eine Stunde lang verabreicht wird.

4. rAAV zur Verwendung nach einem vorhergehenden Anspruch, wobei es sich bei der Muskeldystrophie um Duchenne-Muskeldystrophie oder Muskeldystrophie Becker handelt.

5. Rekombinantes Adeno-assoziiertes Virus (rAAV) zur Verwendung beim Behandeln von Duchenne-Muskeldystrophie in einem menschlichen Individuum, das diesbezüglich Bedarf aufweist, wobei das rAAV von dem Serotyp rh.74 stammt und ein Polynukleotid umfasst, das die Nukleotidsequenz unter SEQ ID NO: 9 umfasst, wobei das rAAV mittels intravenöser Infusion etwa eine Stunde lang in einer Dosis von 2x10¹⁴ vg/kg verabreicht wird.

6. rAAV zur Verwendung nach Anspruch 5, wobei die rAAV-Dosis durch Nutzen eines Supercoiled-DNA-Plasmid-Standards bestimmt wird.

7. rAAV zur Verwendung nach einem vorhergehenden Anspruch, wobei das Mikrodystrophin-Gen-Expressionsniveau in einer Muskelzelle des Individuums nach Verabreichung des rAAV im Vergleich zu dem Mikrodystrophin-Gen-Expressionsniveau vor Verabreichung des rAAV erhöht ist.

8. rAAV zur Verwendung nach einem vorhergehenden Anspruch, wobei der Mikrodystrophin-Proteinspiegel nach rAAV-Verabreichung im Vergleich zu dem Mikrodystrophin-Proteinspiegel vor rAAV-Verabreichung um mindestens 72 % erhöht ist.

9. rAAV zur Verwendung nach einem vorhergehenden Anspruch, wobei der CK-Serumspiegel bei dem Individuum nach Verabreichung des rAAV im Vergleich zum CK-Serumspiegel vor Verabreichung des rAAV verringert ist.

10. rAAV zur Verwendung nach einem vorhergehenden Anspruch, wobei die Anzahl von Mikrodystrophin-positiven Fasern im Muskelgewebe des Individuums nach Verabreichung des rAAV im Vergleich zu der Anzahl von Mikrodystrophin-positiven Fasern vor Verabreichung des rAAV erhöht ist.

11. rAAV zur Verwendung nach einem vorhergehenden Anspruch, wobei der Spiegel von Alpha-Sarcoglycan oder Beta-Sarcoglycan bei dem Individuum nach Verabreichung des rAAV im Vergleich zum Spiegel von Alpha-Sarcoglycan oder Beta-Sarcoglycan vor Verabreichung des rAAV erhöht ist.

12. rAAV zur Verwendung nach einem vorhergehenden Anspruch, wobei der CK-Serumspiegel bei dem Individuum nach Verabreichung des rAAV im Vergleich zum CK-Serumspiegel vor Verabreichung des rAAV um eine Prozentzahl verringert ist, die ausgewählt ist aus der Gruppe bestehend aus:
a) mindestens 78 % nach 90, 180 oder 270 Tagen nach der Verabreichung;
b) mindestens 46, 55, 56, 57, 58, 59, 60, 61, 62, 63, 64, 65, 66, 67, 68, 69, 70 oder 85 % nach 270 Tagen nach der Verabreichung;
c) mindestens 72, 73, 74 oder 95 % nach 180 Tagen nach der Verabreichung;
d) mindestens 87, 88, 93 oder 95 % nach 90 Tagen nach der Verabreichung;
e) mindestens 70 % nach 270 Tagen nach der Verabreichung;
f) 70 bis 95 % nach 90, 180 oder 270 Tagen nach der Verabreichung;
g) mindestens 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 oder 95 % nach 90, 180 oder 270 Tagen nach der Verabreichung; und
h) mindestens 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 oder 95 % nach 90, 180 oder 270 Tagen nach der Verabreichung.

13. rAAV zur Verwendung nach einem vorhergehenden Anspruch, wobei das rAAV in einer Zusammensetzung umfasst ist.

## Revendications

1. Virus adéno-associé recombinant (rAAV) destiné à être utilisé en traitement de dystrophie musculaire chez un sujet humain qui en a besoin, le rAAV étant du sérotype rh.74 et comprenant un polynucléotide comprenant la séquence nucléotidique de SEQ ID N° : 9, le rAAV étant administré à l'aide d'une voie d'administration intraveineuse et à une dose de 2 x 10¹⁴ vg/kg.

2. rAAV destiné à être utilisé selon la revendication 1, la dose du rAAV étant déterminée par l'utilisation d'un standard de plasmide d'ADN superenroulé.

3. rAAV destiné à être utilisé selon la revendication 1 ou 2, la composition étant administrée par perfusion sur approximativement une heure.

4. rAAV destiné à être utilisé selon une quelconque revendication précédente, la dystrophie musculaire étant la dystrophie musculaire de Duchenne ou la dystrophie musculaire de Becker.

5. Virus adéno-associé recombinant (rAAV) destiné à être utilisé en traitement de dystrophie musculaire de Duchenne chez un sujet humain qui en a besoin, le rAAV étant du sérotype rh.74 et comprenant un polynucléotide comprenant la séquence nucléotidique de SEQ ID N° : 9, le rAAV étant administré par perfusion intraveineuse sur approximativement une heure à une dose de 2 x 10¹⁴ vg/kg.

6. rAAV destiné à être utilisé selon la revendication 5, la dose du rAAV étant déterminée par l'utilisation d'un standard de plasmide d'ADN superenroulé.

7. rAAV destiné à être utilisé selon une quelconque revendication précédente, le niveau d'expression du gène de la micro-dystrophine dans une cellule musculaire du sujet étant augmenté après l'administration du rAAV par comparaison avec le niveau d'expression du gène de la micro-dystrophine avant l'administration du rAAV.

8. rAAV destiné à être utilisé selon une quelconque revendication précédente, le niveau de protéine micro-dystrophine étant augmenté d'au moins 72 % après l'administration de rAAV par comparaison avec le niveau de micro-dystrophine avant l'administration de rAAV.

9. rAAV destiné à être utilisé selon une quelconque revendication précédente, le niveau de CK sérique chez le sujet étant diminué après l'administration du rAAV par comparaison avec le niveau de CK sérique avant l'administration du rAAV.

10. rAAV destiné à être utilisé selon une quelconque revendication précédente, le nombre de fibres positives pour la micro-dystrophine dans le tissu musculaire du sujet étant augmenté après l'administration du rAAV par comparaison avec le nombre de fibres positives pour la micro-dystrophine avant l'administration du rAAV.

11. rAAV destiné à être utilisé selon une quelconque revendication précédente, le niveau d'alpha-sarcoglycane ou de bêta-sarcoglycane chez le sujet étant augmenté après l'administration du rAAV par comparaison avec le niveau d'alpha-sarcoglycane ou de bêta-sarcoglycane avant l'administration du rAAV.

12. rAAV destiné à être utilisé selon une quelconque revendication précédente, le niveau de CK sérique chez le sujet étant diminué après l'administration du rAAV par comparaison avec le niveau de CK sérique avant l'administration du rAAV d'un niveau de pourcentage choisi dans le groupe constitué par :
a) au moins 78 % 90, 180 ou 270 jours après l'administration ;
b) au moins 46, 55, 56, 57, 58, 59, 60, 61, 62, 63, 64, 65, 66, 67, 68, 69, 70 ou 85 % 270 jours après l'administration ;
c) au moins 72, 73, 74 ou 95 % 180 jours après l'administration ;
d) au moins 87, 88, 93 ou 95 % 90 jours après l'administration ;
e) au moins 70 % 270 jours après l'administration ;
f) 70 à 95 % 90, 180 ou 270 jours après l'administration ;
g) au moins 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 ou 95 % 90, 180 ou 270 jours après l'administration ; et
h) au moins 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 ou 95 % 90, 180 ou 270 jours après l'administration.

13. rAAV destiné à être utilisé selon une quelconque revendication précédente, le rAAV étant compris dans une composition.


### SEQUENCE LISTING

<110> RESEARCH INSTITUTE AT NATIONWIDE CHILDREN'S HOSPITAL
<120> ADENO-ASSOCIATED VIRUS VECTOR DELIVERY OF MUSCLE SPECIFIC MICRO-DYSTROPHIN TO TREAT MUSCULAR DYSTROPHY
<130> 28335/53169
<160> 9
<170> PatentIn version 3.5
<210> 1
   <211> 3579
   <212> DNA
   <213> Homo sapiens
<400> 1
   atgctgtggt gggaggaggt ggaggattgt tatgaaaggg aggacgtgca gaagaagact      60
   tttaccaagt gggtgaacgc tcagttcagc aaatttggga agcagcacat cgagaatctg     120
   ttttccgacc tgcaggatgg gagacggctg ctggatctgc tggaaggact gactggccag     180
   aagctgccca aagagaaggg gagcactagg gtgcacgccc tgaacaacgt gaacaaagct     240
   ctgagagtgc tgcagaacaa caacgtggat ctggtgaata ttggcagtac tgatatcgtg     300
   gacgggaacc acaaactgac actgggcctg atctggaaca ttattctgca ctggcaggtg     360
   aaaaatgtga tgaagaacat catggccggg ctgcagcaga ccaattccga gaagatcctg     420
   ctgtcttggg tgcggcagag cacccgcaac tatccccagg tgaacgtgat taacttcact     480
   acatcctgga gcgacgggct ggccctgaat gctctgattc acagccacag gcctgatctg     540
   ttcgactgga atagcgtggt gtgccagcag tctgccacac agcgcctgga acatgccttc     600
   aatatcgctc ggtaccagct ggggatcgaa aaactgctgg acccagagga tgtggacact     660
   acatacccag ataaaaagtc tattctgatg tacattacta gcctgttcca ggtgctgcca     720
   cagcaggtgt ctattgaagc cattcaggag gtggaaatgc tgccccgccc ccccaaagtg     780
   actaaagagg agcattttca gctgcatcat cagatgcatt acagccagca gattaccgtg     840
   agcctggctc agggatatga gcgcaccagt agtccaaaac cacggttcaa gtcctacgct     900
   tatacccagg ctgcctacgt gacaactagc gaccctacta gatccccctt tccatcccag     960
   cacctggagg ccccagagga caagagcttt gggtccagcc tgatggaaag cgaggtgaat    1020
   ctggatcggt accagacagc cctggaggag gtgctgagct ggctgctgag tgctgaagac    1080
   acactgcagg cccagggcga aatttccaat gacgtggaag tggtgaagga tcagttccac    1140
   acacacgagg gctatatgat ggacctgaca gctcaccagg ggcgcgtggg caatatcctg    1200
   cagctgggct ctaaactgat cggcaccggg aaactgagtg aggacgagga aacagaagtg    1260
   caggagcaga tgaacctgct gaacagccgc tgggagtgtc tgagagtggc tagtatggag    1320
   aagcagtcca acctgcaccg ggtgctgatg gacctgcaga accagaaact gaaagagctg    1380
   aacgactggc tgacaaagac tgaggaacgc acaaggaaga tggaggagga gccactggga    1440
   cccgacctgg aggatctgaa gagacaggtg cagcagcata aggtgctgca ggaggatctg    1500
   gaacaggagc aggtgcgggt gaactccctg acacatatgg tggtggtggt ggacgaatct 1560
   agtggagatc acgccaccgc cgccctggag gaacagctga aggtgctggg ggaccggtgg 1620
   gccaacattt gccggtggac cgaggacagg tgggtgctgc tgcaggacat cctgctgaaa 1680
   tggcagaggc tgaccgagga gcagtgtctg tttagtgctt ggctgagcga gaaagaggac 1740
   gccgtgaaca agatccacac aaccggcttt aaggatcaga acgaaatgct gtctagcctg 1800
   cagaaactgg ctgtgctgaa ggccgatctg gagaaaaaga agcagagcat gggcaaactg 1860
   tatagcctga aacaggacct gctgagcacc ctgaagaaca agagcgtgac ccagaagaca 1920
   gaagcctggc tggataactt tgcccgctgc tgggacaacc tggtgcagaa actggagaaa 1980
   agtacagctc agatctctca ggctgtgacc acaacccagc ctagcctgac ccagacaacc 2040
   gtgatggaaa ccgtgaccac cgtgacaacc cgcgaacaga tcctggtgaa acatgcccag 2100
   gaagagctgc cacctccacc tccccagaag aagagaaccc tggagcggct gcaggagctg 2160
   caggaagcca ctgacgaact ggacctgaag ctgaggcagg ccgaagtgat taaggggtct 2220
   tggcagcctg tgggcgatct gctgattgat tccctgcagg accacctgga aaaggtgaag 2280
   gctctgagag gcgaaattgc tccactgaag gagaacgtga gtcatgtgaa cgatctggct 2340
   agacagctga caacactggg catccagctg agcccataca atctgagcac actggaggac 2400
   ctgaatacca ggtggaagct gctgcaggtg gctgtggaag accgggtgcg gcagctgcat 2460
   gaggcccatc gcgacttcgg accagccagc cagcactttc tgagcacatc cgtgcagggg 2520
   ccctgggaga gggccatttc tcccaacaag gtgccctact atattaatca cgagacccag 2580
   accacttgtt gggaccatcc caagatgaca gaactgtacc agtccctggc cgatctgaac 2640
   aacgtgaggt ttagcgctta cagaaccgct atgaagctga gacggctgca gaaggccctg 2700
   tgcctggatc tgctgtccct gtccgccgcc tgcgatgccc tggatcagca taatctgaag 2760
   cagaacgatc agccaatgga tatcctgcag atcatcaact gcctgaccac tatctacgac 2820
   aggctggagc aggagcacaa caacctggtg aacgtgcctc tgtgcgtgga tatgtgcctg 2880
   aactggctgc tgaacgtgta tgacactggg cgcaccggcc ggatcagagt gctgagtttt 2940
   aaaactggga ttatctccct gtgtaaggcc cacctggagg acaagtacag gtacctgttc 3000
   aagcaggtgg ctagtagcac tggattttgt gaccagcgcc gcctgggact gctgctgcat 3060
   gatagtatcc agattcctag acagctggga gaggtggcta gtttcggagg atctaacatc 3120
   gaacccagcg tgcgcagctg tttccagttt gccaataaca aacctgaaat cgaggctgct 3180
   ctgttcctgg attggatgcg cctggaacca cagagcatgg tgtggctgcc tgtgctgcac 3240
   agagtggctg ccgccgaaac tgccaagcac caggctaaat gcaacatctg caaggaatgt 3300
   cccattatcg gctttcgcta caggagtctg aaacatttta actacgatat ttgccagagc 3360
   tgcttctttt ccggaagagt ggccaaagga cacaagatgc actaccctat ggtggaatat 3420
   tgcaccccaa ctacatctgg cgaagatgtg cgcgattttg ccaaggtgct gaagaataag 3480
   tttcggacta agaggtactt cgccaagcac ccccgcatgg ggtatctgcc agtgcagaca 3540
   gtgctggaag gagacaatat ggagaccgat acaatgtga 3579
<210> 2
   <211> 810
   <212> DNA
   <213> Adeno-associated virus
<400> 2
   gtttaaacaa gcttgcatgt ctaagctaga cccttcagat taaaaataac tgaggtaagg 60
   gcctgggtag gggaggtggt gtgagacgct cctgtctctc ctctatctgc ccatcggccc 120
   tttggggagg aggaatgtgc ccaaggacta aaaaaaggcc atggagccag aggggcgagg 180
   gcaacagacc tttcatgggc aaaccttggg gccctgctgt ctagcatgcc ccactacggg 240
   tctaggctgc ccatgtaagg aggcaaggcc tggggacacc cgagatgcct ggttataatt 300
   aacccagaca tgtggctgcc cccccccccc caacacctgc tgcctctaaa aataaccctg 360
   tccctggtgg atcccctgca tgcgaagatc ttcgaacaag gctgtggggg actgagggca 420
   ggctgtaaca ggcttggggg ccagggctta tacgtgcctg ggactcccaa agtattactg 480
   ttccatgttc ccggcgaagg gccagctgtc ccccgccagc tagactcagc acttagttta 540
   ggaaccagtg agcaagtcag cccttggggc agcccataca aggccatggg gctgggcaag 600
   ctgcacgcct gggtccgggg tgggcacggt gcccgggcaa cgagctgaaa gctcatctgc 660
   tctcaggggc ccctccctgg ggacagcccc tcctggctag tcacaccctg taggctcctc 720
   tatataaccc aggggcacag gggctgccct cattctacca ccacctccac agcacagaca 780
   gacactcagg agccagccag cggcgcgccc 810
<210> 3
   <211> 8562
   <212> DNA
   <213> Adeno-associated virus
<400> 3
   gcccaatacg caaaccgcct ctccccgcgc gttggccgat tcattaatgc agctgcgcgc 60
   tcgctcgctc actgaggccg cccgggcaaa gcccgggcgt cgggcgacct ttggtcgccc 120
   ggcctcagtg agcgagcgag cgcgcagaga gggagtggcc aactccatca ctaggggttc 180
   cttgtagtta atgattaacc cgccatgcta cttatctacg tagccatgct ctagagttta 240
   aacaagcttg catgtctaag ctagaccctt cagattaaaa ataactgagg taagggcctg 300
   ggtaggggag gtggtgtgag acgctcctgt ctctcctcta tctgcccatc ggccctttgg 360
   ggaggaggaa tgtgcccaag gactaaaaaa aggccatgga gccagagggg cgagggcaac 420
   agacctttca tgggcaaacc ttggggccct gctgtctagc atgccccact acgggtctag 480
   gctgcccatg taaggaggca aggcctgggg acacccgaga tgcctggtta taattaaccc 540
   agacatgtgg ctgccccccc ccccccaaca cctgctgcct ctaaaaataa ccctgtccct 600
   ggtggatccc ctgcatgcga agatcttcga acaaggctgt gggggactga gggcaggctg 660
   taacaggctt gggggccagg gcttatacgt gcctgggact cccaaagtat tactgttcca 720
   tgttcccggc gaagggccag ctgtcccccg ccagctagac tcagcactta gtttaggaac     780
   cagtgagcaa gtcagccctt ggggcagccc atacaaggcc atggggctgg gcaagctgca     840
   cgcctgggtc cggggtgggc acggtgcccg ggcaacgagc tgaaagctca tctgctctca     900
   ggggcccctc cctggggaca gcccctcctg gctagtcaca ccctgtaggc tcctctatat     960
   aacccagggg cacaggggct gccctcattc taccaccacc tccacagcac agacagacac    1020
   tcaggagcag ccagcggcgc gcccaggtaa gtttagtctt tttgtctttt atttcaggtc    1080
   ccggatccgg tggtggtgca aatcaaagaa ctgctcctca gtggatgttg cctttacttc    1140
   taggcctgta cggaagtgtt acttctgctc taaaagctgc ggaattgtac ccgcggccgc    1200
   caccatgctg tggtgggagg aggtggagga ttgttatgaa agggaggacg tgcagaagaa    1260
   gacttttacc aagtgggtga acgctcagtt cagcaaattt gggaagcagc acatcgagaa    1320
   tctgttttcc gacctgcagg atgggagacg gctgctggat ctgctggaag gactgactgg    1380
   ccagaagctg cccaaagaga aggggagcac tagggtgcac gccctgaaca acgtgaacaa    1440
   agctctgaga gtgctgcaga acaacaacgt ggatctggtg aatattggca gtactgatat    1500
   cgtggacggg aaccacaaac tgacactggg cctgatctgg aacattattc tgcactggca    1560
   ggtgaaaaat gtgatgaaga acatcatggc cgggctgcag cagaccaatt ccgagaagat    1620
   cctgctgtct tgggtgcggc agagcacccg caactatccc caggtgaacg tgattaactt    1680
   cactacatcc tggagcgacg ggctggccct gaatgctctg attcacagcc acaggcctga    1740
   tctgttcgac tggaatagcg tggtgtgcca gcagtctgcc acacagcgcc tggaacatgc    1800
   cttcaatatc gctcggtacc agctggggat cgaaaaactg ctggacccag aggatgtgga    1860
   cactacatac ccagataaaa agtctattct gatgtacatt actagcctgt tccaggtgct    1920
   gccacagcag gtgtctattg aagccattca ggaggtggaa atgctgcccc gcccccccaa    1980
   agtgactaaa gaggagcatt ttcagctgca tcatcagatg cattacagcc agcagattac    2040
   cgtgagcctg gctcagggat atgagcgcac cagtagtcca aaaccacggt tcaagtccta    2100
   cgcttatacc caggctgcct acgtgacaac tagcgaccct actagatccc cctttccatc    2160
   ccagcacctg gaggccccag aggacaagag ctttgggtcc agcctgatgg aaagcgaggt    2220
   gaatctggat cggtaccaga cagccctgga ggaggtgctg agctggctgc tgagtgctga    2280
   agacacactg caggcccagg gcgaaatttc caatgacgtg gaagtggtga aggatcagtt    2340
   ccacacacac gagggctata tgatggacct gacagctcac caggggcgcg tgggcaatat    2400
   cctgcagctg ggctctaaac tgatcggcac cgggaaactg agtgaggacg aggaaacaga    2460
   agtgcaggag cagatgaacc tgctgaacag ccgctgggag tgtctgagag tggctagtat    2520
   ggagaagcag tccaacctgc accgggtgct gatggacctg cagaaccaga aactgaaaga    2580
   gctgaacgac tggctgacaa agactgagga acgcacaagg aagatggagg aggagccact    2640
   gggacccgac ctggaggatc tgaagagaca ggtgcagcag cataaggtgc tgcaggagga    2700
   tctggaacag gagcaggtgc gggtgaactc cctgacacat atggtggtgg tggtggacga    2760
   atctagtgga gatcacgcca ccgccgccct ggaggaacag ctgaaggtgc tgggggaccg 2820
   gtgggccaac atttgccggt ggaccgagga caggtgggtg ctgctgcagg acatcctgct 2880
   gaaatggcag aggctgaccg aggagcagtg tctgtttagt gcttggctga gcgagaaaga 2940
   ggacgccgtg aacaagatcc acacaaccgg ctttaaggat cagaacgaaa tgctgtctag 3000
   cctgcagaaa ctggctgtgc tgaaggccga tctggagaaa aagaagcaga gcatgggcaa 3060
   actgtatagc ctgaaacagg acctgctgag caccctgaag aacaagagcg tgacccagaa 3120
   gacagaagcc tggctggata actttgcccg ctgctgggac aacctggtgc agaaactgga 3180
   gaaaagtaca gctcagatct ctcaggctgt gaccacaacc cagcctagcc tgacccagac 3240
   aaccgtgatg gaaaccgtga ccaccgtgac aacccgcgaa cagatcctgg tgaaacatgc 3300
   ccaggaagag ctgccacctc cacctcccca gaagaagaga accctggagc ggctgcagga 3360
   gctgcaggaa gccactgacg aactggacct gaagctgagg caggccgaag tgattaaggg 3420
   gtcttggcag cctgtgggcg atctgctgat tgattccctg caggaccacc tggaaaaggt 3480
   gaaggctctg agaggcgaaa ttgctccact gaaggagaac gtgagtcatg tgaacgatct 3540
   ggctagacag ctgacaacac tgggcatcca gctgagccca tacaatctga gcacactgga 3600
   ggacctgaat accaggtgga agctgctgca ggtggctgtg gaagaccggg tgcggcagct 3660
   gcatgaggcc catcgcgact tcggaccagc cagccagcac tttctgagca catccgtgca 3720
   ggggccctgg gagagggcca tttctcccaa caaggtgccc tactatatta atcacgagac 3780
   ccagaccact tgttgggacc atcccaagat gacagaactg taccagtccc tggccgatct 3840
   gaacaacgtg aggtttagcg cttacagaac cgctatgaag ctgagacggc tgcagaaggc 3900
   cctgtgcctg gatctgctgt ccctgtccgc cgcctgcgat gccctggatc agcataatct 3960
   gaagcagaac gatcagccaa tggatatcct gcagatcatc aactgcctga ccactatcta 4020
   cgacaggctg gagcaggagc acaacaacct ggtgaacgtg cctctgtgcg tggatatgtg 4080
   cctgaactgg ctgctgaacg tgtatgacac tgggcgcacc ggccggatca gagtgctgag 4140
   ttttaaaact gggattatct ccctgtgtaa ggcccacctg gaggacaagt acaggtacct 4200
   gttcaagcag gtggctagta gcactggatt ttgtgaccag cgccgcctgg gactgctgct 4260
   gcatgatagt atccagattc ctagacagct gggagaggtg gctagtttcg gaggatctaa 4320
   catcgaaccc agcgtgcgca gctgtttcca gtttgccaat aacaaacctg aaatcgaggc 4380
   tgctctgttc ctggattgga tgcgcctgga accacagagc atggtgtggc tgcctgtgct 4440
   gcacagagtg gctgccgccg aaactgccaa gcaccaggct aaatgcaaca tctgcaagga 4500
   atgtcccatt atcggctttc gctacaggag tctgaaacat tttaactacg atatttgcca 4560
   gagctgcttc ttttccggaa gagtggccaa aggacacaag atgcactacc ctatggtgga 4620
   atattgcacc ccaactacat ctggcgaaga tgtgcgcgat tttgccaagg tgctgaagaa 4680
   taagtttcgg actaagaggt acttcgccaa gcacccccgc atggggtatc tgccagtgca 4740
   gacagtgctg gaaggagaca atatggagac cgatacaatg tgagcggccg caataaaaga 4800
   tctttatttt cattagatct gtgtgttggt tttttgtgtg tctagagcat ggctacgtag 4860
   ataagtagca tggcgggtta atcattaact acaaggaacc cctagtgatg gagttggcca 4920
   ctccctctct gcgcgctcgc tcgctcactg aggccgggcg accaaaggtc gcccgacgcc 4980
   cgggctttgc ccgggcggcc tcagtgagcg agcgagcgcg cagctggcgt aatagcgaag 5040
   aggcccgcac cgatcgccct tcccaacagt tgcgcagcct gaatggcgaa tggcgattcc 5100
   gttgcaatgg ctggcggtaa tattgttctg gatattacca gcaaggccga tagtttgagt 5160
   tcttctactc aggcaagtga tgttattact aatcaaagaa gtattgcgac aacggttaat 5220
   ttgcgtgatg gacagactct tttactcggt ggcctcactg attataaaaa cacttctcag 5280
   gattctggcg taccgttcct gtctaaaatc cctttaatcg gcctcctgtt tagctcccgc 5340
   tctgattcta acgaggaaag cacgttatac gtgctcgtca aagcaaccat agtacgcgcc 5400
   ctgtagcggc gcattaagcg cggcgggtgt ggtggttacg cgcagcgtga ccgctacact 5460
   tgccagcgcc ctagcgcccg ctcctttcgc tttcttccct tcctttctcg ccacgttcgc 5520
   cggctttccc cgtcaagctc taaatcgggg gctcccttta gggttccgat ttagtgcttt 5580
   acggcacctc gaccccaaaa aacttgatta gggtgatggt tcacgtagtg ggccatcgcc 5640
   ctgatagacg gtttttcgcc ctttgacgtt ggagtccacg ttctttaata gtggactctt 5700
   gttccaaact ggaacaacac tcaaccctat ctcggtctat tcttttgatt tataagggat 5760
   tttgccgatt tcggcctatt ggttaaaaaa tgagctgatt taacaaaaat ttaacgcgaa 5820
   ttttaacaaa atattaacgc ttacaattta aatatttgct tatacaatct tcctgttttt 5880
   ggggcttttc tgattatcaa ccggggtaca tatgattgac atgctagttt tacgattacc 5940
   gttcatcgat tctcttgttt gctccagact ctcaggcaat gacctgatag cctttgtaga 6000
   gacctctcaa aaatagctac cctctccggc atgaatttat cagctagaac ggttgaatat 6060
   catattgatg gtgatttgac tgtctccggc ctttctcacc cgtttgaatc tttacctaca 6120
   cattactcag gcattgcatt taaaatatat gagggttcta aaaattttta tccttgcgtt 6180
   gaaataaagg cttctcccgc aaaagtatta cagggtcata atgtttttgg tacaaccgat 6240
   ttagctttat gctctgaggc tttattgctt aattttgcta attctttgcc ttgcctgtat 6300
   gatttattgg atgttggaat cgcctgatgc ggtattttct ccttacgcat ctgtgcggta 6360
   tttcacaccg catatggtgc actctcagta caatctgctc tgatgccgca tagttaagcc 6420
   agccccgaca cccgccaaca cccgctgacg cgccctgacg ggcttgtctg ctcccggcat 6480
   ccgcttacag acaagctgtg accgtctccg ggagctgcat gtgtcagagg ttttcaccgt 6540
   catcaccgaa acgcgcgaga cgaaagggcc tcgtgatacg cctattttta taggttaatg 6600
   tcatgataat aatggtttct tagacgtcag gtggcacttt tcggggaaat gtgcgcggaa 6660
   cccctatttg tttatttttc taaatacatt caaatatgta tccgctcatg agacaataac 6720
   cctgataaat gcttcaataa tattgaaaaa ggaagagtat gagtattcaa catttccgtg 6780
   tcgcccttat tccctttttt gcggcatttt gccttcctgt ttttgctcac ccagaaacgc 6840
   tggtgaaagt aaaagatgct gaagatcagt tgggtgcacg agtgggttac atcgaactgg 6900
   atctcaacag cggtaagatc cttgagagtt ttcgccccga agaacgtttt ccaatgatga 6960
   gcacttttaa agttctgcta tgtggcgcgg tattatcccg tattgacgcc gggcaagagc 7020
   aactcggtcg ccgcatacac tattctcaga atgacttggt tgagtactca ccagtcacag 7080
   aaaagcatct tacggatggc atgacagtaa gagaattatg cagtgctgcc ataaccatga 7140
   gtgataacac tgcggccaac ttacttctga caacgatcgg aggaccgaag gagctaaccg 7200
   cttttttgca caacatgggg gatcatgtaa ctcgccttga tcgttgggaa ccggagctga 7260
   atgaagccat accaaacgac gagcgtgaca ccacgatgcc tgtagcaatg gcaacaacgt 7320
   tgcgcaaact attaactggc gaactactta ctctagcttc ccggcaacaa ttaatagact 7380
   ggatggaggc ggataaagtt gcaggaccac ttctgcgctc ggcccttccg gctggctggt 7440
   ttattgctga taaatctgga gccggtgagc gtgggtctcg cggtatcatt gcagcactgg 7500
   ggccagatgg taagccctcc cgtatcgtag ttatctacac gacggggagt caggcaacta 7560
   tggatgaacg aaatagacag atcgctgaga taggtgcctc actgattaag cattggtaac 7620
   tgtcagacca agtttactca tatatacttt agattgattt aaaacttcat ttttaattta 7680
   aaaggatcta ggtgaagatc ctttttgata atctcatgac caaaatccct taacgtgagt 7740
   tttcgttcca ctgagcgtca gaccccgtag aaaagatcaa aggatcttct tgagatcctt 7800
   tttttctgcg cgtaatctgc tgcttgcaaa caaaaaaacc accgctacca gcggtggttt 7860
   gtttgccgga tcaagagcta ccaactcttt ttccgaaggt aactggcttc agcagagcgc 7920
   agataccaaa tactgttctt ctagtgtagc cgtagttagg ccaccacttc aagaactctg 7980
   tagcaccgcc tacatacctc gctctgctaa tcctgttacc agtggctgct gccagtggcg 8040
   ataagtcgtg tcttaccggg ttggactcaa gacgatagtt accggataag gcgcagcggt 8100
   cgggctgaac ggggggttcg tgcacacagc ccagcttgga gcgaacgacc tacaccgaac 8160
   tgagatacct acagcgtgag ctatgagaaa gcgccacgct tcccgaaggg agaaaggcgg 8220
   acaggtatcc ggtaagcggc agggtcggaa caggagagcg cacgagggag cttccagggg 8280
   gaaacgcctg gtatctttat agtcctgtcg ggtttcgcca cctctgactt gagcgtcgat 8340
   ttttgtgatg ctcgtcaggg gggcggagcc tatggaaaaa cgccagcaac gcggcctttt 8400
   tacggttcct ggccttttgc tggccttttg ctcacatgtt ctttcctgcg ttatcccctg 8460
   attctgtgga taaccgtatt accgcctttg agtgagctga taccgctcgc cgcagccgaa 8520
   cgaccgagcg cagcgagtca gtgagcgagg aagcggaaga gc 8562
<210> 4
   <211> 564
   <212> DNA
   <213> Adeno-associated virus
<400> 4
   cagccactat gggtctaggc tgcccatgta aggaggcaag gcctggggac acccgagatg       60
   cctggttata attaacccag acatgtggct gctccccccc cccaacacct gctgcctgag      120
   cctcaccccc accccggtgc ctgggtctta ggctctgtac accatggagg agaagctcgc      180
   tctaaaaata accctgtccc tggtgggctg tgggggactg agggcaggct gtaacaggct      240
   tgggggccag ggcttatacg tgcctgggac tcccaaagta ttactgttcc atgttcccgg      300
   cgaagggcca gctgtccccc gccagctaga ctcagcactt agtttaggaa ccagtgagca      360
   agtcagccct tggggcagcc catacaaggc catggggctg ggcaagctgc acgcctgggt      420
   ccggggtggg cacggtgccc gggcaacgag ctgaaagctc atctgctctc aggggcccct      480
   ccctggggac agcccctcct ggctagtcac accctgtagg ctcctctata taacccaggg      540
   gcacaggggc tgcccccggg tcac                                             564
<210> 5
   <211> 8409
   <212> DNA
   <213> Adeno-associated virus
<400> 5
   gcccaatacg caaaccgcct ctccccgcgc gttggccgat tcattaatgc agctggcgcg 60
   ctcgctcgct cactgaggcc gcccgggcaa agcccgggcg tcgggcgacc tttggtcgcc 120
   cggcctcagt gagcgagcga gcgcgcagag agggagtggc caactccatc actaggggtt 180
   ccttgtagtt aatgattaac ccgccatgct aattatctac gtagccatgt ctagacagcc 240
   actatgggtc taggctgccc atgtaaggag gcaaggcctg gggacacccg agatgcctgg 300
   ttataattaa cccagacatg tggctgctcc ccccccccaa cacctgctgc ctgagcctca 360
   cccccacccc ggtgcctggg tcttaggctc tgtacaccat ggaggagaag ctcgctctaa 420
   aaataaccct gtccctggtg ggctgtgggg gactgagggc aggctgtaac aggcttgggg 480
   gccagggctt atacgtgcct gggactccca aagtattact gttccatgtt cccggcgaag 540
   ggccagctgt cccccgccag ctagactcag cacttagttt aggaaccagt gagcaagtca 600
   gcccttgggg cagcccatac aaggccatgg ggctgggcaa gctgcacgcc tgggtccggg 660
   gtgggcacgg tgcccgggca acgagctgaa agctcatctg ctctcagggg cccctccctg 720
   gggacagccc ctcctggcta gtcacaccct gtaggctcct ctatataacc caggggcaca 780
   ggggctgccc ccgggtcacc accacctcca cagcacagac agacactcag gagccagcca 840
   gccaggtaag tttagtcttt ttgtctttta tttcaggtcc cggatccggt ggtggtgcaa 900
   atcaaagaac tgctcctcag tggatgttgc ctttacttct aggcctgtac ggaagtgtta 960
   cttctgctct aaaagctgcg gaattgtacc cgcggccgcc accatgctgt ggtgggagga 1020
   ggtggaggat tgttatgaaa gggaggacgt gcagaagaag acttttacca agtgggtgaa 1080
   cgctcagttc agcaaatttg ggaagcagca catcgagaat ctgttttccg acctgcagga 1140
   tgggagacgg ctgctggatc tgctggaagg actgactggc cagaagctgc ccaaagagaa 1200
   ggggagcact agggtgcacg ccctgaacaa cgtgaacaaa gctctgagag tgctgcagaa 1260
   caacaacgtg gatctggtga atattggcag tactgatatc gtggacggga accacaaact 1320
   gacactgggc ctgatctgga acattattct gcactggcag gtgaaaaatg tgatgaagaa 1380
   catcatggcc gggctgcagc agaccaattc cgagaagatc ctgctgtctt gggtgcggca 1440
   gagcacccgc aactatcccc aggtgaacgt gattaacttc actacatcct ggagcgacgg 1500
   gctggccctg aatgctctga ttcacagcca caggcctgat ctgttcgact ggaatagcgt 1560
   ggtgtgccag cagtctgcca cacagcgcct ggaacatgcc ttcaatatcg ctcggtacca 1620
   gctggggatc gaaaaactgc tggacccaga ggatgtggac actacatacc cagataaaaa 1680
   gtctattctg atgtacatta ctagcctgtt ccaggtgctg ccacagcagg tgtctattga 1740
   agccattcag gaggtggaaa tgctgccccg cccccccaaa gtgactaaag aggagcattt 1800
   tcagctgcat catcagatgc attacagcca gcagattacc gtgagcctgg ctcagggata 1860
   tgagcgcacc agtagtccaa aaccacggtt caagtcctac gcttataccc aggctgccta 1920
   cgtgacaact agcgacccta ctagatcccc ctttccatcc cagcacctgg aggccccaga 1980
   ggacaagagc tttgggtcca gcctgatgga aagcgaggtg aatctggatc ggtaccagac 2040
   agccctggag gaggtgctga gctggctgct gagtgctgaa gacacactgc aggcccaggg 2100
   cgaaatttcc aatgacgtgg aagtggtgaa ggatcagttc cacacacacg agggctatat 2160
   gatggacctg acagctcacc aggggcgcgt gggcaatatc ctgcagctgg gctctaaact 2220
   gatcggcacc gggaaactga gtgaggacga ggaaacagaa gtgcaggagc agatgaacct 2280
   gctgaacagc cgctgggagt gtctgagagt ggctagtatg gagaagcagt ccaacctgca 2340
   ccgggtgctg atggacctgc agaaccagaa actgaaagag ctgaacgact ggctgacaaa 2400
   gactgaggaa cgcacaagga agatggagga ggagccactg ggacccgacc tggaggatct 2460
   gaagagacag gtgcagcagc ataaggtgct gcaggaggat ctggaacagg agcaggtgcg 2520
   ggtgaactcc ctgacacata tggtggtggt ggtggacgaa tctagtggag atcacgccac 2580
   cgccgccctg gaggaacagc tgaaggtgct gggggaccgg tgggccaaca tttgccggtg 2640
   gaccgaggac aggtgggtgc tgctgcagga catcctgctg aaatggcaga ggctgaccga 2700
   ggagcagtgt ctgtttagtg cttggctgag cgagaaagag gacgccgtga acaagatcca 2760
   cacaaccggc tttaaggatc agaacgaaat gctgtctagc ctgcagaaac tggctgtgct 2820
   gaaggccgat ctggagaaaa agaagcagag catgggcaaa ctgtatagcc tgaaacagga 2880
   cctgctgagc accctgaaga acaagagcgt gacccagaag acagaagcct ggctggataa 2940
   ctttgcccgc tgctgggaca acctggtgca gaaactggag aaaagtacag ctcagatctc 3000
   tcaggctgtg accacaaccc agcctagcct gacccagaca accgtgatgg aaaccgtgac 3060
   caccgtgaca acccgcgaac agatcctggt gaaacatgcc caggaagagc tgccacctcc 3120
   acctccccag aagaagagaa ccctggagcg gctgcaggag ctgcaggaag ccactgacga 3180
   actggacctg aagctgaggc aggccgaagt gattaagggg tcttggcagc ctgtgggcga 3240
   tctgctgatt gattccctgc aggaccacct ggaaaaggtg aaggctctga gaggcgaaat 3300
   tgctccactg aaggagaacg tgagtcatgt gaacgatctg gctagacagc tgacaacact 3360
   gggcatccag ctgagcccat acaatctgag cacactggag gacctgaata ccaggtggaa 3420
   gctgctgcag gtggctgtgg aagaccgggt gcggcagctg catgaggccc atcgcgactt 3480
   cggaccagcc agccagcact ttctgagcac atccgtgcag gggccctggg agagggccat 3540
   ttctcccaac aaggtgccct actatattaa tcacgagacc cagaccactt gttgggacca 3600
   tcccaagatg acagaactgt accagtccct ggccgatctg aacaacgtga ggtttagcgc 3660
   ttacagaacc gctatgaagc tgagacggct gcagaaggcc ctgtgcctgg atctgctgtc 3720
   cctgtccgcc gcctgcgatg ccctggatca gcataatctg aagcagaacg atcagccaat 3780
   ggatatcctg cagatcatca actgcctgac cactatctac gacaggctgg agcaggagca 3840
   caacaacctg gtgaacgtgc ctctgtgcgt ggatatgtgc ctgaactggc tgctgaacgt 3900
   gtatgacact gggcgcaccg gccggatcag agtgctgagt tttaaaactg ggattatctc 3960
   cctgtgtaag gcccacctgg aggacaagta caggtacctg ttcaagcagg tggctagtag 4020
   cactggattt tgtgaccagc gccgcctggg actgctgctg catgatagta tccagattcc 4080
   tagacagctg ggagaggtgg ctagtttcgg aggatctaac atcgaaccca gcgtgcgcag 4140
   ctgtttccag tttgccaata acaaacctga aatcgaggct gctctgttcc tggattggat 4200
   gcgcctggaa ccacagagca tggtgtggct gcctgtgctg cacagagtgg ctgccgccga 4260
   aactgccaag caccaggcta aatgcaacat ctgcaaggaa tgtcccatta tcggctttcg 4320
   ctacaggagt ctgaaacatt ttaactacga tatttgccag agctgcttct tttccggaag 4380
   agtggccaaa ggacacaaga tgcactaccc tatggtggaa tattgcaccc caactacatc 4440
   tggcgaagat gtgcgcgatt ttgccaaggt gctgaagaat aagtttcgga ctaagaggta 4500
   cttcgccaag cacccccgca tggggtatct gccagtgcag acagtgctgg aaggagacaa 4560
   tatggagacc gatacaatgt gagcggccgc aataaaagat ctttattttc attagatctg 4620
   tgtgttggtt ttttgtgtgt ctagagcatg gctacgtaga taagtagcat ggcgggttaa 4680
   tcattaacta caaggaaccc ctagtgatgg agttggccac tccctctctg cgcgctcgct 4740
   cgctcactga ggccgggcga ccaaaggtcg cccgacgccc gggctttgcc cgggcggcct 4800
   cagtgagcga gcgagcgcgc cagctggcgt aatagcgaag aggcccgcac cgatcgccct 4860
   tcccaacagt tgcgcagcct gaatggcgaa tggaagttcc agacgattga gcgtcaaaat 4920
   gtaggtattt ccatgagcgt ttttcctgtt gcaatggctg gcggtaatat tgttctggat 4980
   attaccagca aggccgatag tttgagttct tctactcagg caagtgatgt tattactaat 5040
   caaagaagta ttgcgacaac ggttaatttg cgtgatggac agactctttt actcggtggc 5100
   ctcactgatt ataaaaacac ttctcaggat tctggcgtac cgttcctgtc taaaatccct 5160
   ttaatcggcc tcctgtttag ctcccgctct gattctaacg aggaaagcac gttatacgtg 5220
   ctcgtcaaag caaccatagt acgcgccctg tagcggcgca ttaagcgcgg cgggtgtggt 5280
   ggttacgcgc agcgtgaccg ctacacttgc cagcgcccta gcgcccgctc ctttcgcttt 5340
   cttcccttcc tttctcgcca cgttcgccgg ctttccccgt caagctctaa atcgggggct 5400
   ccctttaggg ttccgattta gtgatttacg gcacctcgac cccaaaaaac ttgattaggg 5460
   tgatggttca cgtagtgggc catcgccctg atagacggtt tttcgccctt tgacgttgga 5520
   gtccacgttc tttaatagtg gactcttgtt ccaaactgga acaacactca accctatctc 5580
   ggtctattct tttgatttat aagggatttt gccgatttcg gcctattggt taaaaaatga 5640
   gctgatttaa caaaaattta acgcgaattt taacaaaata ttaacgttta caatttaaat 5700
   atttgcttat acaatcttcc tgtttttggg gcttttctga ttatcaaccg gggtacatat 5760
   gattgacatg ctagttttac gattaccgtt catcgattct cttgtttgct ccagactctc 5820
   aggcaatgac ctgatagcct ttgtagagac ctctcaaaaa tagctaccct ctccggcatg 5880
   aatttatcag ctagaacggt tgaatatcat attgatggtg atttgactgt ctccggcctt 5940
   tctcacccgt ttgaatcttt acctacacat tactcaggca ttgcatttaa aatatatgag 6000
   ggttctaaaa atttttatcc ttgcgttgaa ataaaggctt ctcccgcaaa agtattacag 6060
   ggtcataatg tttttggtac aaccgattta gctttatgct ctgaggcttt attgcttaat 6120
   tttgctaatt ctttgccttg cctgtatgat ttattggatg ttggaagttc ctgatgcggt 6180
   attttctcct tacgcatctg tgcggtattt cacaccgcat atggtgcact ctcagtacaa 6240
   tctgctctga tgccgcatag ttaagccagc cccgacaccc gccaacaccc gctgacgcgc 6300
   cctgacgggc ttgtctgctc ccggcatccg cttacagaca agctgtgacc gtctccggga 6360
   gctgcatgtg tcagaggttt tcaccgtcat caccgaaacg cgcgagacga aagggcctcg 6420
   tgatacgcct atttttatag gttaatgtca tgataataat ggtttcttag acgtcaggtg 6480
   gcacttttcg gggaaatgtg cgcggaaccc ctatttgttt atttttctaa atacattcaa 6540
   atatgtatcc gctcatgaga caataaccct gataaatgct tcaataatat tgaaaaagga 6600
   agagtatgag tattcaacat ttccgtgtcg cccttattcc cttttttgcg gcattttgcc 6660
   ttcctgtttt tgctcaccca gaaacgctgg tgaaagtaaa agatgctgaa gatcagttgg 6720
   gtgcacgagt gggttacatc gaactggatc tcaacagcgg taagatcctt gagagttttc 6780
   gccccgaaga acgttttcca atgatgagca cttttaaagt tctgctatgt ggcgcggtat 6840
   tatcccgtat tgacgccggg caagagcaac tcggtcgccg catacactat tctcagaatg 6900
   acttggttga gtactcacca gtcacagaaa agcatcttac ggatggcatg acagtaagag 6960
   aattatgcag tgctgccata accatgagtg ataacactgc ggccaactta cttctgacaa 7020
   cgatcggagg accgaaggag ctaaccgctt ttttgcacaa catgggggat catgtaactc 7080
   gccttgatcg ttgggaaccg gagctgaatg aagccatacc aaacgacgag cgtgacacca 7140
   cgatgcctgt agcaatggca acaacgttgc gcaaactatt aactggcgaa ctacttactc 7200
   tagcttcccg gcaacaatta atagactgga tggaggcgga taaagttgca ggaccacttc 7260
   tgcgctcggc ccttccggct ggctggttta ttgctgataa atctggagcc ggtgagcgtg 7320
   ggtctcgcgg tatcattgca gcactggggc cagatggtaa gccctcccgt atcgtagtta 7380
   tctacacgac ggggagtcag gcaactatgg atgaacgaaa tagacagatc gctgagatag 7440
   gtgcctcact gattaagcat tggtaactgt cagaccaagt ttactcatat atactttaga     7500
   ttgatttaaa acttcatttt taatttaaaa ggatctaggt gaagatcctt tttgataatc     7560
   tcatgaccaa aatcccttaa cgtgagtttt cgttccactg agcgtcagac cccgtagaaa     7620
   agatcaaagg atcttcttga gatccttttt ttctgcgcgt aatctgctgc ttgcaaacaa     7680
   aaaaaccacc gctaccagcg gtggtttgtt tgccggatca agagctacca actctttttc     7740
   cgaaggtaac tggcttcagc agagcgcaga taccaaatac tgtccttcta gtgtagccgt     7800
   agttaggcca ccacttcaag aactctgtag caccgcgtac atacctcgct ctgctaatcc     7860
   tgttaccagt ggctgctgcc agtggcgata agtcgtgtct taccgggttg gactcaagac     7920
   gatagttacc ggataaggcg cagcggtcgg gctgaacggg gggttcgtgc acacagccca     7980
   gcttggagcg aacgacctac accgaactga gatacctaca gcgtgagcta tgagaaagcg     8040
   ccacgcttcc cgaagggaga aaggcggaca ggtatccggt aagcggcagg gtcggaacag     8100
   gagagcgcac gagggagctt ccagggggaa acgcctggta tctttatagt cctgtcgggt     8160
   ttcgccacct ctgacttgag cgtcgatttt tgtgatgctc gtcagggggg cggagcctat     8220
   ggaaaaacgc cagcaacgcg gcctttttac ggttcctggc cttttgctgg ccttttgctc     8280
   acatgttctt tcctgcgtta tcccctgatt ctgtggataa ccgtattacc gggtttgagt     8340
   gagctgatac cgctcgccgc agccgaacga ccgagcgcag cgagtcagtg agcgaccaag     8400
   cggaagagc                                                             8409
<210> 6
   <211> 8611
   <212> DNA
   <213> Adeno-associated virus
<400> 6
   gcccaatacg caaaccgcct ctccccgcgc gttggccgat tcattaatgc agctggcgcg       60
   ctcgctcgct cactgaggcc gcccgggcaa agcccgggcg tcgggcgacc tttggtcgcc      120
   cggcctcagt gagcgagcga gcgcgcagag agggagtggc caactccatc actaggggtt      180
   ccttgtagtt aatgattaac ccgccatgct aattatctac gtagccatgt ctagagttta      240
   aacaagcttg catgtctaag ctagaccctt cagattaaaa ataactgagg taagggcctg      300
   ggtaggggag gtggtgtgag acgctcctgt ctctcctcta tctgcccatc ggccctttgg      360
   ggaggaggaa tgtgcccaag gactaaaaaa aggccatgga gccagagggg cgagggcaac      420
   agacctttca tgggcaaacc ttggggccct gctgtctagc atgccccact acgggtctag      480
   gctgcccatg taaggaggca aggcctgggg acacccgaga tgcctggtta taattaaccc      540
   agacatgtgg ctgccccccc ccccccaaca cctgctgcct ctaaaaataa ccctgtccct      600
   ggtggatccc ctgcatgcga agatcttcga acaaggctgt gggggactga gggcaggctg      660
   taacaggctt gggggccagg gcttatacgt gcctgggact cccaaagtat tactgttcca      720
   tgttcccggc gaagggccag ctgtcccccg ccagctagac tcagcactta gtttaggaac      780
   cagtgagcaa gtcagccctt ggggcagccc atacaaggcc atggggctgg gcaagctgca 840
   cgcctgggtc cggggtgggc acggtgcccg ggcaacgagc tgaaagctca tctgctctca 900
   ggggcccctc cctggggaca gcccctcctg gctagtcaca ccctgtaggc tcctctatat 960
   aacccagggg cacaggggct gccctcattc taccaccacc tccacagcac agacagacac 1020
   tcaggagcca gccagcggcg cgcccaggta agtttagtct ttttgtcttt tatttcaggt 1080
   cccggatccg gtggtggtgc aaatcaaaga actgctcctc agtggatgtt gcctttactt 1140
   ctaggcctgt acggaagtgt tacttctgct ctaaaagctg cggaattgta cccgcggccg 1200
   ccaccatgct gtggtgggag gaggtggagg attgttatga aagggaggac gtgcagaaga 1260
   agacttttac caagtgggtg aacgctcagt tcagcaaatt tgggaagcag cacatcgaga 1320
   atctgttttc cgacctgcag gatgggagac ggctgctgga tctgctggaa ggactgactg 1380
   gccagaagct gcccaaagag aaggggagca ctagggtgca cgccctgaac aacgtgaaca 1440
   aagctctgag agtgctgcag aacaacaacg tggatctggt gaatattggc agtactgata 1500
   tcgtggacgg gaaccacaaa ctgacactgg gcctgatctg gaacattatt ctgcactggc 1560
   aggtgaaaaa tgtgatgaag aacatcatgg ccgggctgca gcagaccaat tccgagaaga 1620
   tcctgctgtc ttgggtgcgg cagagcaccc gcaactatcc ccaggtgaac gtgattaact 1680
   tcactacatc ctggagcgac gggctggccc tgaatgctct gattcacagc cacaggcctg 1740
   atctgttcga ctggaatagc gtggtgtgcc agcagtctgc cacacagcgc ctggaacatg 1800
   ccttcaatat cgctcggtac cagctgggga tcgaaaaact gctggaccca gaggatgtgg 1860
   acactacata cccagataaa aagtctattc tgatgtacat tactagcctg ttccaggtgc 1920
   tgccacagca ggtgtctatt gaagccattc aggaggtgga aatgctgccc cgccccccca 1980
   aagtgactaa agaggagcat tttcagctgc atcatcagat gcattacagc cagcagatta 2040
   ccgtgagcct ggctcaggga tatgagcgca ccagtagtcc aaaaccacgg ttcaagtcct 2100
   acgcttatac ccaggctgcc tacgtgacaa ctagcgaccc tactagatcc ccctttccat 2160
   cccagcacct ggaggcccca gaggacaaga gctttgggtc cagcctgatg gaaagcgagg 2220
   tgaatctgga tcggtaccag acagccctgg aggaggtgct gagctggctg ctgagtgctg 2280
   aagacacact gcaggcccag ggcgaaattt ccaatgacgt ggaagtggtg aaggatcagt 2340
   tccacacaca cgagggctat atgatggacc tgacagctca ccaggggcgc gtgggcaata 2400
   tcctgcagct gggctctaaa ctgatcggca ccgggaaact gagtgaggac gaggaaacag 2460
   aagtgcagga gcagatgaac ctgctgaaca gccgctggga gtgtctgaga gtggctagta 2520
   tggagaagca gtccaacctg caccgggtgc tgatggacct gcagaaccag aaactgaaag 2580
   agctgaacga ctggctgaca aagactgagg aacgcacaag gaagatggag gaggagccac 2640
   tgggacccga cctggaggat ctgaagagac aggtgcagca gcataaggtg ctgcaggagg 2700
   atctggaaca ggagcaggtg cgggtgaact ccctgacaca tatggtggtg gtggtggacg 2760
   aatctagtgg agatcacgcc accgccgccc tggaggaaca gctgaaggtg ctgggggacc 2820
   ggtgggccaa catttgccgg tggaccgagg acaggtgggt gctgctgcag gacatcctgc 2880
   tgaaatggca gaggctgacc gaggagcagt gtctgtttag tgcttggctg agcgagaaag 2940
   aggacgccgt gaacaagatc cacacaaccg gctttaagga tcagaacgaa atgctgtcta 3000
   gcctgcagaa actggctgtg ctgaaggccg atctggagaa aaagaagcag agcatgggca 3060
   aactgtatag cctgaaacag gacctgctga gcaccctgaa gaacaagagc gtgacccaga 3120
   agacagaagc ctggctggat aactttgccc gctgctggga caacctggtg cagaaactgg 3180
   agaaaagtac agctcagatc tctcaggctg tgaccacaac ccagcctagc ctgacccaga 3240
   caaccgtgat ggaaaccgtg accaccgtga caacccgcga acagatcctg gtgaaacatg 3300
   cccaggaaga gctgccacct ccacctcccc agaagaagag aaccctggag cggctgcagg 3360
   agctgcagga agccactgac gaactggacc tgaagctgag gcaggccgaa gtgattaagg 3420
   ggtcttggca gcctgtgggc gatctgctga ttgattccct gcaggaccac ctggaaaagg 3480
   tgaaggctct gagaggcgaa attgctccac tgaaggagaa cgtgagtcat gtgaacgatc 3540
   tggctagaca gctgacaaca ctgggcatcc agctgagccc atacaatctg agcacactgg 3600
   aggacctgaa taccaggtgg aagctgctgc aggtggctgt ggaagaccgg gtgcggcagc 3660
   tgcatgaggc ccatcgcgac ttcggaccag ccagccagca ctttctgagc acatccgtgc 3720
   aggggccctg ggagagggcc atttctccca acaaggtgcc ctactatatt aatcacgaga 3780
   cccagaccac ttgttgggac catcccaaga tgacagaact gtaccagtcc ctggccgatc 3840
   tgaacaacgt gaggtttagc gcttacagaa ccgctatgaa gctgagacgg ctgcagaagg 3900
   ccctgtgcct ggatctgctg tccctgtccg ccgcctgcga tgccctggat cagcataatc 3960
   tgaagcagaa cgatcagcca atggatatcc tgcagatcat caactgcctg accactatct 4020
   acgacaggct ggagcaggag cacaacaacc tggtgaacgt gcctctgtgc gtggatatgt 4080
   gcctgaactg gctgctgaac gtgtatgaca ctgggcgcac cggccggatc agagtgctga 4140
   gttttaaaac tgggattatc tccctgtgta aggcccacct ggaggacaag tacaggtacc 4200
   tgttcaagca ggtggctagt agcactggat tttgtgacca gcgccgcctg ggactgctgc 4260
   tgcatgatag tatccagatt cctagacagc tgggagaggt ggctagtttc ggaggatcta 4320
   acatcgaacc cagcgtgcgc agctgtttcc agtttgccaa taacaaacct gaaatcgagg 4380
   ctgctctgtt cctggattgg atgcgcctgg aaccacagag catggtgtgg ctgcctgtgc 4440
   tgcacagagt ggctgccgcc gaaactgcca agcaccaggc taaatgcaac atctgcaagg 4500
   aatgtcccat tatcggcttt cgctacagga gtctgaaaca ttttaactac gatatttgcc 4560
   agagctgctt cttttccgga agagtggcca aaggacacaa gatgcactac cctatggtgg 4620
   aatattgcac cccaactaca tctggcgaag atgtgcgcga ttttgccaag gtgctgaaga 4680
   ataagtttcg gactaagagg tacttcgcca agcacccccg catggggtat ctgccagtgc 4740
   agacagtgct ggaaggagac aatatggaga ccgatacaat gtgagcggcc gcaataaaag 4800
   atctttattt tcattagatc tgtgtgttgg ttttttgtgt gtctagagca tggctacgta 4860
   gataagtagc atggcgggtt aatcattaac tacaaggaac ccctagtgat ggagttggcc 4920
   actccctctc tgcgcgctcg ctcgctcact gaggccgggc gaccaaaggt cgcccgacgc 4980
   ccgggctttg cccgggcggc ctcagtgagc gagcgagcgc gccagctggc gtaatagcga 5040
   agaggcccgc accgatcgcc cttcccaaca gttgcgcagc ctgaatggcg aatggaagtt 5100
   ccagacgatt gagcgtcaaa atgtaggtat ttccatgagc gtttttcctg ttgcaatggc 5160
   tggcggtaat attgttctgg atattaccag caaggccgat agtttgagtt cttctactca 5220
   ggcaagtgat gttattacta atcaaagaag tattgcgaca acggttaatt tgcgtgatgg 5280
   acagactctt ttactcggtg gcctcactga ttataaaaac acttctcagg attctggcgt 5340
   accgttcctg tctaaaatcc ctttaatcgg cctcctgttt agctcccgct ctgattctaa 5400
   cgaggaaagc acgttatacg tgctcgtcaa agcaaccata gtacgcgccc tgtagcggcg 5460
   cattaagcgc ggcgggtgtg gtggttacgc gcagcgtgac cgctacactt gccagcgccc 5520
   tagcgcccgc tcctttcgct ttcttccctt cctttctcgc cacgttcgcc ggctttcccc 5580
   gtcaagctct aaatcggggg ctccctttag ggttccgatt tagtgattta cggcacctcg 5640
   accccaaaaa acttgattag ggtgatggtt cacgtagtgg gccatcgccc tgatagacgg 5700
   tttttcgccc tttgacgttg gagtccacgt tctttaatag tggactcttg ttccaaactg 5760
   gaacaacact caaccctatc tcggtctatt cttttgattt ataagggatt ttgccgattt 5820
   cggcctattg gttaaaaaat gagctgattt aacaaaaatt taacgcgaat tttaacaaaa 5880
   tattaacgtt tacaatttaa atatttgctt atacaatctt cctgtttttg gggcttttct 5940
   gattatcaac cggggtacat atgattgaca tgctagtttt acgattaccg ttcatcgatt 6000
   ctcttgtttg ctccagactc tcaggcaatg acctgatagc ctttgtagag acctctcaaa 6060
   aatagctacc ctctccggca tgaatttatc agctagaacg gttgaatatc atattgatgg 6120
   tgatttgact gtctccggcc tttctcaccc gtttgaatct ttacctacac attactcagg 6180
   cattgcattt aaaatatatg agggttctaa aaatttttat ccttgcgttg aaataaaggc 6240
   ttctcccgca aaagtattac agggtcataa tgtttttggt acaaccgatt tagctttatg 6300
   ctctgaggct ttattgctta attttgctaa ttctttgcct tgcctgtatg atttattgga 6360
   tgttggaagt tcctgatgcg gtattttctc cttacgcatc tgtgcggtat ttcacaccgc 6420
   atatggtgca ctctcagtac aatctgctct gatgccgcat agttaagcca gccccgacac 6480
   ccgccaacac ccgctgacgc gccctgacgg gcttgtctgc tcccggcatc cgcttacaga 6540
   caagctgtga ccgtctccgg gagctgcatg tgtcagaggt tttcaccgtc atcaccgaaa 6600
   cgcgcgagac gaaagggcct cgtgatacgc ctatttttat aggttaatgt catgataata 6660
   atggtttctt agacgtcagg tggcactttt cggggaaatg tgcgcggaac ccctatttgt 6720
   ttatttttct aaatacattc aaatatgtat ccgctcatga gacaataacc ctgataaatg 6780
   cttcaataat attgaaaaag gaagagtatg agtattcaac atttccgtgt cgcccttatt 6840
   cccttttttg cggcattttg ccttcctgtt tttgctcacc cagaaacgct ggtgaaagta 6900
   aaagatgctg aagatcagtt gggtgcacga gtgggttaca tcgaactgga tctcaacagc 6960
   ggtaagatcc ttgagagttt tcgccccgaa gaacgttttc caatgatgag cacttttaaa 7020
   gttctgctat gtggcgcggt attatcccgt attgacgccg ggcaagagca actcggtcgc 7080
   cgcatacact attctcagaa tgacttggtt gagtactcac cagtcacaga aaagcatctt 7140
   acggatggca tgacagtaag agaattatgc agtgctgcca taaccatgag tgataacact 7200
   gcggccaact tacttctgac aacgatcgga ggaccgaagg agctaaccgc ttttttgcac 7260
   aacatggggg atcatgtaac tcgccttgat cgttgggaac cggagctgaa tgaagccata 7320
   ccaaacgacg agcgtgacac cacgatgcct gtagcaatgg caacaacgtt gcgcaaacta 7380
   ttaactggcg aactacttac tctagcttcc cggcaacaat taatagactg gatggaggcg 7440
   gataaagttg caggaccact tctgcgctcg gcccttccgg ctggctggtt tattgctgat 7500
   aaatctggag ccggtgagcg tgggtctcgc ggtatcattg cagcactggg gccagatggt 7560
   aagccctccc gtatcgtagt tatctacacg acggggagtc aggcaactat ggatgaacga 7620
   aatagacaga tcgctgagat aggtgcctca ctgattaagc attggtaact gtcagaccaa 7680
   gtttactcat atatacttta gattgattta aaacttcatt tttaatttaa aaggatctag 7740
   gtgaagatcc tttttgataa tctcatgacc aaaatccctt aacgtgagtt ttcgttccac 7800
   tgagcgtcag accccgtaga aaagatcaaa ggatcttctt gagatccttt ttttctgcgc 7860
   gtaatctgct gcttgcaaac aaaaaaacca ccgctaccag cggtggtttg tttgccggat 7920
   caagagctac caactctttt tccgaaggta actggcttca gcagagcgca gataccaaat 7980
   actgtccttc tagtgtagcc gtagttaggc caccacttca agaactctgt agcaccgcgt 8040
   acatacctcg ctctgctaat cctgttacca gtggctgctg ccagtggcga taagtcgtgt 8100
   cttaccgggt tggactcaag acgatagtta ccggataagg cgcagcggtc gggctgaacg 8160
   gggggttcgt gcacacagcc cagcttggag cgaacgacct acaccgaact gagataccta 8220
   cagcgtgagc tatgagaaag cgccacgctt cccgaaggga gaaaggcgga caggtatccg 8280
   gtaagcggca gggtcggaac aggagagcgc acgagggagc ttccaggggg aaacgcctgg 8340
   tatctttata gtcctgtcgg gtttcgccac ctctgacttg agcgtcgatt tttgtgatgc 8400
   tcgtcagggg ggcggagcct atggaaaaac gccagcaacg cggccttttt acggttcctg 8460
   gccttttgct ggccttttgc tcacatgttc tttcctgcgt tatcccctga ttctgtggat 8520
   aaccgtatta ccgggtttga gtgagctgat accgctcgcc gcagccgaac gaccgagcgc 8580
   agcgagtcag tgagcgacca agcggaagag c 8611
<210> 7
   <211> 792
   <212> DNA
   <213> Adeno-associated virus
<400> 7
   aagcttgcat gtctaagcta gacccttcag attaaaaata actgaggtaa gggcctgggt 60
   aggggaggtg gtgtgagacg ctcctgtctc tcctctatct gcccatcggc cctttgggga 120
   ggaggaatgt gcccaaggac taaaaaaagg ccatggagcc agaggggcga gggcaacaga 180
   cctttcatgg gcaaaccttg gggccctgct gtctagcatg ccccactacg ggtctaggct 240
   gcccatgtaa ggaggcaagg cctggggaca cccgagatgc ctggttataa ttaacccaga 300
   catgtggctg cccccccccc cccaacacct gctgcctcta aaaataaccc tgtccctggt 360
   ggatcccctg catgcgaaga tcttcgaaca aggctgtggg ggactgaggg caggctgtaa 420
   caggcttggg ggccagggct tatacgtgcc tgggactccc aaagtattac tgttccatgt 480
   tcccggcgaa gggccagctg tcccccgcca gctagactca gcacttagtt taggaaccag 540
   tgagcaagtc agcccttggg gcagcccata caaggccatg gggctgggca agctgcacgc 600
   ctgggtccgg ggtgggcacg gtgcccgggc aacgagctga aagctcatct gctctcaggg 660
   gcccctccct ggggacagcc cctcctggct agtcacaccc tgtaggctcc tctatataac 720
   ccaggggcac aggggctgcc ctcattctac caccacctcc acagcacaga cagacactca 780
   ggagcagcca gc 792
<210> 8
   <211> 8629
   <212> DNA
   <213> Kanamycin Plasmid
<400> 8
   gcgcgctcgc tcgctcactg aggccgcccg ggcaaagccc gggcgtcggg cgacctttgg      60
   tcgcccggcc tcagtgagcg agcgagcgcg cagagaggga gtggccaact ccatcactag     120
   gggttccttg tagttaatga ttaacccgcc atgctactta tctacgtagc catgctctag     180
   agtttaaaca agcttgcatg tctaagctag acccttcaga ttaaaaataa ctgaggtaag     240
   ggcctgggta ggggaggtgg tgtgagacgc tcctgtctct cctctatctg cccatcggcc     300
   ctttggggag gaggaatgtg cccaaggact aaaaaaaggc catggagcca gaggggcgag     360
   ggcaacagac ctttcatggg caaaccttgg ggccctgctg tctagcatgc cccactacgg     420
   gtctaggctg cccatgtaag gaggcaaggc ctggggacac ccgagatgcc tggttataat     480
   taacccagac atgtggctgc cccccccccc ccaacacctg ctgcctctaa aaataaccct     540
   gtccctggtg gatcccctgc atgcgaagat cttcgaacaa ggctgtgggg gactgagggc     600
   aggctgtaac aggcttgggg gccagggctt atacgtgcct gggactccca aagtattact     660
   gttccatgtt cccggcgaag ggccagctgt cccccgccag ctagactcag cacttagttt     720
   aggaaccagt gagcaagtca gcccttgggg cagcccatac aaggccatgg ggctgggcaa     780
   gctgcacgcc tgggtccggg gtgggcacgg tgcccgggca acgagctgaa agctcatctg     840
   ctctcagggg cccctccctg ggacagcccc ctcctggcta gtcacaccct gtaggctcct     900
   ctatataacc caggggcaca ggggctgccc tcattctacc accacctcca cagcacagac     960
   agacactcag gagcagccag cggcgcgccc aggtaagttt agtctttttg tcttttattt    1020
   caggtcccgg atccggtggt ggtgcaaatc aaagaactgc tcctcagtgg atgttgcctt    1080
   tacttctagg cctgtacgga agtgttactt ctgctctaaa agctgcggaa ttgtacccgc    1140
   ggccgccacc atgctgtggt gggaggaggt ggaggattgt tatgaaaggg aggacgtgca     1200
   gaagaagact tttaccaagt gggtgaacgc tcagttcagc aaatttggga agcagcacat     1260
   cgagaatctg ttttccgacc tgcaggatgg gagacggctg ctggatctgc tggaaggact     1320
   gactggccag aagctgccca aagagaaggg gagcactagg gtgcacgccc tgaacaacgt     1380
   gaacaaagct ctgagagtgc tgcagaacaa caacgtggat ctggtgaata ttggcagtac     1440
   tgatatcgtg gacgggaacc acaaactgac actgggcctg atctggaaca ttattctgca     1500
   ctggcaggtg aaaaatgtga tgaagaacat catggccggg ctgcagcaga ccaattccga     1560
   gaagatcctg ctgtcttggg tgcggcagag cacccgcaac tatccccagg tgaacgtgat     1620
   taacttcact acatcctgga gcgacgggct ggccctgaat gctctgattc acagccacag     1680
   gcctgatctg ttcgactgga atagcgtggt gtgccagcag tctgccacac agcgcctgga     1740
   acatgccttc aatatcgctc ggtaccagct ggggatcgaa aaactgctgg acccagagga     1800
   tgtggacact acatacccag ataaaaagtc tattctgatg tacattacta gcctgttcca     1860
   ggtgctgcca cagcaggtgt ctattgaagc cattcaggag gtggaaatgc tgccccgccc     1920
   ccccaaagtg actaaagagg agcattttca gctgcatcat cagatgcatt acagccagca     1980
   gattaccgtg agcctggctc agggatatga gcgcaccagt agtccaaaac cacggttcaa     2040
   gtcctacgct tatacccagg ctgcctacgt gacaactagc gaccctacta gatccccctt     2100
   tccatcccag cacctggagg ccccagagga caagagcttt gggtccagcc tgatggaaag     2160
   cgaggtgaat ctggatcggt accagacagc cctggaggag gtgctgagct ggctgctgag     2220
   tgctgaagac acactgcagg cccagggcga aatttccaat gacgtggaag tggtgaagga     2280
   tcagttccac acacacgagg gctatatgat ggacctgaca gctcaccagg ggcgcgtggg     2340
   caatatcctg cagctgggct ctaaactgat cggcaccggg aaactgagtg aggacgagga     2400
   aacagaagtg caggagcaga tgaacctgct gaacagccgc tgggagtgtc tgagagtggc     2460
   tagtatggag aagcagtcca acctgcaccg ggtgctgatg gacctgcaga accagaaact     2520
   gaaagagctg aacgactggc tgacaaagac tgaggaacgc acaaggaaga tggaggagga     2580
   gccactggga cccgacctgg aggatctgaa gagacaggtg cagcagcata aggtgctgca     2640
   ggaggatctg gaacaggagc aggtgcgggt gaactccctg acacatatgg tggtggtggt     2700
   ggacgaatct agtggagatc acgccaccgc cgccctggag gaacagctga aggtgctggg     2760
   ggaccggtgg gccaacattt gccggtggac cgaggacagg tgggtgctgc tgcaggacat     2820
   cctgctgaaa tggcagaggc tgaccgagga gcagtgtctg tttagtgctt ggctgagcga     2880
   gaaagaggac gccgtgaaca agatccacac aaccggcttt aaggatcaga acgaaatgct     2940
   gtctagcctg cagaaactgg ctgtgctgaa ggccgatctg gagaaaaaga agcagagcat     3000
   gggcaaactg tatagcctga aacaggacct gctgagcacc ctgaagaaca agagcgtgac     3060
   ccagaagaca gaagcctggc tggataactt tgcccgctgc tgggacaacc tggtgcagaa     3120
   actggagaaa agtacagctc agatctctca ggctgtgacc acaacccagc ctagcctgac     3180
   ccagacaacc gtgatggaaa ccgtgaccac cgtgacaacc cgcgaacaga tcctggtgaa 3240
   acatgcccag gaagagctgc cacctccacc tccccagaag aagagaaccc tggagcggct 3300
   gcaggagctg caggaagcca ctgacgaact ggacctgaag ctgaggcagg ccgaagtgat 3360
   taaggggtct tggcagcctg tgggcgatct gctgattgat tccctgcagg accacctgga 3420
   aaaggtgaag gctctgagag gcgaaattgc tccactgaag gagaacgtga gtcatgtgaa 3480
   cgatctggct agacagctga caacactggg catccagctg agcccataca atctgagcac 3540
   actggaggac ctgaatacca ggtggaagct gctgcaggtg gctgtggaag accgggtgcg 3600
   gcagctgcat gaggcccatc gcgacttcgg accagccagc cagcactttc tgagcacatc 3660
   cgtgcagggg ccctgggaga gggccatttc tcccaacaag gtgccctact atattaatca 3720
   cgagacccag accacttgtt gggaccatcc caagatgaca gaactgtacc agtccctggc 3780
   cgatctgaac aacgtgaggt ttagcgctta cagaaccgct atgaagctga gacggctgca 3840
   gaaggccctg tgcctggatc tgctgtccct gtccgccgcc tgcgatgccc tggatcagca 3900
   taatctgaag cagaacgatc agccaatgga tatcctgcag atcatcaact gcctgaccac 3960
   tatctacgac aggctggagc aggagcacaa caacctggtg aacgtgcctc tgtgcgtgga 4020
   tatgtgcctg aactggctgc tgaacgtgta tgacactggg cgcaccggcc ggatcagagt 4080
   gctgagtttt aaaactggga ttatctccct gtgtaaggcc cacctggagg acaagtacag 4140
   gtacctgttc aagcaggtgg ctagtagcac tggattttgt gaccagcgcc gcctgggact 4200
   gctgctgcat gatagtatcc agattcctag acagctggga gaggtggcta gtttcggagg 4260
   atctaacatc gaacccagcg tgcgcagctg tttccagttt gccaataaca aacctgaaat 4320
   cgaggctgct ctgttcctgg attggatgcg cctggaacca cagagcatgg tgtggctgcc 4380
   tgtgctgcac agagtggctg ccgccgaaac tgccaagcac caggctaaat gcaacatctg 4440
   caaggaatgt cccattatcg gctttcgcta caggagtctg aaacatttta actacgatat 4500
   ttgccagagc tgcttctttt ccggaagagt ggccaaagga cacaagatgc actaccctat 4560
   ggtggaatat tgcaccccaa ctacatctgg cgaagatgtg cgcgattttg ccaaggtgct 4620
   gaagaataag tttcggacta agaggtactt cgccaagcac ccccgcatgg ggtatctgcc 4680
   agtgcagaca gtgctggaag gagacaatat ggagaccgat acaatgtgag cggccgcaat 4740
   aaaagatctt tattttcatt agatctgtgt gttggttttt tgtgtgtcta gagtcgacca 4800
   gagcatggct acgtagataa gtagcatggc gggttaatca ttaactacaa ggaaccccta 4860
   gtgatggagt tggccactcc ctctctgcgc gctcgctcgc tcactgaggc cgggcgacca 4920
   aaggtcgccc gacgcccggg ctttgcccgg gcggcctcag tgagcgagcg agcgcgcagc 4980
   tgcattaatg aatcggccaa cgcgcgggga gaggcggttt gcgtattggg cgctcttccg 5040
   cttcctcgct cactgactcg ctgcgctcgg tcgttcggct gcggcgagcg gtatcagctc 5100
   actcaaaggc ggtaatacgg ttatccacag aatcagggga taacgcagga aagaacatgt 5160
   gagcaaaagg ccagcaaaag gccaggaacc gtaaaaaggc cgcgttgctg gcgtttttcc 5220
   ataggctccg cccccctgac gagcatcaca aaaatcgacg ctcaagtcag aggtggcgaa 5280
   acccgacagg actataaaga taccaggcgt ttccccctgg aagctccctc gtgcgctctc 5340
   ctgttccgac cctgccgctt accggatacc tgtccgcctt tctcccttcg ggaagcgtgg 5400
   cgctttctca tagctcacgc tgtaggtatc tcagttcggt gtaggtcgtt cgctccaagc 5460
   tgggctgtgt gcacgaaccc cccgttcagc ccgaccgctg cgccttatcc ggtaactatc 5520
   gtcttgagtc caacccggta agacacgact tatcgccact ggcagcagcc actggtaaca 5580
   ggattagcag agcgaggtat gtaggcggtg ctacagagtt cttgaagtgg tggcctaact 5640
   acggctacac tagaagaaca gtatttggta tctgcgctct gctgaagcca gttaccttcg 5700
   gaaaaagagt tggtagctct tgatccggca aacaaaccac cgctggtagc ggtggttttt 5760
   ttgtttgcaa gcagcagatt acgcgcagaa aaaaaggatc tcaagaagat cctttgatct 5820
   tttctacggg gtctgacgct cagtggaacg aaaactcacg ttaagggatt ttggtcatga 5880
   gattatcaaa aaggatcttc acctagatcc ttttaaatta aaaatgaagt tttaaatcaa 5940
   tctaaagtat atatgagtaa aaatattccg gaattgccag ctggggcgcc ctctggtaag 6000
   gttgggaagc cctgcaaagt aaactggatg gctttcttgc cgccaaggat ctgatggcgc 6060
   aggggatcaa gatctgatca agagacagga tgaggatcgt ttcgcatgat tgaacaagat 6120
   ggattgcacg caggttctcc ggccgcttgg gtggagaggc tattcggcta tgactgggca 6180
   caacagacaa tcggctgctc tgatgccgcc gtgttccggc tgtcagcgca ggggcgcccg 6240
   gttctttttg tcaagaccga cctgtccggt gccctgaatg aactgcagga cgaggcagcg 6300
   cggctatcgt ggctggccac gacgggcgtt ccttgcgcag ctgtgctcga cgttgtcact 6360
   gaagcgggaa gggactggct gctattgggc gaagtgccgg ggcaggatct cctgtcatcc 6420
   caccttgctc ctgccgagaa agtatccatc atggctgatg caatgcggcg gctgcatacg 6480
   cttgatccgg ctacctgccc attcgaccac caagcgaaac atcgcatcga gcgagcacgt 6540
   actcggatgg aagccggtct tgtcgatcag gatgatctgg acgaagagca tcaggggctc 6600
   gcgccagccg aactgttcgc caggctcaag gcgcgcatgc ccgacggcga ggatctcgtc 6660
   gtgacccatg gcgatgcctg cttgccgaat atcatggtgg aaaatggccg cttttctgga 6720
   ttcatcgact gtggccggct gggtgtggcg gaccgctatc aggacatagc gttggctacc 6780
   cgtgatattg ctgaagagct tggcggcgaa tgggctgacc gcttcctcgt gctttacggt 6840
   atcgccgctc ccgattcgca gcgcatcgcc ttctatcgcc ttcttgacga gttcttctga 6900
   accggtaata ttattgaagc atttatcagg gttattgtct catgagcgga tacatatttg 6960
   aatgtattta gaaaaataaa caaatagggg ttccgcgcac atttccccga aaagtgccac 7020
   ctgacgtcta agaaaccatt attatcatga cattaaccta taaaaatagg cgtatcacga 7080
   ggccctttcg tctcgcgcgt ttcggtgatg acggtgaaaa cctctgacac atgcagctcc 7140
   cggagacggt cacagcttgt ctgtaagcgg atgccgggag cagacaagcc cgtcagggcg 7200
   cgtcagcggg tgttggcggg tgtcggggct ggcttaacta tgcggcatca gagcagattg 7260
   tactgagagt gcaccatatg cggtgtgaaa taccgcacag atgcgtaagg agaaaatacc     7320
   gcatcaggcg attccaacat ccaataaatc atacaggcaa ggcaaagaat tagcaaaatt     7380
   aagcaataaa gcctcagagc ataaagctaa atcggttgta ccaaaaacat tatgaccctg     7440
   taatactttt gcgggagaag cctttatttc aacgcaagga taaaaatttt tagaaccctc     7500
   atatatttta aatgcaatgc ctgagtaatg tgtaggtaaa gattcaaacg ggtgagaaag     7560
   gccggagaca gtcaaatcac catcaatatg atattcaacc gttctagctg ataaattcat     7620
   gccggagagg gtagctattt ttgagaggtc tctacaaagg ctatcaggtc attgcctgag     7680
   agtctggagc aaacaagaga atcgatgaac ggtaatcgta aaactagcat gtcaatcata     7740
   tgtaccccgg ttgataatca gaaaagcccc aaaaacagga agattgtata agcaaatatt     7800
   taaattgtaa gcgttaatat tttgttaaaa ttcgcgttaa atttttgtta aatcagctca     7860
   ttttttaacc aataggccga aatcggcaaa atcccttata aatcaaaaga atagaccgag     7920
   atagggttga gtgttgttcc agtttggaac aagagtccac tattaaagaa cgtggactcc     7980
   aacgtcaaag ggcgaaaaac cgtctatcag ggcgatggcc cactacgtga accatcaccc     8040
   taatcaagtt ttttggggtc gaggtgccgt aaagcactaa atcggaaccc taaagggagc     8100
   ccccgattta gagcttgacg gggaaagccg gcgaacgtgg cgagaaagga agggaagaaa     8160
   gcgaaaggag cgggcgctag ggcgctggca agtgtagcgg tcacgctgcg cgtaaccacc     8220
   acacccgccg cgcttaatgc gccgctacag ggcgcgtact atggttgctt tgacgagcac     8280
   gtataacgtg ctttcctcgt tagaatcaga gcgggagcta aacaggaggc cgattaaagg     8340
   gattttagac aggaacggta cgccagaatc ctgagaagtg tttttataat cagtgaggcc     8400
   accgagtaaa agagtctgtc catcacgcaa attaaccgtt gtcgcaatac ttctttgatt     8460
   agtaataaca tcacttgcct gagtagaaga actcaaacta tcggccttgc tggtaatatc     8520
   cagaacaata ttaccgccag ccattgcaac ggaatcgcca ttcgccattc aggctgcgca     8580
   actgttggga agggcgatcg gtgcgggcct cttcgctatt acgccagct                8629
<210> 9
   <211> 4977
   <212> DNA
   <213> Kanamycin Cassatte
<400> 9
   gcgcgctcgc tcgctcactg aggccgcccg ggcaaagccc gggcgtcggg cgacctttgg      60
   tcgcccggcc tcagtgagcg agcgagcgcg cagagaggga gtggccaact ccatcactag     120
   gggttccttg tagttaatga ttaacccgcc atgctactta tctacgtagc catgctctag     180
   agtttaaaca agcttgcatg tctaagctag acccttcaga ttaaaaataa ctgaggtaag     240
   ggcctgggta ggggaggtgg tgtgagacgc tcctgtctct cctctatctg cccatcggcc     300
   ctttggggag gaggaatgtg cccaaggact aaaaaaaggc catggagcca gaggggcgag     360
   ggcaacagac ctttcatggg caaaccttgg ggccctgctg tctagcatgc cccactacgg     420
   gtctaggctg cccatgtaag gaggcaaggc ctggggacac ccgagatgcc tggttataat 480
   taacccagac atgtggctgc cccccccccc ccaacacctg ctgcctctaa aaataaccct 540
   gtccctggtg gatcccctgc atgcgaagat cttcgaacaa ggctgtgggg gactgagggc 600
   aggctgtaac aggcttgggg gccagggctt atacgtgcct gggactccca aagtattact 660
   gttccatgtt cccggcgaag ggccagctgt cccccgccag ctagactcag cacttagttt 720
   aggaaccagt gagcaagtca gcccttgggg cagcccatac aaggccatgg ggctgggcaa 780
   gctgcacgcc tgggtccggg gtgggcacgg tgcccgggca acgagctgaa agctcatctg 840
   ctctcagggg cccctccctg gggacagccc ctcctggcta gtcacaccct gtaggctcct 900
   ctatataacc caggggcaca ggggctgccc tcattctacc accacctcca cagcacagac 960
   agacactcag gagcagccag cggcgcgccc aggtaagttt agtctttttg tcttttattt 1020
   caggtcccgg atccggtggt ggtgcaaatc aaagaactgc tcctcagtgg atgttgcctt 1080
   tacttctagg cctgtacgga agtgttactt ctgctctaaa agctgcggaa ttgtacccgc 1140
   ggccgccacc atgctgtggt gggaggaggt ggaggattgt tatgaaaggg aggacgtgca 1200
   gaagaagact tttaccaagt gggtgaacgc tcagttcagc aaatttggga agcagcacat 1260
   cgagaatctg ttttccgacc tgcaggatgg gagacggctg ctggatctgc tggaaggact 1320
   gactggccag aagctgccca aagagaaggg gagcactagg gtgcacgccc tgaacaacgt 1380
   gaacaaagct ctgagagtgc tgcagaacaa caacgtggat ctggtgaata ttggcagtac 1440
   tgatatcgtg gacgggaacc acaaactgac actgggcctg atctggaaca ttattctgca 1500
   ctggcaggtg aaaaatgtga tgaagaacat catggccggg ctgcagcaga ccaattccga 1560
   gaagatcctg ctgtcttggg tgcggcagag cacccgcaac tatccccagg tgaacgtgat 1620
   taacttcact acatcctgga gcgacgggct ggccctgaat gctctgattc acagccacag 1680
   gcctgatctg ttcgactgga atagcgtggt gtgccagcag tctgccacac agcgcctgga 1740
   acatgccttc aatatcgctc ggtaccagct ggggatcgaa aaactgctgg acccagagga 1800
   tgtggacact acatacccag ataaaaagtc tattctgatg tacattacta gcctgttcca 1860
   ggtgctgcca cagcaggtgt ctattgaagc cattcaggag gtggaaatgc tgccccgccc 1920
   ccccaaagtg actaaagagg agcattttca gctgcatcat cagatgcatt acagccagca 1980
   gattaccgtg agcctggctc agggatatga gcgcaccagt agtccaaaac cacggttcaa 2040
   gtcctacgct tatacccagg ctgcctacgt gacaactagc gaccctacta gatccccctt 2100
   tccatcccag cacctggagg ccccagagga caagagcttt gggtccagcc tgatggaaag 2160
   cgaggtgaat ctggatcggt accagacagc cctggaggag gtgctgagct ggctgctgag 2220
   tgctgaagac acactgcagg cccagggcga aatttccaat gacgtggaag tggtgaagga 2280
   tcagttccac acacacgagg gctatatgat ggacctgaca gctcaccagg ggcgcgtggg 2340
   caatatcctg cagctgggct ctaaactgat cggcaccggg aaactgagtg aggacgagga 2400
   aacagaagtg caggagcaga tgaacctgct gaacagccgc tgggagtgtc tgagagtggc 2460
   tagtatggag aagcagtcca acctgcaccg ggtgctgatg gacctgcaga accagaaact 2520
   gaaagagctg aacgactggc tgacaaagac tgaggaacgc acaaggaaga tggaggagga 2580
   gccactggga cccgacctgg aggatctgaa gagacaggtg cagcagcata aggtgctgca 2640
   ggaggatctg gaacaggagc aggtgcgggt gaactccctg acacatatgg tggtggtggt 2700
   ggacgaatct agtggagatc acgccaccgc cgccctggag gaacagctga aggtgctggg 2760
   ggaccggtgg gccaacattt gccggtggac cgaggacagg tgggtgctgc tgcaggacat 2820
   cctgctgaaa tggcagaggc tgaccgagga gcagtgtctg tttagtgctt ggctgagcga 2880
   gaaagaggac gccgtgaaca agatccacac aaccggcttt aaggatcaga acgaaatgct 2940
   gtctagcctg cagaaactgg ctgtgctgaa ggccgatctg gagaaaaaga agcagagcat 3000
   gggcaaactg tatagcctga aacaggacct gctgagcacc ctgaagaaca agagcgtgac 3060
   ccagaagaca gaagcctggc tggataactt tgcccgctgc tgggacaacc tggtgcagaa 3120
   actggagaaa agtacagctc agatctctca ggctgtgacc acaacccagc ctagcctgac 3180
   ccagacaacc gtgatggaaa ccgtgaccac cgtgacaacc cgcgaacaga tcctggtgaa 3240
   acatgcccag gaagagctgc cacctccacc tccccagaag aagagaaccc tggagcggct 3300
   gcaggagctg caggaagcca ctgacgaact ggacctgaag ctgaggcagg ccgaagtgat 3360
   taaggggtct tggcagcctg tgggcgatct gctgattgat tccctgcagg accacctgga 3420
   aaaggtgaag gctctgagag gcgaaattgc tccactgaag gagaacgtga gtcatgtgaa 3480
   cgatctggct agacagctga caacactggg catccagctg agcccataca atctgagcac 3540
   actggaggac ctgaatacca ggtggaagct gctgcaggtg gctgtggaag accgggtgcg 3600
   gcagctgcat gaggcccatc gcgacttcgg accagccagc cagcactttc tgagcacatc 3660
   cgtgcagggg ccctgggaga gggccatttc tcccaacaag gtgccctact atattaatca 3720
   cgagacccag accacttgtt gggaccatcc caagatgaca gaactgtacc agtccctggc 3780
   cgatctgaac aacgtgaggt ttagcgctta cagaaccgct atgaagctga gacggctgca 3840
   gaaggccctg tgcctggatc tgctgtccct gtccgccgcc tgcgatgccc tggatcagca 3900
   taatctgaag cagaacgatc agccaatgga tatcctgcag atcatcaact gcctgaccac 3960
   tatctacgac aggctggagc aggagcacaa caacctggtg aacgtgcctc tgtgcgtgga 4020
   tatgtgcctg aactggctgc tgaacgtgta tgacactggg cgcaccggcc ggatcagagt 4080
   gctgagtttt aaaactggga ttatctccct gtgtaaggcc cacctggagg acaagtacag 4140
   gtacctgttc aagcaggtgg ctagtagcac tggattttgt gaccagcgcc gcctgggact 4200
   gctgctgcat gatagtatcc agattcctag acagctggga gaggtggcta gtttcggagg 4260
   atctaacatc gaacccagcg tgcgcagctg tttccagttt gccaataaca aacctgaaat 4320
   cgaggctgct ctgttcctgg attggatgcg cctggaacca cagagcatgg tgtggctgcc 4380
   tgtgctgcac agagtggctg ccgccgaaac tgccaagcac caggctaaat gcaacatctg 4440
   caaggaatgt cccattatcg gctttcgcta caggagtctg aaacatttta actacgatat 4500
   ttgccagagc tgcttctttt ccggaagagt ggccaaagga cacaagatgc actaccctat     4560
   ggtggaatat tgcaccccaa ctacatctgg cgaagatgtg cgcgattttg ccaaggtgct     4620
   gaagaataag tttcggacta agaggtactt cgccaagcac ccccgcatgg ggtatctgcc     4680
   agtgcagaca gtgctggaag gagacaatat ggagaccgat acaatgtgag cggccgcaat     4740
   aaaagatctt tattttcatt agatctgtgt gttggttttt tgtgtgtcta gagtcgacca     4800
   gagcatggct acgtagataa gtagcatggc gggttaatca ttaactacaa ggaaccccta     4860
   gtgatggagt tggccactcc ctctctgcgc gctcgctcgc tcactgaggc cgggcgacca     4920
   aaggtcgccc gacgcccggg ctttgcccgg gcggcctcag tgagcgagcg agcgcgc        4977